# EUROPÄISCHE PATENTSCHRIFT

(11) **EP 2 484 766 B1**
(45) Veröffentlichungstag und Bekanntmachung des Hinweises auf die Patenterteilung: **03.07.2013**
(21) Anmeldenummer: 10180732.9
(22) Anmeldetag: 28.09.2010
(51) Int. Cl.: C12N 15/81, C12R 1/645, C12N 1/16

(54) **Verfahren zum Herstellen eines Ziel-Proteins im Verbund mit einem heterologen Ferritin-Protein unter Verwendung von Hefe-Wirtszellen**
Method for producing a target protein connected with a heterogeneous ferritin protein using yeast host cells
Procédé de fabrication d'une protéine cible en relation avec une protéine de ferritine hétérologue en utilisant des cellules-hôtes de levure

(43) Veröffentlichungstag der Anmeldung: 08.08.2012
(73) Patentinhaber: Artes Biotechnology GmbH, 40764 Langenfeld (DE)
(72) Erfinder: Suckow, Manfred, Dr., 40593 Düsseldorf (DE); Eilert, Eva, 40223 Düsseldorf (DE); Piontek, Michael, Dr., 42549 Velbert (DE)
(74) Vertreter: Zenz

(56) Entgegenhaltungen:
- KR-A- 20020 075 068
- FABER K N ET AL: "Foreign gene expression in Hansenula polymorpha. A system for the synthesis of small functional peptides", APPLIED MICROBIOLOGY AND BIOTECHNOLOGY, Bd. 45, Nr. 1-2, 1996, Seiten 72-79, XP009160678, ISSN: 0175-7598
- LEE JUNG-LIM ET AL: "Functional assembly of recombinant human ferritin subunits in Pichia pastoris", JOURNAL OF MICROBIOLOGY AND BIOTECHNOLOGY, Bd. 17, Nr. 10, Oktober 2007 (2007-10), Seiten 1695-1699 URL, XP002678808, -& WO 02/40695 A1 (RNA INC [KR]; KIM HAE YEONG [KR]; LEE JUNG LIM [KR]; CHUNG DAE KYUN [K) 23. Mai 2002 (2002-05-23)
- SEO H -Y ET AL: "Enhanced expression and functional characterization of the human ferritin H- and L-chain genes in Saccharomyces cerevisiae.", APPLIED MICROBIOLOGY AND BIOTECHNOLOGY, Bd. 63, Nr. 1, November 2003 (2003-11), Seiten 57-63, XP002678809, ISSN: 0175-7598
- VAD R ET AL: "Engineering of a Pichia pastoris expression system for secretion of high amounts of intact human parathyroid hormone", JOURNAL OF BIOTECHNOLOGY, ELSEVIER SCIENCE PUBLISHERS, AMSTERDAM, NL, Bd. 116, Nr. 3, 30. März 2005 (2005-03-30) , Seiten 251-260, XP027663548, ISSN: 0168-1656 [gefunden am 2005-03-30]
- EILERT EVA ET AL: "The use of highly expressed FTH1 as carrier protein for cytosolic targeting in Hansenula polymorpha", JOURNAL OF BIOTECHNOLOGY, Bd. 159, Nr. 3, Juni 2012 (2012-06), Seiten 172-176 URL, XP002678810,

### SEQUENCE LISTING

<110> Artes Biotechnology GmbH
<120> Verfahren zum Herstellen eines Ziel-Proteins im Verbund mit einem heterologen Ferritin-Protein unter Verwendung von Hefe-Wirtszellen
<130> A613EP
<160> 17
<170> PatentIn version 3.5
<210> 1
   <211> 8252
   <212> DNA
   <213> künstliche Sequenz
<220>
   <221> ampR
   <222> (212)..(1072)
   <223> á-lactamase
<220>
   <221> ori
   <222> (1217)..(1889)
<220>
   <221> ScURA3
   <222> (2969) .. (4126)
   <223> URA3 gene from S. cerevisiae
<220>
   <221> HARS1
   <222> (4926)..(5921)
   <223> HARS1 H.polymorpha
<220>
   <221> FMD-P
   <222> (5556)..(6621)
   <223> FMD-Promoter H.polymorpha
<220>
   <221> preCL
   <222> (6629)..(6682)
   <223> chicken lysozyme presequence
<220>
   <221> mhACT
   <222> (6683)..(7882)
   <223> maturer humaner alphal-Antichymotrypsin-Precursor
<220>
   <221> H6
   <222> (7883)..(7900)
   <223> 6-Histidin-His-Tag
<220>
   <221> MOX-T
   <222> (7900)..(8240)
   <223> MOX-Terminator H.polymorpha
<400> 1
   caattcttga agacgaaagg gcctcgtgat acgcctattt ttataggtta atgtcatgat      60
   aataatggtt tcttagacgt caggtggcac ttttcgggga aatgtgcgcg gaacccctat     120
   ttgtttattt ttctaaatac attcaaatat gtatccgctc atgagacaat aaccctgata     180
   aatgcttcaa taatattgaa aaaggaagag tatgagtatt caacatttcc gtgtcgccct     240
   tattcccttt tttgcggcat tttgccttcc tgtttttgct cacccagaaa cgctggtgaa     300
   agtaaaagat gctgaagatc agttgggtgc acgagtgggt tacatcgaac tggatctcaa     360
   cagcggtaag atccttgaga gttttcgccc cgaagaacgt tttccaatga tgagcacttt     420
   taaagttctg ctatgtggcg cggtattatc ccgtgttgac gccgggcaag agcaactcgg     480
   tcgccgcata cactattctc agaatgactt ggttgagtac tcaccagtca cagaaaagca     540
   tcttacggat ggcatgacag taagagaatt atgcagtgct gccataacca tgagtgataa     600
   cactgcggcc aacttacttc tgacaacgat cggaggaccg aaggagctaa ccgctttttt     660
   gcacaacatg ggggatcatg taactcgcct tgatcgttgg gaaccggagc tgaatgaagc     720
   cataccaaac gacgagcgtg acaccacgat gcctgcagca atggcaacaa cgttgcgcaa     780
   actattaact ggcgaactac ttactctagc ttcccggcaa caattaatag actggatgga     840
   ggcggataaa gttgcaggac cacttctgcg ctcggccctt ccggctggct ggtttattgc     900
   tgataaatct ggagccggtg agcgtgggtc tcgcggtatc attgcagcac tggggccaga     960
   tggtaagccc tcccgtatcg tagttatcta cacgacgggg agtcaggcaa ctatggatga    1020
   acgaaataga cagatcgctg agataggtgc ctcactgatt aagcattggt aactgtcaga    1080
   ccaagtttac tcatatatac tttagattga tttaaaactt catttttaat ttaaaaggat    1140
   ctaggtgaag atcctttttg ataatctcat gaccaaaatc ccttaacgtg agttttcgtt    1200
   ccactgagcg tcagaccccg tagaaaagat caaaggatct tcttgagatc ctttttttct 1260
   gcgcgtaatc tgctgcttgc aaacaaaaaa accaccgcta ccagcggtgg tttgtttgcc 1320
   ggatcaagag ctaccaactc tttttccgaa ggtaactggc ttcagcagag cgcagatacc 1380
   aaatactgtc cttctagtgt agccgtagtt aggccaccac ttcaagaact ctgtagcacc 1440
   gcctacatac ctcgctctgc taatcctgtt accagtggct gctgccagtg gcgataagtc 1500
   gtgtcttacc gggttggact caagacgata gttaccggat aaggcgcagc ggtcgggctg 1560
   aacggggggt tcgtgcacac agcccagctt ggagcgaacg acctacaccg aactgagata 1620
   cctacagcgt gagctatgag aaagcgccac gcttcccgaa gggagaaagg cggacaggta 1680
   tccggtaagc ggcagggtcg gaacaggaga gcgcacgagg gagcttccag ggggaaacgc 1740
   ctggtatctt tatagtcctg tcgggtttcg ccacctctga cttgagcgtc gatttttgtg 1800
   atgctcgtca ggggggcgga gcctatggaa aaacgccagc aacgcggcct ttttacggtt 1860
   cctggccttt tgctggcctt ttgctcacat gttctttcct gcgttatccc ctgattctgt 1920
   ggataaccgt attaccgcct ttgagtgagc tgataccgct cgccgcagcc gaacgaccga 1980
   gcgcagcgag tcagtgagcg aggaagcgga agagcgcctg atgcggtatt ttctccttac 2040
   gcatctgtgc ggtatttcac accgcatatg gtgcactctc agtacaatct gctctgatgc 2100
   cgcatagtta agccagtata cactccgcta tcgctacgtg actgggtcat ggctgcgccc 2160
   cgacacccgc caacacccgc tgacgcgccc tgacgggctt gtctgctccc ggcatccgct 2220
   tacagacaag ctgtgaccgt ctccgggagc tgcatgtgtc agaggttttc accgtcatca 2280
   ccgaaacgcg cgaggcagct gcggtaaagc tcatcagcgt ggtcgtgaag cgattcacag 2340
   atgtctgcct gttcatccgc gtccagctcg ttgagtttct ccagaagcgt taatgtctgg 2400
   cttctgataa agcgggccat gttaagggcg gttttttcct gtttggtcac tgatgcctcc 2460
   gtgtaagggg gatttctgtt catgggggta atgataccga tgaaacgaga gaggatgctc 2520
   acgatacggg ttactgatga tgaacatgcc cggttactgg aacgttgtga gggtaaacaa 2580
   ctggcggtat ggatgcggcg ggaccagaga aaaatcactc agggtcaatg ccagcgcttc 2640
   gttaatacag atgtaggtgt tccacagggt agccagcagc atcctgcgat gcagatccgg 2700
   aacataatgg tgcagggcgc tgacttccgc gtttccagac tttacgaaac acggaaaccg 2760
   aagaccattc atgttgttgc tcaggtcgca gacgttttgc agcagcagtc gcttcacgtt 2820
   cgctcgcgta tcggtgattc attctgctaa ccagtaaggc aaccccgcca gcctagccgg 2880
   gtcctcaacg acaggagcac gatcatgcgc acccgtggcc aggacccaac gctgcggggg 2940
   gggggggggg gggggggggg gggggggggt tttctttcca attttttttt ttcgtcatta 3000
   taaaaatcat tacgaccgag attcccgggt aataactgat ataattaaat tgaagctcta 3060
   atttgtgagt ttagtataca tgcatttact tataatacag ttttttagtt ttgctggccg 3120
   catcttctca aatatgcttc ccagcctgct tttctgtaac gttcaccctc taccttagca 3180
   tcccttccct ttgcaaatag tcctcttcca acaataataa tgtcagatcc tgtagagacc 3240
   acatcatcca cggttctata ctgttgaccc aatgcgtctc ccttgtcatc taaacccaca 3300
   ccgggtgtca taatcaacca atcgtaacct tcatctcttc cacccatgtc tctttgagca 3360
   ataaagccga taacaaaatc tttgtcgctc ttcgcaatgt caacagtacc cttagtatat 3420
   tctccagtag atagggagcc cttgcatgac aattctgcta acatcaaaag gcctctaggt 3480
   tcctttgtta cttcttctgc cgcctgcttc aaaccgctaa caatacctgg gcccaccaca 3540
   ccgtgtgcat tcgtaatgtc tgcccattct gctattctgt atacacccgc agagtactgc 3600
   aatttgactg tattaccaat gtcagcaaat tttctgtctt cgaagagtaa aaaattgtac 3660
   ttggcggata atgcctttag cggcttaact gtgccctcca tggaaaaatc agtcaagata 3720
   tccacatgtg tttttagtaa acaaattttg ggacctaatg cttcaactaa ctccagtaat 3780
   tccttggtgg tacgaacatc caatgaagca cacaagtttg tttgcttttc gtgcatgata 3840
   ttaaatagct tggcagcaac aggactagga tgagtagcag cacgttcctt atatgtagct 3900
   ttcgacatga tttatcttcg tttcctgcag gtttttgttc tgtgcagttg ggttaagaat 3960
   actgggcaat ttcatgtttc ttcaacacta catatgcgta tatataccaa tctaagtctg 4020
   tgctccttcc ttcgttcttc cttctgttcg gagattaccg aatcaaaaaa atttcaagga 4080
   aaccgaaatc aaaaaaaaga ataaaaaaaa aatgatgaat tgaaaccccc cccccccccc 4140
   cccccccccg atgcgccgcg tgcggctgct ggagatggcg gacgcgatgg atatgttctg 4200
   ccaagggttg gtttgcgcat tcacagttct ccgcaagaat tgattggctc caattcttgg 4260
   agtggtgaat ccgttagcga ggtgccgccg gcttccattc aggtcgaggt ggcccggctc 4320
   catgcaccgc gacgcaacgc ggggaggcag acaaggtata gggcggcgcc tacaatccat      4380
   gccaacccgt tccatgtgct cgccgaggcg gcataaatcg ccgtgacgat cagcggtcca      4440
   atgatcgaag ttaggctggt aagagccgcg agcgatcctt gaagctgtcc ctgatggtcg      4500
   tcatctacct gcctggacag catggcctgc aacgcgggca tcccgatgcc gccggaagcg      4560
   agaagaatca taatggggaa ggccatccag cctcgcgtcg cgaacgccag caagacgtag      4620
   cccagcgcgt cggccgccat gccggcgata atggcctgct tctcgccgaa acgtttggtg      4680
   gcgggaccag tgacgaaggc ttgagcgagg gcgtgcaaga ttccgaatac cgcaagcgac      4740
   aggccgatca tcgtcgcgct ccagcgaaag cggtcctcgc cgaaaatgac ccagagcgct      4800
   gccggcacct gtcctacgag ttgcatgata aagaagacag tcataagtgc ggcgacgata      4860
   gtcatgcccc gcgcccaccg gaaggagctg actgggttga aggctctcaa gggcatcggt      4920
   cgactcccgc gactcggcgt tcactttcga gctattatca acgccggaat acgtcagaaa      4980
   cagccgtgcc ccagggacca gaaagcctac tggtgagtat gttctttcgt gtgatttttc      5040
   cgaggatgag aacgacgata acgagcacaa ctcggagtcg gaggacacgc ttattgcgtt      5100
   gaacgcagcc aacatcagca ggctgtcaag actgagtatg gccacagagc tggatttctc      5160
   ggcctcatac tcaaagacgt ttagtaaact ccgtctgcca gaaattgctg acgaggatgt      5220
   ataataatag atgaattacg aacaattgta gttcaaaaaa atttagtaac aatattgtct      5280
   agatgacaga tttgctgaaa ccagtgaact ccaataaacc actcaccgct acccaagaga      5340
   aacagatcag agtgctaggg cttgtttcag agtactacaa cgtttaccag aagcttgagc      5400
   aagttctcaa acgcgggttt gtcgacgctc tcccttatgc gactcctgca ttaggaagca      5460
   gcccagtagt aggttgaggc cgttgagcac cgccgccgca aggaatggtg tcgacgcgga      5520
   gaacgatctc ctcgacgcgg agaacgatct cctcgagatc caggaagcgc aacgacgcgg      5580
   ggaatttcgt aatcttcccc gaggaaaacc gcttcccaaa gatttcaccg gcaataacgc      5640
   cttcattgac cgcacagagt accacttgaa caacattctg aaacggcagg aggcggttcc      5700
   gccgtggata gagaaacagg gcagtgtgga ggctcagatc cgccagtttc ggcgccagct      5760
   cgactccgat tggacactgc aggccgtgca catcgtttgc gagaaacact cgaagaaaag      5820
   cgacacggac aagatccaga tcatgagcca gtacgccgag gcagagcgca acaataactc      5880
   gagttatacg aagctgcgca gcagccaatg ggaacagaaa tacagcaact atctggaggt 5940
   caaattaaaa gcgctgaatg acagcatccg aggttataat ctgcaggctc ctttggcatc 6000
   gcagaaactg tatctaaacg caaactccga gctggaaaaa tgttaccggc gatgcgcgga 6060
   caatttagag gcggcgatca agaaacacct gctgggcgag cagtctggag cacagtcttc 6120
   gatgggcccg agatcccacc gcgttcctgg gtaccgggac gtgaggcagc gcgacatcca 6180
   tcaaatatac caggcgccaa ccgagtctct cggaaaacag cttctggata tcttccgctg 6240
   gcggcgcaac gacgaataat agtccctgga ggtgacggaa tatatatgtg tggagggtaa 6300
   atctgacagg gtgtagcaaa ggtaatattt tcctaaaaca tgcaatcggc tgccccgcaa 6360
   cgggaaaaag aatgactttg gcactcttca ccagagtggg gtgtcccgct cgtgtgtgca 6420
   aataggctcc cactggtcac cccggatttt gcagaaaaac agcaagttcc ggggtgtctc 6480
   actggtgtcc gccaataaga ggagccggca ggcacggagt ctacatcaag ctgtctccga 6540
   tacactcgac taccatccgg gtctctcaga gaggggaatg gcactataaa taccgcctcc 6600
   ttgcgctctc tgccttcatc gggaattcat gagatccctg ctgatactag tgctgtgttt 6660
   cttgccgcta gctgccttgg gccaccctaa ctcgcctttg gatgaggaaa acctgactca 6720
   ggaaaaccag gatagaggaa ctcacgtaga tctgggattg gcttctgcta acgtggactt 6780
   cgctttctcg ctgtacaagc agctggtttt gaaggctcct gacaagaacg tgatcttctc 6840
   tcctctgtct atttctaccg ctctggcttt cttgtctctg ggtgctcaca acactaccct 6900
   gaccgagatt ctgaagggac tgaagttcaa cctgaccgag acttccgagg ctgagattca 6960
   ccagtctttc cagcacctgc tgagaacttt gaaccagtcc tctgacgagt tgcagctgtc 7020
   tatgggaaac gctatgttcg ttaaggaaca gctgtctctg ttggacagat tcaccgagga 7080
   cgctaagaga ttgtacggat ctgaggcttt cgctactgac tttcaggatt ctgctgctgc 7140
   caagaagctg attaacgact acgtcaagaa cggaaccaga ggaaagatta ccgacctgat 7200
   caaggacttg gactctcaga ccatgatggt cctggtcaac tacattttct tcaaggccaa 7260
   gtgggagatg ccttttgacc ctcaggatac ccaccagtct agattctacc tgtccaagaa 7320
   gaagtgggtc atggtcccta tgatgtctct gcaccacctg accattcctt acttcagaga 7380
   tgaggaactg tcttgcactg ttgtcgagct gaagtacacc ggaaacgcct ctgctctgtt 7440
   cattttgcct gaccaggaca agatggaaga ggttgaggct atgttgctgc ctgagacttt 7500
   gaagagatgg cgcgactctt tggagttcag agagatcgga gagctgtacc tgcctaagtt 7560
   ctctatttcc cgcgactaca acctgaacga cattctgctg cagttgggaa ttgaggaagc 7620
   attcacctct aaggctgacc tgtctggaat taccggtgct agaaacctgg ctgtctctca 7680
   ggttgtccac aaggctgttc tggacgtttt tgaggaagga actgaggctt ctgctgctac 7740
   cgctgtcaag attaccctgc tgtctgctct ggttgagact agaaccattg tcagattcaa 7800
   ccgccctttc ctgatgatta ttgtccctac cgacacccag aacatctttt tcatgtccaa 7860
   ggtcaccaac cctaagcagg ctcaccatca tcaccaccac taataaggat ccccctcgcg 7920
   gacttgccag attctaagga gacgtggaag gacataccgc ttttgagaag cgtgtttgaa 7980
   aatagttctt tttctggttt atatcgttta tgaagtgatg agatgaaaag ctgaaatagc 8040
   gagtatagga aaatttaatg aaaattaaat taaatatttt cttaggctat tagtcacctt 8100
   caaaatgccg gccgcttcta agaacgttgt catgatcgac aactacgact cgtttacctg 8160
   gaacctgtac gagtacctgt gtcaggaggg agccaatgtc gaggttttca ggaacgatca 8220
   gatcaccatt ccggagattg agcaggggta cc 8252
<210> 2
   <211> 7734
   <212> DNA
   <213> künstliche Sequenz
<220>
   <221> MOX-T
   <222> (13) .. (390)
   <223> MOX-Terminator H. polymorpha
<220>
   <221> IFNalpha-2a
   <222> (350)..(847)
   <223> matures humanes Interferon alpha 2a
<220>
   <221> MFa
   <222> (848)..(1102)
   <223> Prepro-Sequenz des Mating Faktor alpha aus S. cerevisiae mit D83E-Austausch
<220>
   <221> FMD-P
   <222> (1110)..(2175)
   <223> FMD-Promotor H. polymorpha
<220>
   <221> HARS1
   <222> (2310)..(2809)
   <223> HARS1 H. polymorpha
<220>
   <221> ScURA3
   <222> (3609)..(4766)
   <223> URA3 gene from S. cerevisiae
<220>
   <221> ori
   <222> (5851)..(6518)
<220>
   <221> ampR
   <222> (6663)..(7523)
   <223> á-lactamase
<400> 2
   ggtacccctg ctcaatctcc ggaatggtga tctgatcgtt cctgaaaacc tcgacattgg      60
   ctccctcctg acacaggtac tcgtacaggt tccaggtaaa cgagtcgtag ttgtcgatca     120
   tgacaacgtt cttagaagcg gccggcattt tgaaggtgac taatagccta agaaaatatt     180
   taatttaatt ttcattaaat tttcctatac tcgctatttc agcttttcat ctcatcactt     240
   cataaacgat ataaaccaga aaaagaacta ttttcaaaca cgcttctcaa aagcggtatg     300
   tccttccacg tctccttaga atctggcaag tccgcgaggg ggatccttat tactccttcg     360
   atctcaaaga ctcctgcagg tttgtggaca gcgaaaagga tctcatgatc tctgctctga     420
   cgacctccca ggcgcatggc gagtactttt tctccttcag gtacaatgta atgcgctgga     480
   agtactttct aactgccagg atggaatcct ccttcatcaa aggggtttct gtaacgccga     540
   caccttggat cacgcatgcc tccaggtcgt tcagttgctg atacaactca gtgtaaaatt     600
   tatccagcaa agtctcatcc caagctgccg aggagtcctt cgtagagaac aggttaaaaa     660
   tctgctgaat catctcgtgc agcacaggga ttgtttctgc tttctgaaac tggtttccaa     720
   attcctcctg cgggaagcca aagtcgtgtc tgtccttcaa acacgaaaac agagagatct     780
   ttctcatctg agccaacagc atcagggttc tgcgcgagcc cagggagtgt gtctgtggca     840
   aatcacatct cttttccaag cttacccctt cttctttagc agcaatgctg gcaatagtag 900
   tatttataaa caataacccg ttatttgtgc tgttggaaaa tggcaaaaca gcaacatcga 960
   aatccccttc taaatctgag taaccgatga cagcttcagc cggaatttgt gccgtttcat 1020
   cttctgttgt agtgttgact ggagcagcta atgcggagga tgctgcgaat aaaactgcag 1080
   taaaaattga aggaaatctc atgaattccc gatgaaggca gagagcgcaa ggaggcggta 1140
   tttatagtgc cattcccctc tctgagagac ccggatggta gtcgagtgta tcggagacag 1200
   cttgatgtag actccgtgcc tgccggctcc tcttattggc ggacaccagt gagacacccc 1260
   ggaacttgct gtttttctgc aaaatccggg gtgaccagtg ggagcctatt tgcacacacg 1320
   agcgggacac cccactctgg tgaagagtgc caaagtcatt ctttttcccg ttgcggggca 1380
   gccgattgca tgttttagga aaatattacc tttgctacac cctgtcagat ttaccctcca 1440
   cacatatata ttccgtcacc tccagggact attattcgtc gttgcgccgc cagcggaaga 1500
   tatccagaag ctgttttccg agagactcgg ttggcgcctg gtatatttga tggatgtcgc 1560
   gctgcctcac gtcccggtac ccaggaacgc ggtgggatct cgggcccatc gaagactgtg 1620
   ctccagactg ctcgcccagc aggtgtttct tgatcgccgc ctctaaattg tccgcgcatc 1680
   gccggtaaca tttttccagc tcggagtttg cgtttagata cagtttctgc gatgccaaag 1740
   gagcctgcag attataacct cggatgctgt cattcagcgc ttttaatttg acctccagat 1800
   agttgctgta tttctgttcc cattggctgc tgcgcagctt cgtataactc gagttattgt 1860
   tgcgctctgc ctcggcgtac tggctcatga tctggatctt gtccgtgtcg cttttcttcg 1920
   agtgtttctc gcaaacgatg tgcacggcct gcagtgtcca atcggagtcg agctggcgcc 1980
   gaaactggcg gatctgagcc tccacactgc cctgtttctc tatccacggc ggaaccgcct 2040
   cctgccgttt cagaatgttg ttcaagtggt actctgtgcg gtcaatgaag gcgttattgc 2100
   cggtgaaatc tttgggaagc ggttttcctc ggggaagatt acgaaattcc ccgcgtcgtt 2160
   gcgcttcctg gatctcgagg agatcgttct ccgcgtcgag gagatcgttc tccgcgtcga 2220
   caccattcct tgcggcggcg gtgctcaacg gcctcaacct actactgggc tgcttcctaa 2280
   tgcaggagtc gcataaggga gagcgtcgac aaacccgcgt ttgagaactt gctcaagcta 2340
   gcttctggta aacgttgtag tactctgaaa caagccctag cactctgatc tgtttctctt 2400
   gggtagcggt gagtggttta ttggagttca ctggtttcag caaatctgtc atctagacaa 2460
   tattgttact aaattttttt gaactacaat tgttcgtaat tcatctatta ttatacatcc 2520
   tcgtcagcaa tttctggcag acggagttta ctaaacgtct ttgagtatga ggccgagaaa 2580
   tccagctctg tggccatact cagtcttgac agcctgctga tgttggctgc gttcaacgca 2640
   ataagcgtgt cctccgactc cgagttgtgc tcgttatcgt cgttctcatc ctcggaaaaa 2700
   tcacacgaaa gaacatactc accagtaggc tttctggtcc ctggggcacg gctgtttctg 2760
   acgtattccg gcgttgataa tagctcgaaa gtgaacgccg agtcgcggga gtcgaccgat 2820
   gcccttgaga gccttcaacc cagtcagctc cttccggtgg gcgcggggca tgactatcgt 2880
   cgccgcactt atgactgtct tctttatcat gcaactcgta ggacaggtgc cggcagcgct 2940
   ctgggtcatt ttcggcgagg accgctttcg ctggagcgcg acgatgatcg gcctgtcgct 3000
   tgcggtattc ggaatcttgc acgccctcgc tcaagccttc gtcactggtc ccgccaccaa 3060
   acgtttcggc gagaagcagg ccattatcgc cggcatggcg gccgacgcgc tgggctacgt 3120
   cttgctggcg ttcgcgacgc gaggctggat ggccttcccc attatgattc ttctcgcttc 3180
   cggcggcatc gggatgcccg cgttgcaggc catgctgtcc aggcaggtag atgacgacca 3240
   tcagggacag cttcaaggat cgctcgcggc tcttaccagc ctaacttcga tcattggacc 3300
   gctgatcgtc acggcgattt atgccgcctc ggcgagcaca tggaacgggt tggcatggat 3360
   tgtaggcgcc gccctatacc ttgtctgcct ccccgcgttg cgtcgcggtg catggagccg 3420
   ggccacctcg acctgaatgg aagccggcgg cacctcgcta acggattcac cactccaaga 3480
   attggagcca atcaattctt gcggagaact gtgaatgcgc aaaccaaccc ttggcagaac 3540
   atatccatcg cgtccgccat ctccagcagc cgcacgcggc gcatcggggg gggggggggg 3600
   gggggggggt ttcaattcat catttttttt ttattctttt ttttgatttc ggtttccttg 3660
   aaattttttt gattcggtaa tctccgaaca gaaggaagaa cgaaggaagg agcacagact 3720
   tagattggta tatatacgca tatgtagtgt tgaagaaaca tgaaattgcc cagtattctt 3780
   aacccaactg cacagaacaa aaacctgcag gaaacgaaga taaatcatgt cgaaagctac 3840
   atataaggaa cgtgctgcta ctcatcctag tcctgttgct gccaagctat ttaatatcat 3900
   gcacgaaaag caaacaaact tgtgtgcttc attggatgtt cgtaccacca aggaattact 3960
   ggagttagtt gaagcattag gtcccaaaat ttgtttacta aaaacacatg tggatatctt 4020
   gactgatttt tccatggagg gcacagttaa gccgctaaag gcattatccg ccaagtacaa 4080
   ttttttactc ttcgaagaca gaaaatttgc tgacattggt aatacagtca aattgcagta 4140
   ctctgcgggt gtatacagaa tagcagaatg ggcagacatt acgaatgcac acggtgtggt 4200
   gggcccaggt attgttagcg gtttgaagca ggcggcagaa gaagtaacaa aggaacctag 4260
   aggccttttg atgttagcag aattgtcatg caagggctcc ctatctactg gagaatatac 4320
   taagggtact gttgacattg cgaagagcga caaagatttt gttatcggct ttattgctca 4380
   aagagacatg ggtggaagag atgaaggtta cgattggttg attatgacac ccggtgtggg 4440
   tttagatgac aagggagacg cattgggtca acagtataga accgtggatg atgtggtctc 4500
   tacaggatct gacattatta ttgttggaag aggactattt gcaaagggaa gggatgctaa 4560
   ggtagagggt gaacgttaca gaaaagcagg ctgggaagca tatttgagaa gatgcggcca 4620
   gcaaaactaa aaaactgtat tataagtaaa tgcatgtata ctaaactcac aaattagagc 4680
   ttcaatttaa ttatatcagt tattacccgg gaatctcggt cgtaatgatt tttataatga 4740
   cgaaaaaaaa aaattggaaa gaaaaccccc cccccccccc cccccccccc cccccccccg 4800
   cagcgttggg tcctggccac gggtgcgcat gatcgtgctc ctgtcgttga ggacccggct 4860
   aggctggcgg ggttgcctta ctggttagca gaatgaatca ccgatacgcg agcgaacgtg 4920
   aagcgactgc tgctgcaaaa cgtctgcgac ctgagcaaca acatgaatgg tcttcggttt 4980
   ccgtgtttcg taaagtctgg aaacgcggaa gtcagcgccc tgcaccatta tgttccggat 5040
   ctgcatcgca ggatgctgct ggctaccctg tggaacacct acatctgtat taacgaagcg 5100
   ctggcattga ccctgagtga tttttctctg gtcccgccgc atccataccg ccagttgttt 5160
   accctcacaa cgttccagta accgggcatg ttcatcatca gtaacccgta tcgtgagcat 5220
   cctctctcgt ttcatcggta tcattacccc catgaacaga aatccccctt acacggaggc 5280
   atcagtgacc aaacaggaaa aaaccgccct taacatggcc cgctttatca gaagccagac 5340
   attaacgctt ctggagaaac tcaacgagct ggacgcggat gaacaggcag acatctgtga 5400
   atcgcttcac gaccacgctg atgagcttta ccgcagctgc ctcgcgcgtt tcggtgatga 5460
   cggtgaaaac ctctgacaca tgcagctccc ggagacggtc acagcttgtc tgtaagcgga 5520
   tgccgggagc agacaagccc gtcagggcgc gtcagcgggt gttggcgggt gtcggggcgc 5580
   agccatgacc cagtcacgta gcgatagcgg agtgtatact ggcttaacta tgcggcatca 5640
   gagcagattg tactgagagt gcaccatatg cggtgtgaaa taccgcacag atgcgtaagg 5700
   agaaaatacc gcatcaggcg ctcttccgct tcctcgctca ctgactcgct gcgctcggtc 5760
   gttcggctgc ggcgagcggt atcagctcac tcaaaggcgg taatacggtt atccacagaa 5820
   tcaggggata acgcaggaaa gaacatgtga gcaaaaggcc agcaaaaggc caggaaccgt 5880
   aaaaaggccg cgttgctggc gtttttccat aggctccgcc cccctgacga gcatcacaaa 5940
   aatcgacgct caagtcagag gtggcgaaac ccgacaggac tataaagata ccaggcgttt 6000
   ccccctggaa gctccctcgt gcgctctcct gttccgaccc tgccgcttac cggatacctg 6060
   tccgcctttc tcccttcggg aagcgtggcg ctttctcata gctcacgctg taggtatctc 6120
   agttcggtgt aggtcgttcg ctccaagctg ggctgtgtgc acgaaccccc cgttcagccc 6180
   gaccgctgcg ccttatccgg taactatcgt cttgagtcca acccggtaag acacgactta 6240
   tcgccactgg cagcagccac tggtaacagg attagcagag cgaggtatgt aggcggtgct 6300
   acagagttct tgaagtggtg gcctaactac ggctacacta gaaggacagt atttggtatc 6360
   tgcgctctgc tgaagccagt taccttcgga aaaagagttg gtagctcttg atccggcaaa 6420
   caaaccaccg ctggtagcgg tggttttttt gtttgcaagc agcagattac gcgcagaaaa 6480
   aaaggatctc aagaagatcc tttgatcttt tctacggggt ctgacgctca gtggaacgaa 6540
   aactcacgtt aagggatttt ggtcatgaga ttatcaaaaa ggatcttcac ctagatcctt 6600
   ttaaattaaa aatgaagttt taaatcaatc taaagtatat atgagtaaac ttggtctgac 6660
   agttaccaat gcttaatcag tgaggcacct atctcagcga tctgtctatt tcgttcatcc 6720
   atagttgcct gactccccgt cgtgtagata actacgatac gggagggctt accatctggc 6780
   cccagtgctg caatgatacc gcgagaccca cgctcaccgg ctccagattt atcagcaata 6840
   aaccagccag ccggaagggc cgagcgcaga agtggtcctg caactttatc cgcctccatc 6900
   cagtctatta attgttgccg ggaagctaga gtaagtagtt cgccagttaa tagtttgcgc 6960
   aacgttgttg ccattgctgc aggcatcgtg gtgtcacgct cgtcgtttgg tatggcttca 7020
   ttcagctccg gttcccaacg atcaaggcga gttacatgat cccccatgtt gtgcaaaaaa 7080
   gcggttagct ccttcggtcc tccgatcgtt gtcagaagta agttggccgc agtgttatca     7140
   ctcatggtta tggcagcact gcataattct cttactgtca tgccatccgt aagatgcttt     7200
   tctgtgactg gtgagtactc aaccaagtca ttctgagaat agtgtatgcg gcgaccgagt     7260
   tgctcttgcc cggcgtcaac acgggataat accgcgccac atagcagaac tttaaaagtg     7320
   ctcatcattg gaaaacgttc ttcggggcga aaactctcaa ggatcttacc gctgttgaga     7380
   tccagttcga tgtaacccac tcgtgcaccc aactgatctt cagcatcttt tactttcacc     7440
   agcgtttctg ggtgagcaaa aacaggaagg caaaatgccg caaaaaaggg aataagggcg     7500
   acacggaaat gttgaatact catactcttc ctttttcaat attattgaag catttatcag     7560
   ggttattgtc tcatgagcgg atacatattt gaatgtattt agaaaaataa acaaataggg     7620
   gttccgcgca catttccccg aaaagtgcca cctgacgtct aagaaaccat tattatcatg     7680
   acattaacct ataaaaatag gcgtatcacg aggccctttc gtcttcaaga attg           7734
<210> 3
   <211> 7607
   <212> DNA
   <213> künstliche Sequenz
<220>
   <221> ampR
   <222> (212)..(1072)
   <223> á-lactamase
<220>
   <221> ori
   <222> (1217)..(1884)
<220>
   <221> ScURA3
   <222> (2969) .. (4126)
   <223> URA3 gene from S. cerevisiae
<220>
   <221> HARS1
   <222> (4926)..(5421)
   <223> HARS1 H.polymorpha
<220>
   <221> FMD-P
   <222> (5556)..(6621)
   <223> FMD-Promoter H.polymorpha
<220>
   <221> preCL
   <222> (6629)..(6682)
   <223> chicken lysozyme presequence
<220>
   <221> mhIL6
   <222> (6683)..(7237)
   <223> matures humanes Interleukin 6
<220>
   <221> H6
   <222> (7238)..(7255)
   <223> 6-Histidin-His-Tag
<220>
   <221> MOX-T
   <222> (7268)..(7595)
   <223> MOX-Terminator H.polymorpha
<400> 3
   caattcttga agacgaaagg gcctcgtgat acgcctattt ttataggtta atgtcatgat        60
   aataatggtt tcttagacgt caggtggcac ttttcgggga aatgtgcgcg gaacccctat       120
   ttgtttattt ttctaaatac attcaaatat gtatccgctc atgagacaat aaccctgata       180
   aatgcttcaa taatattgaa aaaggaagag tatgagtatt caacatttcc gtgtcgccct       240
   tattcccttt tttgcggcat tttgccttcc tgtttttgct cacccagaaa cgctggtgaa       300
   agtaaaagat gctgaagatc agttgggtgc acgagtgggt tacatcgaac tggatctcaa       360
   cagcggtaag atccttgaga gttttcgccc cgaagaacgt tttccaatga tgagcacttt       420
   taaagttctg ctatgtggcg cggtattatc ccgtgttgac gccgggcaag agcaactcgg       480
   tcgccgcata cactattctc agaatgactt ggttgagtac tcaccagtca cagaaaagca       540
   tcttacggat ggcatgacag taagagaatt atgcagtgct gccataacca tgagtgataa       600
   cactgcggcc aacttacttc tgacaacgat cggaggaccg aaggagctaa ccgctttttt       660
   gcacaacatg ggggatcatg taactcgcct tgatcgttgg gaaccggagc tgaatgaagc       720
   cataccaaac gacgagcgtg acaccacgat gcctgcagca atggcaacaa cgttgcgcaa       780
   actattaact ggcgaactac ttactctagc ttcccggcaa caattaatag actggatgga       840
   ggcggataaa gttgcaggac cacttctgcg ctcggccctt ccggctggct ggtttattgc       900
   tgataaatct ggagccggtg agcgtgggtc tcgcggtatc attgcagcac tggggccaga 960
   tggtaagccc tcccgtatcg tagttatcta cacgacgggg agtcaggcaa ctatggatga 1020
   acgaaataga cagatcgctg agataggtgc ctcactgatt aagcattggt aactgtcaga 1080
   ccaagtttac tcatatatac tttagattga tttaaaactt catttttaat ttaaaaggat 1140
   ctaggtgaag atcctttttg ataatctcat gaccaaaatc ccttaacgtg agttttcgtt 1200
   ccactgagcg tcagaccccg tagaaaagat caaaggatct tcttgagatc ctttttttct 1260
   gcgcgtaatc tgctgcttgc aaacaaaaaa accaccgcta ccagcggtgg tttgtttgcc 1320
   ggatcaagag ctaccaactc tttttccgaa ggtaactggc ttcagcagag cgcagatacc 1380
   aaatactgtc cttctagtgt agccgtagtt aggccaccac ttcaagaact ctgtagcacc 1440
   gcctacatac ctcgctctgc taatcctgtt accagtggct gctgccagtg gcgataagtc 1500
   gtgtcttacc gggttggact caagacgata gttaccggat aaggcgcagc ggtcgggctg 1560
   aacggggggt tcgtgcacac agcccagctt ggagcgaacg acctacaccg aactgagata 1620
   cctacagcgt gagctatgag aaagcgccac gcttcccgaa gggagaaagg cggacaggta 1680
   tccggtaagc ggcagggtcg gaacaggaga gcgcacgagg gagcttccag ggggaaacgc 1740
   ctggtatctt tatagtcctg tcgggtttcg ccacctctga cttgagcgtc gatttttgtg 1800
   atgctcgtca ggggggcgga gcctatggaa aaacgccagc aacgcggcct ttttacggtt 1860
   cctggccttt tgctggcctt ttgctcacat gttctttcct gcgttatccc ctgattctgt 1920
   ggataaccgt attaccgcct ttgagtgagc tgataccgct cgccgcagcc gaacgaccga 1980
   gcgcagcgag tcagtgagcg aggaagcgga agagcgcctg atgcggtatt ttctccttac 2040
   gcatctgtgc ggtatttcac accgcatatg gtgcactctc agtacaatct gctctgatgc 2100
   cgcatagtta agccagtata cactccgcta tcgctacgtg actgggtcat ggctgcgccc 2160
   cgacacccgc caacacccgc tgacgcgccc tgacgggctt gtctgctccc ggcatccgct 2220
   tacagacaag ctgtgaccgt ctccgggagc tgcatgtgtc agaggttttc accgtcatca 2280
   ccgaaacgcg cgaggcagct gcggtaaagc tcatcagcgt ggtcgtgaag cgattcacag 2340
   atgtctgcct gttcatccgc gtccagctcg ttgagtttct ccagaagcgt taatgtctgg 2400
   cttctgataa agcgggccat gttaagggcg gttttttcct gtttggtcac tgatgcctcc 2460
   gtgtaagggg gatttctgtt catgggggta atgataccga tgaaacgaga gaggatgctc 2520
   acgatacggg ttactgatga tgaacatgcc cggttactgg aacgttgtga gggtaaacaa 2580
   ctggcggtat ggatgcggcg ggaccagaga aaaatcactc agggtcaatg ccagcgcttc 2640
   gttaatacag atgtaggtgt tccacagggt agccagcagc atcctgcgat gcagatccgg 2700
   aacataatgg tgcagggcgc tgacttccgc gtttccagac tttacgaaac acggaaaccg 2760
   aagaccattc atgttgttgc tcaggtcgca gacgttttgc agcagcagtc gcttcacgtt 2820
   cgctcgcgta tcggtgattc attctgctaa ccagtaaggc aaccccgcca gcctagccgg 2880
   gtcctcaacg acaggagcac gatcatgcgc acccgtggcc aggacccaac gctgcggggg 2940
   gggggggggg gggggggggg gggggggggt tttctttcca attttttttt ttcgtcatta 3000
   taaaaatcat tacgaccgag attcccgggt aataactgat ataattaaat tgaagctcta 3060
   atttgtgagt ttagtataca tgcatttact tataatacag ttttttagtt ttgctggccg 3120
   catcttctca aatatgcttc ccagcctgct tttctgtaac gttcaccctc taccttagca 3180
   tcccttccct ttgcaaatag tcctcttcca acaataataa tgtcagatcc tgtagagacc 3240
   acatcatcca cggttctata ctgttgaccc aatgcgtctc ccttgtcatc taaacccaca 3300
   ccgggtgtca taatcaacca atcgtaacct tcatctcttc cacccatgtc tctttgagca 3360
   ataaagccga taacaaaatc tttgtcgctc ttcgcaatgt caacagtacc cttagtatat 3420
   tctccagtag atagggagcc cttgcatgac aattctgcta acatcaaaag gcctctaggt 3480
   tcctttgtta cttcttctgc cgcctgcttc aaaccgctaa caatacctgg gcccaccaca 3540
   ccgtgtgcat tcgtaatgtc tgcccattct gctattctgt atacacccgc agagtactgc 3600
   aatttgactg tattaccaat gtcagcaaat tttctgtctt cgaagagtaa aaaattgtac 3660
   ttggcggata atgcctttag cggcttaact gtgccctcca tggaaaaatc agtcaagata 3720
   tccacatgtg tttttagtaa acaaattttg ggacctaatg cttcaactaa ctccagtaat 3780
   tccttggtgg tacgaacatc caatgaagca cacaagtttg tttgcttttc gtgcatgata 3840
   ttaaatagct tggcagcaac aggactagga tgagtagcag cacgttcctt atatgtagct 3900
   ttcgacatga tttatcttcg tttcctgcag gtttttgttc tgtgcagttg ggttaagaat 3960
   actgggcaat ttcatgtttc ttcaacacta catatgcgta tatataccaa tctaagtctg 4020
   tgctccttcc ttcgttcttc cttctgttcg gagattaccg aatcaaaaaa atttcaagga 4080
   aaccgaaatc aaaaaaaaga ataaaaaaaa aatgatgaat tgaaaccccc cccccccccc 4140
   cccccccccg atgcgccgcg tgcggctgct ggagatggcg gacgcgatgg atatgttctg 4200
   ccaagggttg gtttgcgcat tcacagttct ccgcaagaat tgattggctc caattcttgg 4260
   agtggtgaat ccgttagcga ggtgccgccg gcttccattc aggtcgaggt ggcccggctc 4320
   catgcaccgc gacgcaacgc ggggaggcag acaaggtata gggcggcgcc tacaatccat 4380
   gccaacccgt tccatgtgct cgccgaggcg gcataaatcg ccgtgacgat cagcggtcca 4440
   atgatcgaag ttaggctggt aagagccgcg agcgatcctt gaagctgtcc ctgatggtcg 4500
   tcatctacct gcctggacag catggcctgc aacgcgggca tcccgatgcc gccggaagcg 4560
   agaagaatca taatggggaa ggccatccag cctcgcgtcg cgaacgccag caagacgtag 4620
   cccagcgcgt cggccgccat gccggcgata atggcctgct tctcgccgaa acgtttggtg 4680
   gcgggaccag tgacgaaggc ttgagcgagg gcgtgcaaga ttccgaatac cgcaagcgac 4740
   aggccgatca tcgtcgcgct ccagcgaaag cggtcctcgc cgaaaatgac ccagagcgct 4800
   gccggcacct gtcctacgag ttgcatgata aagaagacag tcataagtgc ggcgacgata 4860
   gtcatgcccc gcgcccaccg gaaggagctg actgggttga aggctctcaa gggcatcggt 4920
   cgactcccgc gactcggcgt tcactttcga gctattatca acgccggaat acgtcagaaa 4980
   cagccgtgcc ccagggacca gaaagcctac tggtgagtat gttctttcgt gtgatttttc 5040
   cgaggatgag aacgacgata acgagcacaa ctcggagtcg gaggacacgc ttattgcgtt 5100
   gaacgcagcc aacatcagca ggctgtcaag actgagtatg gccacagagc tggatttctc 5160
   ggcctcatac tcaaagacgt ttagtaaact ccgtctgcca gaaattgctg acgaggatgt 5220
   ataataatag atgaattacg aacaattgta gttcaaaaaa atttagtaac aatattgtct 5280
   agatgacaga tttgctgaaa ccagtgaact ccaataaacc actcaccgct acccaagaga 5340
   aacagatcag agtgctaggg cttgtttcag agtactacaa cgtttaccag aagcttgagc 5400
   aagttctcaa acgcgggttt gtcgacgctc tcccttatgc gactcctgca ttaggaagca 5460
   gcccagtagt aggttgaggc cgttgagcac cgccgccgca aggaatggtg tcgacgcgga 5520
   gaacgatctc ctcgacgcgg agaacgatct cctcgagatc caggaagcgc aacgacgcgg 5580
   ggaatttcgt aatcttcccc gaggaaaacc gcttcccaaa gatttcaccg gcaataacgc 5640
   cttcattgac cgcacagagt accacttgaa caacattctg aaacggcagg aggcggttcc 5700
   gccgtggata gagaaacagg gcagtgtgga ggctcagatc cgccagtttc ggcgccagct 5760
   cgactccgat tggacactgc aggccgtgca catcgtttgc gagaaacact cgaagaaaag 5820
   cgacacggac aagatccaga tcatgagcca gtacgccgag gcagagcgca acaataactc 5880
   gagttatacg aagctgcgca gcagccaatg ggaacagaaa tacagcaact atctggaggt 5940
   caaattaaaa gcgctgaatg acagcatccg aggttataat ctgcaggctc ctttggcatc 6000
   gcagaaactg tatctaaacg caaactccga gctggaaaaa tgttaccggc gatgcgcgga 6060
   caatttagag gcggcgatca agaaacacct gctgggcgag cagtctggag cacagtcttc 6120
   gatgggcccg agatcccacc gcgttcctgg gtaccgggac gtgaggcagc gcgacatcca 6180
   tcaaatatac caggcgccaa ccgagtctct cggaaaacag cttctggata tcttccgctg 6240
   gcggcgcaac gacgaataat agtccctgga ggtgacggaa tatatatgtg tggagggtaa 6300
   atctgacagg gtgtagcaaa ggtaatattt tcctaaaaca tgcaatcggc tgccccgcaa 6360
   cgggaaaaag aatgactttg gcactcttca ccagagtggg gtgtcccgct cgtgtgtgca 6420
   aataggctcc cactggtcac cccggatttt gcagaaaaac agcaagttcc ggggtgtctc 6480
   actggtgtcc gccaataaga ggagccggca ggcacggagt ctacatcaag ctgtctccga 6540
   tacactcgac taccatccgg gtctctcaga gaggggaatg gcactataaa taccgcctcc 6600
   ttgcgctctc tgccttcatc gggaattcat gagatccctg ctgatactag tgctgtgttt 6660
   cttgccgcta gctgccttgg gcgctcctgt tcctcctggt gaagattcta aggacgtcgc 6720
   tgctcctcac agacagcctt tgacctcttc tgagcgcatc gataagcaga ttcgctacat 6780
   tctggacgga atttctgccc tgagaaagga aacctgcaac aagtctaaca tgtgcgagtc 6840
   ctctaaggaa gctctggctg agaacaacct gaacctgcct aagatggctg agaaggacgg 6900
   atgtttccag tctggattca acgaggaaac ctgcctggtc aagattatta ccggactgtt 6960
   ggagttcgag gtctacttgg agtacctgca gaacagattc gagtcctctg aagaacaggc 7020
   tagagctgtc cagatgtcta ccaaggtcct gattcagttc ctgcagaaga aggctaagaa 7080
   cctggacgct attactactc ctgaccctac cactaacgct tctctgctga ctaagctgca 7140
   ggctcagaat cagtggttgc aggacatgac cactcacctg atcctgagat ccttcaagga     7200
   atttctgcag tcctctctga gagctttgag acagatgcac catcatcacc accactaata     7260
   aggatccccc tcgcggactt gccagattct aaggagacgt ggaaggacat accgcttttg     7320
   agaagcgtgt ttgaaaatag ttctttttct ggtttatatc gtttatgaag tgatgagatg     7380
   aaaagctgaa atagcgagta taggaaaatt taatgaaaat taaattaaat attttcttag     7440
   gctattagtc accttcaaaa tgccggccgc ttctaagaac gttgtcatga tcgacaacta     7500
   cgactcgttt acctggaacc tgtacgagta cctgtgtcag gagggagcca atgtcgaggt     7560
   tttcaggaac gatcagatca ccattccgga gattgagcag gggtacc                   7607
<210> 4
   <211> 7547
   <212> DNA
   <213> künstliche Sequenz
<220>
   <221> ampR
   <222> (212)..(1072)
   <223> á-lactamase
<220>
   <221> ori
   <222> (1072)..(1884)
<220>
   <221> ScURA3
   <222> (2969)..(4126)
   <223> URA3 gene from S. cerevisiae
<220>
   <221> HARS1
   <222> (9926)..(5421)
   <223> HARS1 H.polymorpha
<220>
   <221> FMD-P
   <222> (5556)..(6621)
   <223> FMD-Promoter H.polymorpha
<220>
   <221> FTH1
   <222> (6629)..(7177)
<220>
   <221> H6
   <222> (7178)..(7195)
   <223> 6-Hhistidin-His-Tag
<220>
   <221> MOX-T
   <222> (7208)..(7535)
   <223> MOX-Terminator H.polymorpha
<400> 4
   caattcttga agacgaaagg gcctcgtgat acgcctattt ttataggtta atgtcatgat      60
   aataatggtt tcttagacgt caggtggcac ttttcgggga aatgtgcgcg gaacccctat     120
   ttgtttattt ttctaaatac attcaaatat gtatccgctc atgagacaat aaccctgata     180
   aatgcttcaa taatattgaa aaaggaagag tatgagtatt caacatttcc gtgtcgccct     240
   tattcccttt tttgcggcat tttgccttcc tgtttttgct cacccagaaa cgctggtgaa     300
   agtaaaagat gctgaagatc agttgggtgc acgagtgggt tacatcgaac tggatctcaa     360
   cagcggtaag atccttgaga gttttcgccc cgaagaacgt tttccaatga tgagcacttt     420
   taaagttctg ctatgtggcg cggtattatc ccgtgttgac gccgggcaag agcaactcgg     480
   tcgccgcata cactattctc agaatgactt ggttgagtac tcaccagtca cagaaaagca     540
   tcttacggat ggcatgacag taagagaatt atgcagtgct gccataacca tgagtgataa     600
   cactgcggcc aacttacttc tgacaacgat cggaggaccg aaggagctaa ccgctttttt     660
   gcacaacatg ggggatcatg taactcgcct tgatcgttgg gaaccggagc tgaatgaagc     720
   cataccaaac gacgagcgtg acaccacgat gcctgcagca atggcaacaa cgttgcgcaa     780
   actattaact ggcgaactac ttactctagc ttcccggcaa caattaatag actggatgga     840
   ggcggataaa gttgcaggac cacttctgcg ctcggccctt ccggctggct ggtttattgc     900
   tgataaatct ggagccggtg agcgtgggtc tcgcggtatc attgcagcac tggggccaga     960
   tggtaagccc tcccgtatcg tagttatcta cacgacgggg agtcaggcaa ctatggatga    1020
   acgaaataga cagatcgctg agataggtgc ctcactgatt aagcattggt aactgtcaga    1080
   ccaagtttac tcatatatac tttagattga tttaaaactt catttttaat ttaaaaggat    1140
   ctaggtgaag atcctttttg ataatctcat gaccaaaatc ccttaacgtg agttttcgtt    1200
   ccactgagcg tcagaccccg tagaaaagat caaaggatct tcttgagatc cttttttctt    1260
   gcgcgtaatc tgctgcttgc aaacaaaaaa accaccgcta ccagcggtgg tttgtttgcc 1320
   ggatcaagag ctaccaactc tttttccgaa ggtaactggc ttcagcagag cgcagatacc 1380
   aaatactgtc cttctagtgt agccgtagtt aggccaccac ttcaagaact ctgtagcacc 1440
   gcctacatac ctcgctctgc taatcctgtt accagtggct gctgccagtg gcgataagtc 1500
   gtgtcttacc gggttggact caagacgata gttaccggat aaggcgcagc ggtcgggctg 1560
   aacggggggt tcgtgcacac agcccagctt ggagcgaacg acctacaccg aactgagata 1620
   cctacagcgt gagctatgag aaagcgccac gcttcccgaa gggagaaagg cggacaggta 1680
   tccggtaagc ggcagggtcg gaacaggaga gcgcacgagg gagcttccag ggggaaacgc 1740
   ctggtatctt tatagtcctg tcgggtttcg ccacctctga cttgagcgtc gatttttgtg 1800
   atgctcgtca ggggggcgga gcctatggaa aaacgccagc aacgcggcct ttttacggtt 1860
   cctggccttt tgctggcctt ttgctcacat gttctttcct gcgttatccc ctgattctgt 1920
   ggataaccgt attaccgcct ttgagtgagc tgataccgct cgccgcagcc gaacgaccga 1980
   gcgcagcgag tcagtgagcg aggaagcgga agagcgcctg atgcggtatt ttctccttac 2040
   gcatctgtgc ggtatttcac accgcatatg gtgcactctc agtacaatct gctctgatgc 2100
   cgcatagtta agccagtata cactccgcta tcgctacgtg actgggtcat ggctgcgccc 2160
   cgacacccgc caacacccgc tgacgcgccc tgacgggctt gtctgctccc ggcatccgct 2220
   tacagacaag ctgtgaccgt ctccgggagc tgcatgtgtc agaggttttc accgtcatca 2280
   ccgaaacgcg cgaggcagct gcggtaaagc tcatcagcgt ggtcgtgaag cgattcacag 2340
   atgtctgcct gttcatccgc gtccagctcg ttgagtttct ccagaagcgt taatgtctgg 2400
   cttctgataa agcgggccat gttaagggcg gttttttcct gtttggtcac tgatgcctcc 2460
   gtgtaagggg gatttctgtt catgggggta atgataccga tgaaacgaga gaggatgctc 2520
   acgatacggg ttactgatga tgaacatgcc cggttactgg aacgttgtga gggtaaacaa 2580
   ctggcggtat ggatgcggcg ggaccagaga aaaatcactc agggtcaatg ccagcgcttc 2640
   gttaatacag atgtaggtgt tccacagggt agccagcagc atcctgcgat gcagatccgg 2700
   aacataatgg tgcagggcgc tgacttccgc gtttccagac tttacgaaac acggaaaccg 2760
   aagaccattc atgttgttgc tcaggtcgca gacgttttgc agcagcagtc gcttcacgtt 2820
   cgctcgcgta tcggtgattc attctgctaa ccagtaaggc aaccccgcca gcctagccgg 2880
   gtcctcaacg acaggagcac gatcatgcgc acccgtggcc aggacccaac gctgcggggg 2940
   gggggggggg gggggggggg gggggggggt tttctttcca attttttttt ttcgtcatta 3000
   taaaaatcat tacgaccgag attcccgggt aataactgat ataattaaat tgaagctcta 3060
   atttgtgagt ttagtataca tgcatttact tataatacag ttttttagtt ttgctggccg 3120
   catcttctca aatatgcttc ccagcctgct tttctgtaac gttcaccctc taccttagca 3180
   tcccttccct ttgcaaatag tcctcttcca acaataataa tgtcagatcc tgtagagacc 3240
   acatcatcca cggttctata ctgttgaccc aatgcgtctc ccttgtcatc taaacccaca 3300
   ccgggtgtca taatcaacca atcgtaacct tcatctcttc cacccatgtc tctttgagca 3360
   ataaagccga taacaaaatc tttgtcgctc ttcgcaatgt caacagtacc cttagtatat 3420
   tctccagtag atagggagcc cttgcatgac aattctgcta acatcaaaag gcctctaggt 3480
   tcctttgtta cttcttctgc cgcctgcttc aaaccgctaa caatacctgg gcccaccaca 3540
   ccgtgtgcat tcgtaatgtc tgcccattct gctattctgt atacacccgc agagtactgc 3600
   aatttgactg tattaccaat gtcagcaaat ttctgtcttt cgaagagtaa aaaattgtac 3660
   ttggcggata atgcctttag cggcttaact gtgccctcca tggaaaaatc agtcaagata 3720
   tccacatgtg tttttagtaa acaaattttg ggacctaatg cttcaactaa ctccagtaat 3780
   tccttggtgg tacgaacatc caatgaagca cacaagtttg tttgcttttc gtgcatgata 3840
   ttaaatagct tggcagcaac aggactagga tgagtagcag cacgttcctt atatgtagct 3900
   ttcgacatga tttatcttcg tttcctgcag gtttttgttc tgtgcagttg ggttaagaat 3960
   actgggcaat ttcatgtttc ttcaacacta catatgcgta tatataccaa tctaagtctg 4020
   tgctccttcc ttcgttcttc cttctgttcg gagattaccg aatcaaaaaa atttcaagga 4080
   aaccgaaatc aaaaaaaaga ataaaaaaaa aatgatgaat tgaaaccccc cccccccccc 4140
   cccccccccg atgcgccgcg tgcggctgct ggagatggcg gacgcgatgg atatgttctg 4200
   ccaagggttg gtttgcgcat tcacagttct ccgcaagaat tgattggctc caattcttgg 4260
   agtggtgaat ccgttagcga ggtgccgccg gcttccattc aggtcgaggt ggcccggctc 4320
   catgcaccgc gacgcaacgc ggggaggcag acaaggtata gggcggcgcc tacaatccat 4380
   gccaacccgt tccatgtgct cgccgaggcg gcataaatcg ccgtgacgat cagcggtcca 4440
   atgatcgaag ttaggctggt aagagccgcg agcgatcctt gaagctgtcc ctgatggtcg 4500
   tcatctacct gcctggacag catggcctgc aacgcgggca tcccgatgcc gccggaagcg 4560
   agaagaatca taatggggaa ggccatccag cctcgcgtcg cgaacgccag caagacgtag 4620
   cccagcgcgt cggccgccat gccggcgata atggcctgct tctcgccgaa acgtttggtg 4680
   gcgggaccag tgacgaaggc ttgagcgagg gcgtgcaaga ttccgaatac cgcaagcgac 4740
   aggccgatca tcgtcgcgct ccagcgaaag cggtcctcgc cgaaaatgac ccagagcgct 4800
   gccggcacct gtcctacgag ttgcatgata aagaagacag tcataagtgc ggcgacgata 4860
   gtcatgcccc gcgcccaccg gaaggagctg actgggttga aggctctcaa gggcatcggt 4920
   cgactcccgc gactcggcgt tcactttcga gctattatca acgccggaat acgtcagaaa 4980
   cagccgtgcc ccagggacca gaaagcctac tggtgagtat gttctttcgt gtgatttttc 5040
   cgaggatgag aacgacgata acgagcacaa ctcggagtcg gaggacacgc ttattgcgtt 5100
   gaacgcagcc aacatcagca ggctgtcaag actgagtatg gccacagagc tggatttctc 5160
   ggcctcatac tcaaagacgt ttagtaaact ccgtctgcca gaaattgctg acgaggatgt 5220
   ataataatag atgaattacg aacaattgta gttcaaaaaa atttagtaac aatattgtct 5280
   agatgacaga tttgctgaaa ccagtgaact ccaataaacc actcaccgct acccaagaga 5340
   aacagatcag agtgctaggg cttgtttcag agtactacaa cgtttaccag aagcttgagc 5400
   aagttctcaa acgcgggttt gtcgacgctc tcccttatgc gactcctgca ttaggaagca 5460
   gcccagtagt aggttgaggc cgttgagcac cgccgccgca aggaatggtg tcgacgcgga 5520
   gaacgatctc ctcgacgcgg agaacgatct cctcgagatc caggaagcgc aacgacgcgg 5580
   ggaatttcgt aatcttcccc gaggaaaacc gcttcccaaa gatttcaccg gcaataacgc 5640
   cttcattgac cgcacagagt accacttgaa caacattctg aaacggcagg aggcggttcc 5700
   gccgtggata gagaaacagg gcagtgtgga ggctcagatc cgccagtttc ggcgccagct 5760
   cgactccgat tggacactgc aggccgtgca catcgtttgc gagaaacact cgaagaaaag 5820
   cgacacggac aagatccaga tcatgagcca gtacgccgag gcagagcgca acaataactc 5880
   gagttatacg aagctgcgca gcagccaatg ggaacagaaa tacagcaact atctggaggt 5940
   caaattaaaa gcgctgaatg acagcatccg aggttataat ctgcaggctc ctttggcatc 6000
   gcagaaactg tatctaaacg caaactccga gctggaaaaa tgttaccggc gatgcgcgga 6060
   caatttagag gcggcgatca agaaacacct gctgggcgag cagtctggag cacagtcttc 6120
   gatgggcccg agatcccacc gcgttcctgg gtaccgggac gtgaggcagc gcgacatcca 6180
   tcaaatatac caggcgccaa ccgagtctct cggaaaacag cttctggata tcttccgctg 6240
   gcggcgcaac gacgaataat agtccctgga ggtgacggaa tatatatgtg tggagggtaa 6300
   atctgacagg gtgtagcaaa ggtaatattt tcctaaaaca tgcaatcggc tgccccgcaa 6360
   cgggaaaaag aatgactttg gcactcttca ccagagtggg gtgtcccgct cgtgtgtgca 6420
   aataggctcc cactggtcac cccggatttt gcagaaaaac agcaagttcc ggggtgtctc 6480
   actggtgtcc gccaataaga ggagccggca ggcacggagt ctacatcaag ctgtctccga 6540
   tacactcgac taccatccgg gtctctcaga gaggggaatg gcactataaa taccgcctcc 6600
   ttgcgctctc tgccttcatc gggaattcat gaccaccgct tctacctctc aggtcagaca 6660
   gaactaccac caggattctg aggctgctat taaccgccag atcaacttgg agctgtacgc 6720
   ttcttacgtc tacctgtcca tgtcttacta cttcgaccgt gacgacgttg ctttgaagaa 6780
   cttcgccaag tacttcttgc accagtctca cgaggaaaga gagcacgctg agaagctgat 6840
   gaagctgcag aatcagagag gtggaagaat cttcttgcag gacatcaaga agcctgattg 6900
   cgacgattgg gagtctggac tgaacgctat ggaatgcgct ttgcacttgg agaagaacgt 6960
   caaccagtct ctgttggagt tgcacaagct ggctactgac aagaacgacc ctcacctgtg 7020
   cgactttatc gagactcact acctgaacga gcaggtcaag gctattaagg aactgggtga 7080
   ccacgttacc aacctgagaa agatgggtgc tcctgagtct ggattggctg agtacctgtt 7140
   cgacaagcac actttgggag actctgataa cgaatctcac catcatcacc accactaata 7200
   aggatccccc tcgcggactt gccagattct aaggagacgt ggaaggacat accgcttttg 7260
   agaagcgtgt ttgaaaatag ttctttttct ggtttatatc gtttatgaag tgatgagatg 7320
   aaaagctgaa atagcgagta taggaaaatt taatgaaaat taaattaaat attttcttag 7380
   gctattagtc accttcaaaa tgccggccgc ttctaagaac gttgtcatga tcgacaacta 7440
   cgactcgttt acctggaacc tgtacgagta cctgtgtcag gagggagcca atgtcgaggt 7500
   tttcaggaac gatcagatca ccattccgga gattgagcag gggtacc                  7547
<210> 5
   <211> 8771
   <212> DNA
   <213> künstliche Sequenz
<220>
   <221> ampR
   <222> (212)..(1072)
   <223> á-lactamase
<220>
   <221> ori
   <222> (1217)..(1884)
<220>
   <221> ScURA3
   <222> (2969)..(4126)
   <223> URA3 gene from S. cerevisiae
<220>
   <221> HARS1
   <222> (4926)..(5421)
   <223> HARS1 H.polymorpha
<220>
   <221> FMD-P
   <222> (5556)..(6621)
   <223> FMD-Promoter H.polymorpha
<220>
   <221> FTH1
   <222> (6629)..(7176)
   <223> humanes Ferritin (H-Kette)
<220>
   <221> G3
   <222> (7178) .. (7186)
   <223> G3-Linker
<220>
   <221> Ek
   <222> (7187)..(7201)
   <223> Enteropeptidase-Spaltstelle
<220>
   <221> mhACT
   <222> (7202)..(8401)
   <223> maturer humaner alphal-Antichymotrypsin-Precursor
<220>
   <221> H6
   <222> (8402)..(8419)
   <223> 6-Histidin-His-Tag
<220>
   <221> MOX-T
   <222> (8432)..(8759)
   <223> MOX-Terminator H.polymorpha
<400> 5
   caattcttga agacgaaagg gcctcgtgat acgcctattt ttataggtta atgtcatgat      60
   aataatggtt tcttagacgt caggtggcac ttttcgggga aatgtgcgcg gaacccctat     120
   ttgtttattt ttctaaatac attcaaatat gtatccgctc atgagacaat aaccctgata     180
   aatgcttcaa taatattgaa aaaggaagag tatgagtatt caacatttcc gtgtcgccct     240
   tattcccttt tttgcggcat tttgccttcc tgtttttgct cacccagaaa cgctggtgaa     300
   agtaaaagat gctgaagatc agttgggtgc acgagtgggt tacatcgaac tggatctcaa     360
   cagcggtaag atccttgaga gttttcgccc cgaagaacgt tttccaatga tgagcacttt     420
   taaagttctg ctatgtggcg cggtattatc ccgtgttgac gccgggcaag agcaactcgg     480
   tcgccgcata cactattctc agaatgactt ggttgagtac tcaccagtca cagaaaagca     540
   tcttacggat ggcatgacag taagagaatt atgcagtgct gccataacca tgagtgataa     600
   cactgcggcc aacttacttc tgacaacgat cggaggaccg aaggagctaa ccgctttttt     660
   gcacaacatg ggggatcatg taactcgcct tgatcgttgg gaaccggagc tgaatgaagc     720
   cataccaaac gacgagcgtg acaccacgat gcctgcagca atggcaacaa cgttgcgcaa     780
   actattaact ggcgaactac ttactctagc ttcccggcaa caattaatag actggatgga     840
   ggcggataaa gttgcaggac cacttctgcg ctcggccctt ccggctggct ggtttattgc     900
   tgataaatct ggagccggtg agcgtgggtc tcgcggtatc attgcagcac tggggccaga     960
   tggtaagccc tcccgtatcg tagttatcta cacgacgggg agtcaggcaa ctatggatga    1020
   acgaaataga cagatcgctg agataggtgc ctcactgatt aagcattggt aactgtcaga    1080
   ccaagtttac tcatatatac tttagattga tttaaaactt catttttaat ttaaaaggat    1140
   ctaggtgaag atcctttttg ataatctcat gaccaaaatc ccttaacgtg agttttcgtt    1200
   ccactgagcg tcagaccccg tagaaaagat caaaggatct tcttgagatc ctttttttct 1260
   gcgcgtaatc tgctgcttgc aaacaaaaaa accaccgcta ccagcggtgg tttgtttgcc 1320
   ggatcaagag ctaccaactc tttttccgaa ggtaactggc ttcagcagag cgcagatacc 1380
   aaatactgtc cttctagtgt agccgtagtt aggccaccac ttcaagaact ctgtagcacc 1440
   gcctacatac ctcgctctgc taatcctgtt accagtggct gctgccagtg gcgataagtc 1500
   gtgtcttacc gggttggact caagacgata gttaccggat aaggcgcagc ggtcgggctg 1560
   aacggggggt tcgtgcacac agcccagctt ggagcgaacg acctacaccg aactgagata 1620
   cctacagcgt gagctatgag aaagcgccac gcttcccgaa gggagaaagg cggacaggta 1680
   tccggtaagc ggcagggtcg gaacaggaga gcgcacgagg gagcttccag ggggaaacgc 1740
   ctggtatctt tatagtcctg tcgggtttcg ccacctctga cttgagcgtc gatttttgtg 1800
   atgctcgtca ggggggcgga gcctatggaa aaacgccagc aacgcggcct ttttacggtt 1860
   cctggccttt tgctggcctt ttgctcacat gttctttcct gcgttatccc ctgattctgt 1920
   ggataaccgt attaccgcct ttgagtgagc tgataccgct cgccgcagcc gaacgaccga 1980
   gcgcagcgag tcagtgagcg aggaagcgga agagcgcctg atgcggtatt ttctccttac 2040
   gcatctgtgc ggtatttcac accgcatatg gtgcactctc agtacaatct gctctgatgc 2100
   cgcatagtta agccagtata cactccgcta tcgctacgtg actgggtcat ggctgcgccc 2160
   cgacacccgc caacacccgc tgacgcgccc tgacgggctt gtctgctccc ggcatccgct 2220
   tacagacaag ctgtgaccgt ctccgggagc tgcatgtgtc agaggttttc accgtcatca 2280
   ccgaaacgcg cgaggcagct gcggtaaagc tcatcagcgt ggtcgtgaag cgattcacag 2340
   atgtctgcct gttcatccgc gtccagctcg ttgagtttct ccagaagcgt taatgtctgg 2400
   cttctgataa agcgggccat gttaagggcg gttttttcct gtttggtcac tgatgcctcc 2460
   gtgtaagggg gatttctgtt catgggggta atgataccga tgaaacgaga gaggatgctc 2520
   acgatacggg ttactgatga tgaacatgcc cggttactgg aacgttgtga gggtaaacaa 2580
   ctggcggtat ggatgcggcg ggaccagaga aaaatcactc agggtcaatg ccagcgcttc 2640
   gttaatacag atgtaggtgt tccacagggt agccagcagc atcctgcgat gcagatccgg 2700
   aacataatgg tgcagggcgc tgacttccgc gtttccagac tttacgaaac acggaaaccg 2760
   aagaccattc atgttgttgc tcaggtcgca gacgttttgc agcagcagtc gcttcacgtt 2820
   cgctcgcgta tcggtgattc attctgctaa ccagtaaggc aaccccgcca gcctagccgg 2880
   gtcctcaacg acaggagcac gatcatgcgc acccgtggcc aggacccaac gctgcggggg 2940
   gggggggggg gggggggggg gggggggggt tttctttcca attttttttt ttcgtcatta 3000
   taaaaatcat tacgaccgag attcccgggt aataactgat ataattaaat tgaagctcta 3060
   atttgtgagt ttagtataca tgcatttact tataatacag ttttttagtt ttgctggccg 3120
   catcttctca aatatgcttc ccagcctgct tttctgtaac gttcaccctc taccttagca 3180
   tcccttccct ttgcaaatag tcctcttcca acaataataa tgtcagatcc tgtagagacc 3240
   acatcatcca cggttctata ctgttgaccc aatgcgtctc ccttgtcatc taaacccaca 3300
   ccgggtgtca taatcaacca atcgtaacct tcatctcttc cacccatgtc tctttgagca 3360
   ataaagccga taacaaaatc tttgtcgctc ttcgcaatgt caacagtacc cttagtatat 3420
   tctccagtag atagggagcc cttgcatgac aattctgcta acatcaaaag gcctctaggt 3480
   tcctttgtta cttcttctgc cgcctgcttc aaaccgctaa caatacctgg gcccaccaca 3540
   ccgtgtgcat tcgtaatgtc tgcccattct gctattctgt atacacccgc agagtactgc 3600
   aatttgactg tattaccaat gtcagcaaat tttctgtctt cgaagagtaa aaaattgtac 3660
   ttggcggata atgcctttag cggcttaact gtgccctcca tggaaaaatc agtcaagata 3720
   tccacatgtg tttttagtaa acaaattttg ggacctaatg cttcaactaa ctccagtaat 3780
   tccttggtgg tacgaacatc caatgaagca cacaagtttg tttgcttttc gtgcatgata 3840
   ttaaatagct tggcagcaac aggactagga tgagtagcag cacgttcctt atatgtagct 3900
   ttcgacatga tttatcttcg tttcctgcag gtttttgttc tgtgcagttg ggttaagaat 3960
   actgggcaat ttcatgtttc ttcaacacta catatgcgta tatataccaa tctaagtctg 4020
   tgctccttcc ttcgttcttc cttctgttcg gagattaccg aatcaaaaaa atttcaagga 4080
   aaccgaaatc aaaaaaaaga ataaaaaaaa aatgatgaat tgaaaccccc cccccccccc 4140
   cccccccccg atgcgccgcg tgcggctgct ggagatggcg gacgcgatgg atatgttctg 4200
   ccaagggttg gtttgcgcat tcacagttct ccgcaagaat tgattggctc caattcttgg 4260
   agtggtgaat ccgttagcga ggtgccgccg gcttccattc aggtcgaggt ggcccggctc 4320
   catgcaccgc gacgcaacgc ggggaggcag acaaggtata gggcggcgcc tacaatccat 4380
   gccaacccgt tccatgtgct cgccgaggcg gcataaatcg ccgtgacgat cagcggtcca 4440
   atgatcgaag ttaggctggt aagagccgcg agcgatcctt gaagctgtcc ctgatggtcg 4500
   tcatctacct gcctggacag catggcctgc aacgcgggca tcccgatgcc gccggaagcg 4560
   agaagaatca taatggggaa ggccatccag cctcgcgtcg cgaacgccag caagacgtag 4620
   cccagcgcgt cggccgccat gccggcgata atggcctgct tctcgccgaa acgtttggtg 4680
   gcgggaccag tgacgaaggc ttgagcgagg gcgtgcaaga ttccgaatac cgcaagcgac 4740
   aggccgatca tcgtcgcgct ccagcgaaag cggtcctcgc cgaaaatgac ccagagcgct 4800
   gccggcacct gtcctacgag ttgcatgata aagaagacag tcataagtgc ggcgacgata 4860
   gtcatgcccc gcgcccaccg gaaggagctg actgggttga aggctctcaa gggcatcggt 4920
   cgactcccgc gactcggcgt tcactttcga gctattatca acgccggaat acgtcagaaa 4980
   cagccgtgcc ccagggacca gaaagcctac tggtgagtat gttctttcgt gtgatttttc 5040
   cgaggatgag aacgacgata acgagcacaa ctcggagtcg gaggacacgc ttattgcgtt 5100
   gaacgcagcc aacatcagca ggctgtcaag actgagtatg gccacagagc tggatttctc 5160
   ggcctcatac tcaaagacgt ttagtaaact ccgtctgcca gaaattgctg acgaggatgt 5220
   ataataatag atgaattacg aacaattgta gttcaaaaaa atttagtaac aatattgtct 5280
   agatgacaga tttgctgaaa ccagtgaact ccaataaacc actcaccgct acccaagaga 5340
   aacagatcag agtgctaggg cttgtttcag agtactacaa cgtttaccag aagcttgagc 5400
   aagttctcaa acgcgggttt gtcgacgctc tcccttatgc gactcctgca ttaggaagca 5460
   gcccagtagt aggttgaggc cgttgagcac cgccgccgca aggaatggtg tcgacgcgga 5520
   gaacgatctc ctcgacgcgg agaacgatct cctcgagatc caggaagcgc aacgacgcgg 5580
   ggaatttcgt aatcttcccc gaggaaaacc gcttcccaaa gatttcaccg gcaataacgc 5640
   cttcattgac cgcacagagt accacttgaa caacattctg aaacggcagg aggcggttcc 5700
   gccgtggata gagaaacagg gcagtgtgga ggctcagatc cgccagtttc ggcgccagct 5760
   cgactccgat tggacactgc aggccgtgca catcgtttgc gagaaacact cgaagaaaag 5820
   cgacacggac aagatccaga tcatgagcca gtacgccgag gcagagcgca acaataactc 5880
   gagttatacg aagctgcgca gcagccaatg ggaacagaaa tacagcaact atctggaggt 5940
   caaattaaaa gcgctgaatg acagcatccg aggttataat ctgcaggctc ctttggcatc 6000
   gcagaaactg tatctaaacg caaactccga gctggaaaaa tgttaccggc gatgcgcgga 6060
   caatttagag gcggcgatca agaaacacct gctgggcgag cagtctggag cacagtcttc 6120
   gatgggcccg agatcccacc gcgttcctgg gtaccgggac gtgaggcagc gcgacatcca 6180
   tcaaatatac caggcgccaa ccgagtctct cggaaaacag cttctggata tcttccgctg 6240
   gcggcgcaac gacgaataat agtccctgga ggtgacggaa tatatatgtg tggagggtaa 6300
   atctgacagg gtgtagcaaa ggtaatattt tcctaaaaca tgcaatcggc tgccccgcaa 6360
   cgggaaaaag aatgactttg gcactcttca ccagagtggg gtgtcccgct cgtgtgtgca 6420
   aataggctcc cactggtcac cccggatttt gcagaaaaac agcaagttcc ggggtgtctc 6480
   actggtgtcc gccaataaga ggagccggca ggcacggagt ctacatcaag ctgtctccga 6540
   tacactcgac taccatccgg gtctctcaga gaggggaatg gcactataaa taccgcctcc 6600
   ttgcgctctc tgccttcatc gggaattcat gaccaccgct tctacctctc aggtcagaca 6660
   gaactaccac caggattctg aggctgctat taaccgccag atcaacttgg agctgtacgc 6720
   ttcttacgtc tacctgtcca tgtcttacta cttcgaccgt gacgacgttg ctttgaagaa 6780
   cttcgccaag tacttcttgc accagtctca cgaggaaaga gagcacgctg agaagctgat 6840
   gaagctgcag aatcagagag gtggaagaat cttcttgcag gacatcaaga agcctgattg 6900
   cgacgattgg gagtctggac tgaacgctat ggaatgcgct ttgcacttgg agaagaacgt 6960
   caaccagtct ctgttggagt tgcacaagct ggctactgac aagaacgacc ctcacctgtg 7020
   cgactttatc gagactcact acctgaacga gcaggtcaag gctattaagg aactgggtga 7080
   ccacgttacc aacctgagaa agatgggtgc tcctgagtct ggattggctg agtacctgtt 7140
   cgacaagcac actttgggag actctgataa cgaatctgga ggtggagacg atgatgacaa 7200
   gcaccctaac tcgcctttgg atgaggaaaa cctgactcag gaaaaccagg atagaggaac 7260
   tcacgtagat ctgggattgg cttctgctaa cgtggacttc gctttctcgc tgtacaagca 7320
   gctggttttg aaggctcctg acaagaacgt gatcttctct cctctgtcta tttctaccgc 7380
   tctggctttc ttgtctctgg gtgctcacaa cactaccctg accgagattc tgaagggact 7440
   gaagttcaac ctgaccgaga cttccgaggc tgagattcac cagtctttcc agcacctgct 7500
   gagaactttg aaccagtcct ctgacgagtt gcagctgtct atgggaaacg ctatgttcgt 7560
   taaggaacag ctgtctctgt tggacagatt caccgaggac gctaagagat tgtacggatc 7620
   tgaggctttc gctactgact ttcaggattc tgctgctgcc aagaagctga ttaacgacta 7680
   cgtcaagaac ggaaccagag gaaagattac cgacctgatc aaggacttgg actctcagac 7740
   catgatggtc ctggtcaact acattttctt caaggccaag tgggagatgc cttttgaccc 7800
   tcaggatacc caccagtcta gattctacct gtccaagaag aagtgggtca tggtccctat 7860
   gatgtctctg caccacctga ccattcctta cttcagagat gaggaactgt cttgcactgt 7920
   tgtcgagctg aagtacaccg gaaacgcctc tgctctgttc attttgcctg accaggacaa 7980
   gatggaagag gttgaggcta tgttgctgcc tgagactttg aagagatggc gcgactcttt 8040
   ggagttcaga gagatcggag agctgtacct gcctaagttc tctatttccc gcgactacaa 8100
   cctgaacgac attctgctgc agttgggaat tgaggaagca ttcacctcta aggctgacct 8160
   gtctggaatt accggtgcta gaaacctggc tgtctctcag gttgtccaca aggctgttct 8220
   ggacgttttt gaggaaggaa ctgaggcttc tgctgctacc gctgtcaaga ttaccctgct 8280
   gtctgctctg gttgagacta gaaccattgt cagattcaac cgccctttcc tgatgattat 8340
   tgtccctacc gacacccaga acatcttttt catgtccaag gtcaccaacc ctaagcaggc 8400
   tcaccatcat caccaccact aataaggatc cccctcgcgg acttgccaga ttctaaggag 8460
   acgtggaagg acataccgct tttgagaagc gtgtttgaaa atagttcttt ttctggttta 8520
   tatcgtttat gaagtgatga gatgaaaagc tgaaatagcg agtataggaa aatttaatga 8580
   aaattaaatt aaatattttc ttaggctatt agtcaccttc aaaatgccgg ccgcttctaa 8640
   gaacgttgtc atgatcgaca actacgactc gtttacctgg aacctgtacg agtacctgtg 8700
   tcaggaggga gccaatgtcg aggttttcag gaacgatcag atcaccattc cggagattga 8760
   gcaggggtac c 8771
<210> 6
   <211> 8126
   <212> DNA
   <213> künstliche Sequenz
<220>
   <221> MOX-T
   <222> (149)..(7948)
   <223> MOX-Terminator H.polymorpha
<220>
   <221> ampR
   <222> (273)..(1233)
   <223> á-lactamase
<220>
   <221> ori
   <222> (1378)..(2045)
<220>
   <221> ScURA3
   <222> (3130)..(4287)
   <223> URA3 gene from S. cerevisiae
<220>
   <221> HARS1
   <222> (5087)..(5582)
   <223> HARS1 H.polymorpha
<220>
   <221> FMD-P
   <222> (5717)..(6782)
   <223> FMD-Promoter H.polymorpha
<220>
   <221> FTH1
   <222> (6790)..(7337)
   <223> humanes Ferritin (H-Kette)
<220>
   <221> G3
   <222> (7339)..(7347)
   <223> G3-Linker
<220>
   <221> Ek
   <222> (7348)..(7362)
   <223> Enteropeptifase-Spaltstelle
<220>
   <221> mhIL6
   <222> (7363)..(7917)
   <223> matures humanes Interleukin 6
<220>
   <221> H6
   <222> (7918)..(7935)
   <223> 6-Histidin-His-Tag
<400> 6
   agtcaccttc aaaatgccgg ccgcttctaa gaacgttgtc atgatcgaca actacgactc 60
   gtttacctgg aacctgtacg agtacctgtg tcaggaggga gccaatgtcg aggttttcag 120
   gaacgatcag atcaccattc cggagattga gcaggggtac ccaattcttg aagacgaaag 180
   ggcctcgtga tacgcctatt tttataggtt aatgtcatga taataatggt ttcttagacg 240
   tcaggtggca cttttcgggg aaatgtgcgc ggaaccccta tttgtttatt tttctaaata 300
   cattcaaata tgtatccgct catgagacaa taaccctgat aaatgcttca ataatattga 360
   aaaaggaaga gtatgagtat tcaacatttc cgtgtcgccc ttattccctt ttttgcggca 420
   ttttgccttc ctgtttttgc tcacccagaa acgctggtga aagtaaaaga tgctgaagat 480
   cagttgggtg cacgagtggg ttacatcgaa ctggatctca acagcggtaa gatccttgag 540
   agttttcgcc ccgaagaacg ttttccaatg atgagcactt ttaaagttct gctatgtggc 600
   gcggtattat cccgtgttga cgccgggcaa gagcaactcg gtcgccgcat acactattct 660
   cagaatgact tggttgagta ctcaccagtc acagaaaagc atcttacgga tggcatgaca 720
   gtaagagaat tatgcagtgc tgccataacc atgagtgata acactgcggc caacttactt 780
   ctgacaacga tcggaggacc gaaggagcta accgcttttt tgcacaacat gggggatcat 840
   gtaactcgcc ttgatcgttg ggaaccggag ctgaatgaag ccataccaaa cgacgagcgt 900
   gacaccacga tgcctgcagc aatggcaaca acgttgcgca aactattaac tggcgaacta 960
   cttactctag cttcccggca acaattaata gactggatgg aggcggataa agttgcagga 1020
   ccacttctgc gctcggccct tccggctggc tggtttattg ctgataaatc tggagccggt 1080
   gagcgtgggt ctcgcggtat cattgcagca ctggggccag atggtaagcc ctcccgtatc 1140
   gtagttatct acacgacggg gagtcaggca actatggatg aacgaaatag acagatcgct 1200
   gagataggtg cctcactgat taagcattgg taactgtcag accaagttta ctcatatata 1260
   ctttagattg atttaaaact tcatttttaa tttaaaagga tctaggtgaa gatccttttt 1320
   gataatctca tgaccaaaat cccttaacgt gagttttcgt tccactgagc gtcagacccc 1380
   gtagaaaaga tcaaaggatc ttcttgagat cctttttttc tgcgcgtaat ctgctgcttg 1440
   caaacaaaaa aaccaccgct accagcggtg gtttgtttgc cggatcaaga gctaccaact 1500
   ctttttccga aggtaactgg cttcagcaga gcgcagatac caaatactgt ccttctagtg 1560
   tagccgtagt taggccacca cttcaagaac tctgtagcac cgcctacata cctcgctctg 1620
   ctaatcctgt taccagtggc tgctgccagt ggcgataagt cgtgtcttac cgggttggac 1680
   tcaagacgat agttaccgga taaggcgcag cggtcgggct gaacgggggg ttcgtgcaca 1740
   cagcccagct tggagcgaac gacctacacc gaactgagat acctacagcg tgagctatga 1800
   gaaagcgcca cgcttcccga agggagaaag gcggacaggt atccggtaag cggcagggtc 1860
   ggaacaggag agcgcacgag ggagcttcca gggggaaacg cctggtatct ttatagtcct 1920
   gtcgggtttc gccacctctg acttgagcgt cgatttttgt gatgctcgtc aggggggcgg 1980
   agcctatgga aaaacgccag caacgcggcc tttttacggt tcctggcctt ttgctggcct 2040
   tttgctcaca tgttctttcc tgcgttatcc cctgattctg tggataaccg tattaccgcc 2100
   tttgagtgag ctgataccgc tcgccgcagc cgaacgaccg agcgcagcga gtcagtgagc 2160
   gaggaagcgg aagagcgcct gatgcggtat tttctcctta cgcatctgtg cggtatttca 2220
   caccgcatat ggtgcactct cagtacaatc tgctctgatg ccgcatagtt aagccagtat 2280
   acactccgct atcgctacgt gactgggtca tggctgcgcc ccgacacccg ccaacacccg 2340
   ctgacgcgcc ctgacgggct tgtctgctcc cggcatccgc ttacagacaa gctgtgaccg 2400
   tctccgggag ctgcatgtgt cagaggtttt caccgtcatc accgaaacgc gcgaggcagc 2460
   tgcggtaaag ctcatcagcg tggtcgtgaa gcgattcaca gatgtctgcc tgttcatccg 2520
   cgtccagctc gttgagtttc tccagaagcg ttaatgtctg gcttctgata aagcgggcca 2580
   tgttaagggc ggttttttcc tgtttggtca ctgatgcctc cgtgtaaggg ggatttctgt 2640
   tcatgggggt aatgataccg atgaaacgag agaggatgct cacgatacgg gttactgatg 2700
   atgaacatgc ccggttactg gaacgttgtg agggtaaaca actggcggta tggatgcggc 2760
   gggaccagag aaaaatcact cagggtcaat gccagcgctt cgttaataca gatgtaggtg 2820
   ttccacaggg tagccagcag catcctgcga tgcagatccg gaacataatg gtgcagggcg 2880
   ctgacttccg cgtttccaga ctttacgaaa cacggaaacc gaagaccatt catgttgttg 2940
   ctcaggtcgc agacgttttg cagcagcagt cgcttcacgt tcgctcgcgt atcggtgatt 3000
   cattctgcta accagtaagg caaccccgcc agcctagccg ggtcctcaac gacaggagca 3060
   cgatcatgcg cacccgtggc caggacccaa cgctgcgggg gggggggggg gggggggggg 3120
   gggggggggg ttttctttcc aatttttttt tttcgtcatt ataaaaatca ttacgaccga 3180
   gattcccggg taataactga tataattaaa ttgaagctct aatttgtgag tttagtatac 3240
   atgcatttac ttataataca gttttttagt tttgctggcc gcatcttctc aaatatgctt 3300
   cccagcctgc ttttctgtaa cgttcaccct ctaccttagc atcccttccc tttgcaaata 3360
   gtcctcttcc aacaataata atgtcagatc ctgtagagac cacatcatcc acggttctat 3420
   actgttgacc caatgcgtct cccttgtcat ctaaacccac accgggtgtc ataatcaacc 3480
   aatcgtaacc ttcatctctt ccacccatgt ctctttgagc aataaagccg ataacaaaat 3540
   ctttgtcgct cttcgcaatg tcaacagtac ccttagtata ttctccagta gatagggagc 3600
   ccttgcatga caattctgct aacatcaaaa ggcctctagg ttcctttgtt acttcttctg 3660
   ccgcctgctt caaaccgcta acaatacctg ggcccaccac accgtgtgca ttcgtaatgt 3720
   ctgcccattc tgctattctg tatacacccg cagagtactg caatttgact gtattaccaa 3780
   tgtcagcaaa ttttctgtct tcgaagagta aaaaattgta cttggcggat aatgccttta 3840
   gcggcttaac tgtgccctcc atggaaaaat cagtcaagat atccacatgt gtttttagta 3900
   aacaaatttt gggacctaat gcttcaacta actccagtaa ttccttggtg gtacgaacat 3960
   ccaatgaagc acacaagttt gtttgctttt cgtgcatgat attaaatagc ttggcagcaa 4020
   caggactagg atgagtagca gcacgttcct tatatgtagc tttcgacatg atttatcttc 4080
   gtttcctgca ggtttttgtt ctgtgcagtt gggttaagaa tactgggcaa tttcatgttt 4140
   cttcaacact acatatgcgt atatatacca atctaagtct gtgctccttc cttcgttctt 4200
   ccttctgttc ggagattacc gaatcaaaaa aatttcaagg aaaccgaaat caaaaaaaag 4260
   aataaaaaaa aaatgatgaa ttgaaacccc cccccccccc cccccccccc gatgcgccgc 4320
   gtgcggctgc tggagatggc ggacgcgatg gatatgttct gccaagggtt ggtttgcgca 4380
   ttcacagttc tccgcaagaa ttgattggct ccaattcttg gagtggtgaa tccgttagcg 4440
   aggtgccgcc ggcttccatt caggtcgagg tggcccggct ccatgcaccg cgacgcaacg 4500
   cggggaggca gacaaggtat agggcggcgc ctacaatcca tgccaacccg ttccatgtgc 4560
   tcgccgaggc ggcataaatc gccgtgacga tcagcggtcc aatgatcgaa gttaggctgg     4620
   taagagccgc gagcgatcct tgaagctgtc cctgatggtc gtcatctacc tgcctggaca     4680
   gcatggcctg caacgcgggc atcccgatgc cgccggaagc gagaagaatc ataatgggga     4740
   aggccatcca gcctcgcgtc gcgaacgcca gcaagacgta gcccagcgcg tcggccgcca     4800
   tgccggcgat aatggcctgc ttctcgccga aacgtttggt ggcgggacca gtgacgaagg     4860
   cttgagcgag ggcgtgcaag attccgaata ccgcaagcga caggccgatc atcgtcgcgc     4920
   tccagcgaaa gcggtcctcg ccgaaaatga cccagagcgc tgccggcacc tgtcctacga     4980
   gttgcatgat aaagaagaca gtcataagtg cggcgacgat agtcatgccc cgcgcccacc     5040
   ggaaggagct gactgggttg aaggctctca agggcatcgg tcgactcccg cgactcggcg     5100
   ttcactttcg agctattatc aacgccggaa tacgtcagaa acagccgtgc cccagggacc     5160
   agaaagccta ctggtgagta tgttctttcg tgtgattttt ccgaggatga gaacgacgat     5220
   aacgagcaca actcggagtc ggaggacacg cttattgcgt tgaacgcagc caacatcagc     5280
   aggctgtcaa gactgagtat ggccacagag ctggatttct cggcctcata ctcaaagacg     5340
   tttagtaaac tccgtctgcc agaaattgct gacgaggatg tataataata gatgaattac     5400
   gaacaattgt agttcaaaaa aatttagtaa caatattgtc tagatgacag atttgctgaa     5460
   accagtgaac tccaataaac cactcaccgc tacccaagag aaacagatca gagtgctagg     5520
   gcttgtttca gagtactaca acgtttacca gaagcttgag caagttctca aacgcgggtt     5580
   tgtcgacgct ctcccttatg cgactcctgc attaggaagc agcccagtag taggttgagg     5640
   ccgttgagca ccgccgccgc aaggaatggt gtcgacgcgg agaacgatct cctcgacgcg     5700
   gagaacgatc tcctcgagat ccaggaagcg caacgacgcg gggaatttcg taatcttccc     5760
   cgaggaaaac cgcttcccaa agatttcacc ggcaataacg ccttcattga ccgcacagag     5820
   taccacttga acaacattct gaaacggcag gaggcggttc cgccgtggat agagaaacag     5880
   ggcagtgtgg aggctcagat ccgccagttt cggcgccagc tcgactccga ttggacactg     5940
   caggccgtgc acatcgtttg cgagaaacac tcgaagaaaa gcgacacgga caagatccag     6000
   atcatgagcc agtacgccga ggcagagcgc aacaataact cgagttatac gaagctgcgc     6060
   agcagccaat gggaacagaa atacagcaac tatctggagg tcaaattaaa agcgctgaat     6120
   gacagcatcc gaggttataa tctgcaggct cctttggcat cgcagaaact gtatctaaac 6180
   gcaaactccg agctggaaaa atgttaccgg cgatgcgcgg acaatttaga ggcggcgatc 6240
   aagaaacacc tgctgggcga gcagtctgga gcacagtctt cgatgggccc gagatcccac 6300
   cgcgttcctg ggtaccggga cgtgaggcag cgcgacatcc atcaaatata ccaggcgcca 6360
   accgagtctc tcggaaaaca gcttctggat atcttccgct ggcggcgcaa cgacgaataa 6420
   tagtccctgg aggtgacgga atatatatgt gtggagggta aatctgacag ggtgtagcaa 6480
   aggtaatatt ttcctaaaac atgcaatcgg ctgccccgca acgggaaaaa gaatgacttt 6540
   ggcactcttc accagagtgg ggtgtcccgc tcgtgtgtgc aaataggctc ccactggtca 6600
   ccccggattt tgcagaaaaa cagcaagttc cggggtgtct cactggtgtc cgccaataag 6660
   aggagccggc aggcacggag tctacatcaa gctgtctccg atacactcga ctaccatccg 6720
   ggtctctcag agaggggaat ggcactataa ataccgcctc cttgcgctct ctgccttcat 6780
   cgggaattca tgaccaccgc ttctacctct caggtcagac agaactacca ccaggattct 6840
   gaggctgcta ttaaccgcca gatcaacttg gagctgtacg cttcttacgt ctacctgtcc 6900
   atgtcttact acttcgaccg tgacgacgtt gctttgaaga acttcgccaa gtacttcttg 6960
   caccagtctc acgaggaaag agagcacgct gagaagctga tgaagctgca gaatcagaga 7020
   ggtggaagaa tcttcttgca ggacatcaag aagcctgatt gcgacgattg ggagtctgga 7080
   ctgaacgcta tggaatgcgc tttgcacttg gagaagaacg tcaaccagtc tctgttggag 7140
   ttgcacaagc tggctactga caagaacgac cctcacctgt gcgactttat cgagactcac 7200
   tacctgaacg agcaggtcaa ggctattaag gaactgggtg accacgttac caacctgaga 7260
   aagatgggtg ctcctgagtc tggattggct gagtacctgt tcgacaagca cactttggga 7320
   gactctgata acgaatctgg aggtggagac gatgatgaca aggctcctgt tcctcctggt 7380
   gaagattcta aggacgtcgc tgctcctcac agacagcctt tgacctcttc tgagcgcatc 7440
   gataagcaga ttcgctacat tctggacgga atttctgccc tgagaaagga aacctgcaac 7500
   aagtctaaca tgtgcgagtc ctctaaggaa gctctggctg agaacaacct gaacctgcct 7560
   aagatggctg agaaggacgg atgtttccag tctggattca acgaggaaac ctgcctggtc 7620
   aagattatta ccggactgtt ggagttcgag gtctacttgg agtacctgca gaacagattc 7680
   gagtcctctg aagaacaggc tagagctgtc cagatgtcta ccaaggtcct gattcagttc 7740
   ctgcagaaga aggctaagaa cctggacgct attactactc ctgaccctac cactaacgct 7800
   tctctgctga ctaagctgca ggctcagaat cagtggttgc aggacatgac cactcacctg 7860
   atcctgagat ccttcaagga atttctgcag tcctctctga gagctttgag acagatgcac 7920
   catcatcacc accactaata aggatccccc tcgcggactt gccagattct aaggagacgt 7980
   ggaaggacat accgcttttg agaagcgtgt ttgaaaatag ttctttttct ggtttatatc 8040
   gtttatgaag tgatgagatg aaaagctgaa atagcgagta taggaaaatt taatgaaaat 8100
   taaattaaat attttcttag gctatt 8126
<210> 7
   <211> 8052
   <212> DNA
   <213> künstliche Sequenz
<220>
   <221> ampR
   <222> (212)..(1072)
   <223> á-lactamase
<220>
   <221> ori
   <222> (1217)..(1889)
<220>
   <221> ScURA3
   <222> (2969) .. (4126)
   <223> URA3 gene from S. cerevisiae
<220>
   <221> HARS1
   <222> (4926)..(5925)
   <223> HARS1 H. polymorpha
<220>
   <221> FMD-P
   <222> (5560)..(6625)
   <223> FMD-Promotor H. polymorpha
<220>
   <221> FTH1
   <222> (6633)..(7181)
   <223> humanes Ferritin (schwere Kette)
<220>
   <221> G3
   <222> (7182)..(7190)
   <223> G3-Linker
<220>
   <221> Ek
   <222> (7191)..(7205)
   <223> Enteropeptifase-Spaltstelle
<220>
   <221> mhIFNalpha-2a
   <222> (7206) .. (7703)
   <223> matures humanes Interferon alpha 2a
<220>
   <221> MOX-T
   <222> (7713)..(8040)
   <223> MOX-Terminator H. polymorpha
<400> 7
   caattcttga agacgaaagg gcctcgtgat acgcctattt ttataggtta atgtcatgat      60
   aataatggtt tcttagacgt caggtggcac ttttcgggga aatgtgcgcg gaacccctat     120
   ttgtttattt ttctaaatac attcaaatat gtatccgctc atgagacaat aaccctgata     180
   aatgcttcaa taatattgaa aaaggaagag tatgagtatt caacatttcc gtgtcgccct     240
   tattcccttt tttgcggcat tttgccttcc tgtttttgct cacccagaaa cgctggtgaa     300
   agtaaaagat gctgaagatc agttgggtgc acgagtgggt tacatcgaac tggatctcaa     360
   cagcggtaag atccttgaga gttttcgccc cgaagaacgt tttccaatga tgagcacttt     420
   taaagttctg ctatgtggcg cggtattatc ccgtgttgac gccgggcaag agcaactcgg     480
   tcgccgcata cactattctc agaatgactt ggttgagtac tcaccagtca cagaaaagca     540
   tcttacggat ggcatgacag taagagaatt atgcagtgct gccataacca tgagtgataa     600
   cactgcggcc aacttacttc tgacaacgat cggaggaccg aaggagctaa ccgctttttt     660
   gcacaacatg ggggatcatg taactcgcct tgatcgttgg gaaccggagc tgaatgaagc     720
   cataccaaac gacgagcgtg acaccacgat gcctgcagca atggcaacaa cgttgcgcaa     780
   actattaact ggcgaactac ttactctagc ttcccggcaa caattaatag actggatgga     840
   ggcggataaa gttgcaggac cacttctgcg ctcggccctt ccggctggct ggtttattgc     900
   tgataaatct ggagccggtg agcgtgggtc tcgcggtatc attgcagcac tggggccaga 960
   tggtaagccc tcccgtatcg tagttatcta cacgacgggg agtcaggcaa ctatggatga 1020
   acgaaataga cagatcgctg agataggtgc ctcactgatt aagcattggt aactgtcaga 1080
   ccaagtttac tcatatatac tttagattga tttaaaactt catttttaat ttaaaaggat 1140
   ctaggtgaag atcctttttg ataatctcat gaccaaaatc ccttaacgtg agttttcgtt 1200
   ccactgagcg tcagaccccg tagaaaagat caaaggatct tcttgagatc ctttttttct 1260
   gcgcgtaatc tgctgcttgc aaacaaaaaa accaccgcta ccagcggtgg tttgtttgcc 1320
   ggatcaagag ctaccaactc tttttccgaa ggtaactggc ttcagcagag cgcagatacc 1380
   aaatactgtc cttctagtgt agccgtagtt aggccaccac ttcaagaact ctgtagcacc 1440
   gcctacatac ctcgctctgc taatcctgtt accagtggct gctgccagtg gcgataagtc 1500
   gtgtcttacc gggttggact caagacgata gttaccggat aaggcgcagc ggtcgggctg 1560
   aacggggggt tcgtgcacac agcccagctt ggagcgaacg acctacaccg aactgagata 1620
   cctacagcgt gagctatgag aaagcgccac gcttcccgaa gggagaaagg cggacaggta 1680
   tccggtaagc ggcagggtcg gaacaggaga gcgcacgagg gagcttccag ggggaaacgc 1740
   ctggtatctt tatagtcctg tcgggtttcg ccacctctga cttgagcgtc gatttttgtg 1800
   atgctcgtca ggggggcgga gcctatggaa aaacgccagc aacgcggcct ttttacggtt 1860
   cctggccttt tgctggcctt ttgctcacat gttctttcct gcgttatccc ctgattctgt 1920
   ggataaccgt attaccgcct ttgagtgagc tgataccgct cgccgcagcc gaacgaccga 1980
   gcgcagcgag tcagtgagcg aggaagcgga agagcgcctg atgcggtatt ttctccttac 2040
   gcatctgtgc ggtatttcac accgcatatg gtgcactctc agtacaatct gctctgatgc 2100
   cgcatagtta agccagtata cactccgcta tcgctacgtg actgggtcat ggctgcgccc 2160
   cgacacccgc caacacccgc tgacgcgccc tgacgggctt gtctgctccc ggcatccgct 2220
   tacagacaag ctgtgaccgt ctccgggagc tgcatgtgtc agaggttttc accgtcatca 2280
   ccgaaacgcg cgaggcagct gcggtaaagc tcatcagcgt ggtcgtgaag cgattcacag 2340
   atgtctgcct gttcatccgc gtccagctcg ttgagtttct ccagaagcgt taatgtctgg 2400
   cttctgataa agcgggccat gttaagggcg gttttttcct gtttggtcac tgatgcctcc 2460
   gtgtaagggg gatttctgtt catgggggta atgataccga tgaaacgaga gaggatgctc 2520
   acgatacggg ttactgatga tgaacatgcc cggttactgg aacgttgtga gggtaaacaa 2580
   ctggcggtat ggatgcggcg ggaccagaga aaaatcactc agggtcaatg ccagcgcttc 2640
   gttaatacag atgtaggtgt tccacagggt agccagcagc atcctgcgat gcagatccgg 2700
   aacataatgg tgcagggcgc tgacttccgc gtttccagac tttacgaaac acggaaaccg 2760
   aagaccattc atgttgttgc tcaggtcgca gacgttttgc agcagcagtc gcttcacgtt 2820
   cgctcgcgta tcggtgattc attctgctaa ccagtaaggc aaccccgcca gcctagccgg 2880
   gtcctcaacg acaggagcac gatcatgcgc acccgtggcc aggacccaac gctgcggggg 2940
   gggggggggg gggggggggg gggggggggt tttctttcca attttttttt ttcgtcatta 3000
   taaaaatcat tacgaccgag attcccgggt aataactgat ataattaaat tgaagctcta 3060
   atttgtgagt ttagtataca tgcatttact tataatacag ttttttagtt ttgctggccg 3120
   catcttctca aatatgcttc ccagcctgct tttctgtaac gttcaccctc taccttagca 3180
   tcccttccct ttgcaaatag tcctcttcca acaataataa tgtcagatcc tgtagagacc 3240
   acatcatcca cggttctata ctgttgaccc aatgcgtctc ccttgtcatc taaacccaca 3300
   ccgggtgtca taatcaacca atcgtaacct tcatctcttc cacccatgtc tctttgagca 3360
   ataaagccga taacaaaatc tttgtcgctc ttcgcaatgt caacagtacc cttagtatat 3420
   tctccagtag atagggagcc cttgcatgac aattctgcta acatcaaaag gcctctaggt 3480
   tcctttgtta cttcttctgc cgcctgcttc aaaccgctaa caatacctgg gcccaccaca 3540
   ccgtgtgcat tcgtaatgtc tgcccattct gctattctgt atacacccgc agagtactgc 3600
   aatttgactg tattaccaat gtcagcaaat tttctgtctt cgaagagtaa aaaattgtac 3660
   ttggcggata atgcctttag cggcttaact gtgccctcca tggaaaaatc agtcaagata 3720
   tccacatgtg tttttagtaa acaaattttg ggacctaatg cttcaactaa ctccagtaat 3780
   tccttggtgg tacgaacatc caatgaagca cacaagtttg tttgcttttc gtgcatgata 3840
   ttaaatagct tggcagcaac aggactagga tgagtagcag cacgttcctt atatgtagct 3900
   ttcgacatga tttatcttcg tttcctgcag gtttttgttc tgtgcagttg ggttaagaat 3960
   actgggcaat ttcatgtttc ttcaacacta catatgcgta tatataccaa tctaagtctg 4020
   tgctccttcc ttcgttcttc cttctgttcg gagattaccg aatcaaaaaa atttcaagga 4080
   aaccgaaatc aaaaaaaaga ataaaaaaaa aatgatgaat tgaaaccccc cccccccccc 4140
   cccccccccg atgcgccgcg tgcggctgct ggagatggcg gacgcgatgg atatgttctg 4200
   ccaagggttg gtttgcgcat tcacagttct ccgcaagaat tgattggctc caattcttgg 4260
   agtggtgaat ccgttagcga ggtgccgccg gcttccattc aggtcgaggt ggcccggctc 4320
   catgcaccgc gacgcaacgc ggggaggcag acaaggtata gggcggcgcc tacaatccat 4380
   gccaacccgt tccatgtgct cgccgaggcg gcataaatcg ccgtgacgat cagcggtcca 4440
   atgatcgaag ttaggctggt aagagccgcg agcgatcctt gaagctgtcc ctgatggtcg 4500
   tcatctacct gcctggacag catggcctgc aacgcgggca tcccgatgcc gccggaagcg 4560
   agaagaatca taatggggaa ggccatccag cctcgcgtcg cgaacgccag caagacgtag 4620
   cccagcgcgt cggccgccat gccggcgata atggcctgct tctcgccgaa acgtttggtg 4680
   gcgggaccag tgacgaaggc ttgagcgagg gcgtgcaaga ttccgaatac cgcaagcgac 4740
   aggccgatca tcgtcgcgct ccagcgaaag cggtcctcgc cgaaaatgac ccagagcgct 4800
   gccggcacct gtcctacgag ttgcatgata aagaagacag tcataagtgc ggcgacgata 4860
   gtcatgcccc gcgcccaccg gaaggagctg actgggttga aggctctcaa gggcatcggt 4920
   cgactcccgc gactcggcgt tcactttcga gctattatca acgccggaat acgtcagaaa 4980
   cagccgtgcc ccagggacca gaaagcctac tggtgagtat gttctttcgt gtgatttttc 5040
   cgaggatgag aacgacgata acgagcacaa ctcggagtcg gaggacacgc ttattgcgtt 5100
   gaacgcagcc aacatcagca ggctgtcaag actgagtatg gccacagagc tggatttctc 5160
   ggcctcatac tcaaagacgt ttagtaaact ccgtctgcca gaaattgctg acgaggatgt 5220
   ataataatag atgaattacg aacaattgta gttcaaaaaa atttagtaac aatattgtct 5280
   agatgacaga tttgctgaaa ccagtgaact ccaataaacc actcaccgct acccaagaga 5340
   aacagatcag agtgctaggg cttgtttcag agtactacaa cgtttaccag aagctagctt 5400
   gagcaagttc tcaaacgcgg gtttgtcgac gctctccctt atgcgactcc tgcattagga 5460
   agcagcccag tagtaggttg aggccgttga gcaccgccgc cgcaaggaat ggtgtcgacg 5520
   cggagaacga tctcctcgac gcggagaacg atctcctcga gatccaggaa gcgcaacgac 5580
   gcggggaatt tcgtaatctt ccccgaggaa aaccgcttcc caaagatttc accggcaata 5640
   acgccttcat tgaccgcaca gagtaccact tgaacaacat tctgaaacgg caggaggcgg 5700
   ttccgccgtg gatagagaaa cagggcagtg tggaggctca gatccgccag tttcggcgcc 5760
   agctcgactc cgattggaca ctgcaggccg tgcacatcgt ttgcgagaaa cactcgaaga 5820
   aaagcgacac ggacaagatc cagatcatga gccagtacgc cgaggcagag cgcaacaata 5880
   actcgagtta tacgaagctg cgcagcagcc aatgggaaca gaaatacagc aactatctgg 5940
   aggtcaaatt aaaagcgctg aatgacagca tccgaggtta taatctgcag gctcctttgg 6000
   catcgcagaa actgtatcta aacgcaaact ccgagctgga aaaatgttac cggcgatgcg 6060
   cggacaattt agaggcggcg atcaagaaac acctgctggg cgagcagtct ggagcacagt 6120
   cttcgatggg cccgagatcc caccgcgttc ctgggtaccg ggacgtgagg cagcgcgaca 6180
   tccatcaaat ataccaggcg ccaaccgagt ctctcggaaa acagcttctg gatatcttcc 6240
   gctggcggcg caacgacgaa taatagtccc tggaggtgac ggaatatata tgtgtggagg 6300
   gtaaatctga cagggtgtag caaaggtaat attttcctaa aacatgcaat cggctgcccc 6360
   gcaacgggaa aaagaatgac tttggcactc ttcaccagag tggggtgtcc cgctcgtgtg 6420
   tgcaaatagg ctcccactgg tcaccccgga ttttgcagaa aaacagcaag ttccggggtg 6480
   tctcactggt gtccgccaat aagaggagcc ggcaggcacg gagtctacat caagctgtct 6540
   ccgatacact cgactaccat ccgggtctct cagagagggg aatggcacta taaataccgc 6600
   ctccttgcgc tctctgcctt catcgggaat tcatgaccac cgcttctacc tctcaggtca 6660
   gacagaacta ccaccaggat tctgaggctg ctattaaccg ccagatcaac ttggagctgt 6720
   acgcttctta cgtctacctg tccatgtctt actacttcga ccgtgacgac gttgctttga 6780
   agaacttcgc caagtacttc ttgcaccagt ctcacgagga aagagagcac gctgagaagc 6840
   tgatgaagct gcagaatcag agaggtggaa gaatcttctt gcaggacatc aagaagcctg 6900
   attgcgacga ttgggagtct ggactgaacg ctatggaatg cgctttgcac ttggagaaga 6960
   acgtcaacca gtctctgttg gagttgcaca agctggctac tgacaagaac gaccctcacc 7020
   tgtgcgactt tatcgagact cactacctga acgagcaggt caaggctatt aaggaactgg 7080
   gtgaccacgt taccaacctg agaaagatgg gtgctcctga gtctggattg gctgagtacc 7140
   tgttcgacaa gcacactttg ggagactctg ataacgaatc tggaggtgga gacgatgatg      7200
   acaagtgtga tttgccacag acacactccc tgggctcgcg cagaaccctg atgctgttgg      7260
   ctcagatgag aaagatctct ctgttttcgt gtttgaagga cagacacgac tttggcttcc      7320
   cgcaggagga atttggaaac cagtttcaga aagcagaaac aatccctgtg ctgcacgaga      7380
   tgattcagca gatttttaac ctgttctcta cgaaggactc ctcggcagct tgggatgaga      7440
   ctttgctgga taaattttac actgagttgt atcagcaact gaacgacctg gaggcatgcg      7500
   tgatccaagg tgtcggcgtt acagaaaccc ctttgatgaa ggaggattcc atcctggcag      7560
   ttagaaagta cttccagcgc attacattgt acctgaagga gaaaaagtac tcgccatgcg      7620
   cctgggaggt cgtcagagca gagatcatga gatccttttc gctgtccaca aacctgcagg      7680
   agtctttgag atcgaaggag taataaggat ccccctcgcg gacttgccag attctaagga      7740
   gacgtggaag gacataccgc ttttgagaag cgtgtttgaa aatagttctt tttctggttt      7800
   atatcgttta tgaagtgatg agatgaaaag ctgaaatagc gagtatagga aaatttaatg      7860
   aaaattaaat taaatatttt cttaggctat tagtcacctt caaaatgccg gccgcttcta      7920
   agaacgttgt catgatcgac aactacgact cgtttacctg gaacctgtac gagtacctgt      7980
   gtcaggaggg agccaatgtc gaggttttca ggaacgatca gatcaccatt ccggagattg      8040
   agcaggggta cc                                                          8052
<210> 8
   <211> 7655
   <212> DNA
   <213> künstliche Sequenz
<220>
   <221> ampR
   <222> (212)..(1072)
   <223> á-lactamase
<220>
   <221> ori
   <222> (1217)..(1889)
<220>
   <221> ScURA3
   <222> (2969)..(4126)
   <223> URA3-Gen aus S. cerevisiae
<220>
   <221> HARS1
   <222> (4926)..(5921)
   <223> HARS1 H.polymorpha
<220>
   <221> FMD-P
   <222> (5556)..(6621)
   <223> FMD-Promoter H.polymorpha
<220>
   <221> FTH1
   <222> (6629)..(7177)
   <223> FTH1
<220>
   <221> G3
   <222> (7178)..(7186)
   <223> G3 -Spacer
<220>
   <221> Ek
   <222> (7187)..(7201)
   <223> Enteropeptidase-Trennstelle
<220>
   <221> PTH
   <222> (7202)..(7303)
   <223> Parathyroid-Hormon
<220>
   <221> MOX-T
   <222> (7316)..(7693)
   <223> MOX-Terminator H.polymorpha
<400> 8
   caattcttga agacgaaagg gcctcgtgat acgcctattt ttataggtta atgtcatgat 60
   aataatggtt tcttagacgt caggtggcac ttttcgggga aatgtgcgcg gaacccctat 120
   ttgtttattt ttctaaatac attcaaatat gtatccgctc atgagacaat aaccctgata 180
   aatgcttcaa taatattgaa aaaggaagag tatgagtatt caacatttcc gtgtcgccct 240
   tattcccttt tttgcggcat tttgccttcc tgtttttgct cacccagaaa cgctggtgaa 300
   agtaaaagat gctgaagatc agttgggtgc acgagtgggt tacatcgaac tggatctcaa 360
   cagcggtaag atccttgaga gttttcgccc cgaagaacgt tttccaatga tgagcacttt 420
   taaagttctg ctatgtggcg cggtattatc ccgtgttgac gccgggcaag agcaactcgg 480
   tcgccgcata cactattctc agaatgactt ggttgagtac tcaccagtca cagaaaagca 540
   tcttacggat ggcatgacag taagagaatt atgcagtgct gccataacca tgagtgataa 600
   cactgcggcc aacttacttc tgacaacgat cggaggaccg aaggagctaa ccgctttttt 660
   gcacaacatg ggggatcatg taactcgcct tgatcgttgg gaaccggagc tgaatgaagc 720
   cataccaaac gacgagcgtg acaccacgat gcctgcagca atggcaacaa cgttgcgcaa 780
   actattaact ggcgaactac ttactctagc ttcccggcaa caattaatag actggatgga 840
   ggcggataaa gttgcaggac cacttctgcg ctcggccctt ccggctggct ggtttattgc 900
   tgataaatct ggagccggtg agcgtgggtc tcgcggtatc attgcagcac tggggccaga 960
   tggtaagccc tcccgtatcg tagttatcta cacgacgggg agtcaggcaa ctatggatga 1020
   acgaaataga cagatcgctg agataggtgc ctcactgatt aagcattggt aactgtcaga 1080
   ccaagtttac tcatatatac tttagattga tttaaaactt catttttaat ttaaaaggat 1140
   ctaggtgaag atcctttttg ataatctcat gaccaaaatc ccttaacgtg agttttcgtt 1200
   ccactgagcg tcagaccccg tagaaaagat caaaggatct tcttgagatc ctttttttct 1260
   gcgcgtaatc tgctgcttgc aaacaaaaaa accaccgcta ccagcggtgg tttgtttgcc 1320
   ggatcaagag ctaccaactc tttttccgaa ggtaactggc ttcagcagag cgcagatacc 1380
   aaatactgtc cttctagtgt agccgtagtt aggccaccac ttcaagaact ctgtagcacc 1440
   gcctacatac ctcgctctgc taatcctgtt accagtggct gctgccagtg gcgataagtc 1500
   gtgtcttacc gggttggact caagacgata gttaccggat aaggcgcagc ggtcgggctg 1560
   aacggggggt tcgtgcacac agcccagctt ggagcgaacg acctacaccg aactgagata 1620
   cctacagcgt gagctatgag aaagcgccac gcttcccgaa gggagaaagg cggacaggta 1680
   tccggtaagc ggcagggtcg gaacaggaga gcgcacgagg gagcttccag ggggaaacgc 1740
   ctggtatctt tatagtcctg tcgggtttcg ccacctctga cttgagcgtc gatttttgtg 1800
   atgctcgtca ggggggcgga gcctatggaa aaacgccagc aacgcggcct ttttacggtt 1860
   cctggccttt tgctggcctt ttgctcacat gttctttcct gcgttatccc ctgattctgt 1920
   ggataaccgt attaccgcct ttgagtgagc tgataccgct cgccgcagcc gaacgaccga 1980
   gcgcagcgag tcagtgagcg aggaagcgga agagcgcctg atgcggtatt ttctccttac 2040
   gcatctgtgc ggtatttcac accgcatatg gtgcactctc agtacaatct gctctgatgc 2100
   cgcatagtta agccagtata cactccgcta tcgctacgtg actgggtcat ggctgcgccc 2160
   cgacacccgc caacacccgc tgacgcgccc tgacgggctt gtctgctccc ggcatccgct 2220
   tacagacaag ctgtgaccgt ctccgggagc tgcatgtgtc agaggttttc accgtcatca 2280
   ccgaaacgcg cgaggcagct gcggtaaagc tcatcagcgt ggtcgtgaag cgattcacag 2340
   atgtctgcct gttcatccgc gtccagctcg ttgagtttct ccagaagcgt taatgtctgg 2400
   cttctgataa agcgggccat gttaagggcg gttttttcct gtttggtcac tgatgcctcc 2460
   gtgtaagggg gatttctgtt catgggggta atgataccga tgaaacgaga gaggatgctc 2520
   acgatacggg ttactgatga tgaacatgcc cggttactgg aacgttgtga gggtaaacaa 2580
   ctggcggtat ggatgcggcg ggaccagaga aaaatcactc agggtcaatg ccagcgcttc 2640
   gttaatacag atgtaggtgt tccacagggt agccagcagc atcctgcgat gcagatccgg 2700
   aacataatgg tgcagggcgc tgacttccgc gtttccagac tttacgaaac acggaaaccg 2760
   aagaccattc atgttgttgc tcaggtcgca gacgttttgc agcagcagtc gcttcacgtt 2820
   cgctcgcgta tcggtgattc attctgctaa ccagtaaggc aaccccgcca gcctagccgg 2880
   gtcctcaacg acaggagcac gatcatgcgc acccgtggcc aggacccaac gctgcggggg 2940
   gggggggggg gggggggggg gggggggggt tttctttcca attttttttt ttcgtcatta 3000
   taaaaatcat tacgaccgag attcccgggt aataactgat ataattaaat tgaagctcta 3060
   atttgtgagt ttagtataca tgcatttact tataatacag ttttttagtt ttgctggccg 3120
   catcttctca aatatgcttc ccagcctgct tttctgtaac gttcaccctc taccttagca 3180
   tcccttccct ttgcaaatag tcctcttcca acaataataa tgtcagatcc tgtagagacc 3240
   acatcatcca cggttctata ctgttgaccc aatgcgtctc ccttgtcatc taaacccaca 3300
   ccgggtgtca taatcaacca atcgtaacct tcatctcttc cacccatgtc tctttgagca 3360
   ataaagccga taacaaaatc tttgtcgctc ttcgcaatgt caacagtacc cttagtatat 3420
   tctccagtag atagggagcc cttgcatgac aattctgcta acatcaaaag gcctctaggt 3480
   tcctttgtta cttcttctgc cgcctgcttc aaaccgctaa caatacctgg gcccaccaca 3540
   ccgtgtgcat tcgtaatgtc tgcccattct gctattctgt atacacccgc agagtactgc 3600
   aatttgactg tattaccaat gtcagcaaat tttctgtctt cgaagagtaa aaaattgtac 3660
   ttggcggata atgcctttag cggcttaact gtgccctcca tggaaaaatc agtcaagata 3720
   tccacatgtg tttttagtaa acaaattttg ggacctaatg cttcaactaa ctccagtaat 3780
   tccttggtgg tacgaacatc caatgaagca cacaagtttg tttgcttttc gtgcatgata 3840
   ttaaatagct tggcagcaac aggactagga tgagtagcag cacgttcctt atatgtagct 3900
   ttcgacatga tttatcttcg tttcctgcag gtttttgttc tgtgcagttg ggttaagaat 3960
   actgggcaat ttcatgtttc ttcaacacta catatgcgta tatataccaa tctaagtctg 4020
   tgctccttcc ttcgttcttc cttctgttcg gagattaccg aatcaaaaaa atttcaagga 4080
   aaccgaaatc aaaaaaaaga ataaaaaaaa aatgatgaat tgaaaccccc cccccccccc 4140
   cccccccccg atgcgccgcg tgcggctgct ggagatggcg gacgcgatgg atatgttctg 4200
   ccaagggttg gtttgcgcat tcacagttct ccgcaagaat tgattggctc caattcttgg 4260
   agtggtgaat ccgttagcga ggtgccgccg gcttccattc aggtcgaggt ggcccggctc 4320
   catgcaccgc gacgcaacgc ggggaggcag acaaggtata gggcggcgcc tacaatccat 4380
   gccaacccgt tccatgtgct cgccgaggcg gcataaatcg ccgtgacgat cagcggtcca 4440
   atgatcgaag ttaggctggt aagagccgcg agcgatcctt gaagctgtcc ctgatggtcg 4500
   tcatctacct gcctggacag catggcctgc aacgcgggca tcccgatgcc gccggaagcg 4560
   agaagaatca taatggggaa ggccatccag cctcgcgtcg cgaacgccag caagacgtag 4620
   cccagcgcgt cggccgccat gccggcgata atggcctgct tctcgccgaa acgtttggtg 4680
   gcgggaccag tgacgaaggc ttgagcgagg gcgtgcaaga ttccgaatac cgcaagcgac 4740
   aggccgatca tcgtcgcgct ccagcgaaag cggtcctcgc cgaaaatgac ccagagcgct 4800
   gccggcacct gtcctacgag ttgcatgata aagaagacag tcataagtgc ggcgacgata 4860
   gtcatgcccc gcgcccaccg gaaggagctg actgggttga aggctctcaa gggcatcggt 4920
   cgactcccgc gactcggcgt tcactttcga gctattatca acgccggaat acgtcagaaa 4980
   cagccgtgcc ccagggacca gaaagcctac tggtgagtat gttctttcgt gtgatttttc 5040
   cgaggatgag aacgacgata acgagcacaa ctcggagtcg gaggacacgc ttattgcgtt 5100
   gaacgcagcc aacatcagca ggctgtcaag actgagtatg gccacagagc tggatttctc 5160
   ggcctcatac tcaaagacgt ttagtaaact ccgtctgcca gaaattgctg acgaggatgt 5220
   ataataatag atgaattacg aacaattgta gttcaaaaaa atttagtaac aatattgtct 5280
   agatgacaga tttgctgaaa ccagtgaact ccaataaacc actcaccgct acccaagaga 5340
   aacagatcag agtgctaggg cttgtttcag agtactacaa cgtttaccag aagcttgagc 5400
   aagttctcaa acgcgggttt gtcgacgctc tcccttatgc gactcctgca ttaggaagca 5460
   gcccagtagt aggttgaggc cgttgagcac cgccgccgca aggaatggtg tcgacgcgga 5520
   gaacgatctc ctcgacgcgg agaacgatct cctcgagatc caggaagcgc aacgacgcgg 5580
   ggaatttcgt aatcttcccc gaggaaaacc gcttcccaaa gatttcaccg gcaataacgc 5640
   cttcattgac cgcacagagt accacttgaa caacattctg aaacggcagg aggcggttcc 5700
   gccgtggata gagaaacagg gcagtgtgga ggctcagatc cgccagtttc ggcgccagct 5760
   cgactccgat tggacactgc aggccgtgca catcgtttgc gagaaacact cgaagaaaag 5820
   cgacacggac aagatccaga tcatgagcca gtacgccgag gcagagcgca acaataactc 5880
   gagttatacg aagctgcgca gcagccaatg ggaacagaaa tacagcaact atctggaggt 5940
   caaattaaaa gcgctgaatg acagcatccg aggttataat ctgcaggctc ctttggcatc 6000
   gcagaaactg tatctaaacg caaactccga gctggaaaaa tgttaccggc gatgcgcgga 6060
   caatttagag gcggcgatca agaaacacct gctgggcgag cagtctggag cacagtcttc 6120
   gatgggcccg agatcccacc gcgttcctgg gtaccgggac gtgaggcagc gcgacatcca 6180
   tcaaatatac caggcgccaa ccgagtctct cggaaaacag cttctggata tcttccgctg 6240
   gcggcgcaac gacgaataat agtccctgga ggtgacggaa tatatatgtg tggagggtaa 6300
   atctgacagg gtgtagcaaa ggtaatattt tcctaaaaca tgcaatcggc tgccccgcaa 6360
   cgggaaaaag aatgactttg gcactcttca ccagagtggg gtgtcccgct cgtgtgtgca 6420
   aataggctcc cactggtcac cccggatttt gcagaaaaac agcaagttcc ggggtgtctc 6480
   actggtgtcc gccaataaga ggagccggca ggcacggagt ctacatcaag ctgtctccga 6540
   tacactcgac taccatccgg gtctctcaga gaggggaatg gcactataaa taccgcctcc 6600
   ttgcgctctc tgccttcatc gggaattcat gaccaccgct tctacctctc aggtcagaca 6660
   gaactaccac caggattctg aggctgctat taaccgccag atcaacttgg agctgtacgc 6720
   ttcttacgtc tacctgtcca tgtcttacta cttcgaccgt gacgacgttg ctttgaagaa 6780
   cttcgccaag tacttcttgc accagtctca cgaggaaaga gagcacgctg agaagctgat 6840
   gaagctgcag aatcagagag gtggaagaat cttcttgcag gacatcaaga agcctgattg 6900
   cgacgattgg gagtctggac tgaacgctat ggaatgcgct ttgcacttgg agaagaacgt 6960
   caaccagtct ctgttggagt tgcacaagct ggctactgac aagaacgacc ctcacctgtg 7020
   cgactttatc gagactcact acctgaacga gcaggtcaag gctattaagg aactgggtga 7080
   ccacgttacc aacctgagaa agatgggtgc tcctgagtct ggattggctg agtacctgtt 7140
   cgacaagcac actttgggag actctgataa cgaatctgga ggtggagacg atgatgacaa 7200
   gtcggtgtct gagattcagc tgatgcacaa cctgggtaag cacctgaact ctatggagag 7260
   agtggagtgg ctgagaaaga agttgcagga tgtccacaac ttctagtagg gatccccctc 7320
   gcggacttgc cagattctaa ggagacgtgg aaggacatac cgcttttgag aagcgtgttt 7380
   gaaaatagtt ctttttctgg tttatatcgt ttatgaagtg atgagatgaa aagctgaaat 7440
   agcgagtata ggaaaattta atgaaaatta aattaaatat tttcttaggc tattagtcac 7500
   cttcaaaatg ccggccgctt ctaagaacgt tgtcatgatc gacaactacg actcgtttac 7560
   ctggaacctg tacgagtacc tgtgtcagga gggagccaat gtcgaggttt tcaggaacga 7620
   tcagatcacc attccggaga ttgagcaggg gtacc 7655
<210> 9
   <211> 2782
   <212> DNA
   <213> künstliche Sequenz
<400> 9
   ctaaattgta agcgttaata ttttgttaaa attcgcgtta aatttttgtt aaatcagctc 60
   attttttaac caataggccg aaatcggcaa aatcccttat aaatcaaaag aatagaccga 120
   gatagggttg agtggccgct acagggcgct cccattcgcc attcaggctg cgcaactgtt 180
   gggaagggcg tttcggtgcg ggcctcttcg ctattacgcc agctggcgaa agggggatgt 240
   gctgcaaggc gattaagttg ggtaacgcca gggttttccc agtcacgacg ttgtaaaacg 300
   acggccagtg agcgcgacgt aatacgactc actatagggc gaattggcgg aaggccgtca 360
   aggcctaggc gcgccgaatt cggtgaccac gttaccaacc tgagaaagat gggtgctcct 420
   gagtctggat tggctgagta cctgttcgac aagcacactt tgggagactc tgataacgaa 480
   tctggaggtg gagacgatga tgacaagcac cctaactcgc ctttggatga ggaaaacctg 540
   actcaggaaa accaggatag aggaactcac gtagatctgg gattggcttc tgctaacgtg 600
   gacttcgctt tctcgctgta caaagcttgg agaaaagagc tcctacttct tcgtctacca 660
   agaagactca gctgcagttg gagcacttgt tgctagatct aagcttggaa aagagagctc 720
   ctgttcctcc tggtgaagat tctaaggacg tcgctgctcc tcacagacag cctttgacct 780
   cttctgagcg catcgatgga tccttaatta actggcctca tgggccttcc gctcactgcc 840
   cgctttccag tcgggaaacc tgtcgtgcca gctgcattaa catggtcata gctgtttcct 900
   tgcgtattgg gcgctctccg cttcctcgct cactgactcg ctgcgctcgg tcgttcgggt 960
   aaagcctggg gtgcctaatg agcaaaaggc cagcaaaagg ccaggaaccg taaaaaggcc 1020
   gcgttgctgg cgtttttcca taggctccgc ccccctgacg agcatcacaa aaatcgacgc 1080
   tcaagtcaga ggtggcgaaa cccgacagga ctataaagat accaggcgtt tccccctgga 1140
   agctccctcg tgcgctctcc tgttccgacc ctgccgctta ccggatacct gtccgccttt 1200
   ctcccttcgg gaagcgtggc gctttctcat agctcacgct gtaggtatct cagttcggtg 1260
   taggtcgttc gctccaagct gggctgtgtg cacgaacccc ccgttcagcc cgaccgctgc 1320
   gccttatccg gtaactatcg tcttgagtcc aacccggtaa gacacgactt atcgccactg 1380
   gcagcagcca ctggtaacag gattagcaga gcgaggtatg taggcggtgc tacagagttc 1440
   ttgaagtggt ggcctaacta cggctacact agaagaacag tatttggtat ctgcgctctg 1500
   ctgaagccag ttaccttcgg aaaaagagtt ggtagctctt gatccggcaa acaaaccacc 1560
   gctggtagcg gtggtttttt tgtttgcaag cagcagatta cgcgcagaaa aaaaggatct 1620
   caagaagatc ctttgatctt ttctacgggg tctgacgctc agtggaacga aaactcacgt 1680
   taagggattt tggtcatgag attatcaaaa aggatcttca cctagatcct tttaaattaa 1740
   aaatgaagtt ttaaatcaat ctaaagtata tatgagtaaa cttggtctga cagttaccaa 1800
   tgcttaatca gtgaggcacc tatctcagcg atctgtctat ttcgttcatc catagttgcc 1860
   tgactccccg tcgtgtagat aactacgata cgggagggct taccatctgg ccccagtgct 1920
   gcaatgatac cgcgagaacc acgctcaccg gctccagatt tatcagcaat aaaccagcca      1980
   gccggaaggg ccgagcgcag aagtggtcct gcaactttat ccgcctccat ccagtctatt      2040
   aattgttgcc gggaagctag agtaagtagt tcgccagtta atagtttgcg caacgttgtt      2100
   gccattgcta caggcatcgt ggtgtcacgc tcgtcgtttg gtatggcttc attcagctcc      2160
   ggttcccaac gatcaaggcg agttacatga tcccccatgt tgtgcaaaaa agcggttagc      2220
   tccttcggtc ctccgatcgt tgtcagaagt aagttggccg cagtgttatc actcatggtt      2280
   atggcagcac tgcataattc tcttactgtc atgccatccg taagatgctt ttctgtgact      2340
   ggtgagtact caaccaagtc attctgagaa tagtgtatgc ggcgaccgag ttgctcttgc      2400
   ccggcgtcaa tacgggataa taccgcgcca catagcagaa ctttaaaagt gctcatcatt      2460
   ggaaaacgtt cttcggggcg aaaactctca aggatcttac cgctgttgag atccagttcg      2520
   atgtaaccca ctcgtgcacc caactgatct tcagcatctt ttactttcac cagcgtttct      2580
   gggtgagcaa aaacaggaag gcaaaatgcc gcaaaaaagg gaataagggc gacacggaaa      2640
   tgttgaatac tcatactctt cctttttcaa tattattgaa gcatttatca gggttattgt      2700
   ctcatgagcg gatacatatt tgaatgtatt tagaaaaata aacaaatagg ggttccgcgc      2760
   acatttcccc gaaaagtgcc ac                                               2782
<210> 10
   <211> 2906
   <212> DNA
   <213> künstliche Sequenz
<400> 10
   ctaaattgta agcgttaata ttttgttaaa attcgcgtta aatttttgtt aaatcagctc      60
   attttttaac caataggccg aaatcggcaa aatcccttat aaatcaaaag aatagaccga      120
   gatagggttg agtggccgct acagggcgct cccattcgcc attcaggctg cgcaactgtt     180
   gggaagggcg tttcggtgcg ggcctcttcg ctattacgcc agctggcgaa agggggatgt     240
   gctgcaaggc gattaagttg ggtaacgcca gggttttccc agtcacgacg ttgtaaaacg     300
   acggccagtg agcgcgacgt aatacgactc actatagggc gaattggcgg aaggccgtca     360
   aggccgcatg gcgcgccgaa ttcaagcttg gagaaaagac accctaactc gcctttggat     420
   gaggaaaacc tgactcagga aaaccaggat agaggaactc acgtagatct gggattggct     480
   tctgctaacg tggacttcgc tttctcgctg tacaggtgac cacgttacca acctgagaaa      540
   gatgggtgct cctgagtctg gattggctga gtacctgttc gacaagcaca ctttgggaga      600
   ctctgataac gaatctggag gtggagacga tgatgacaag gctcctgttc ctcctggtga      660
   agattctaag gacgtcgctg ctcctcacag acagcctttg acctcttctg agcgcatcga      720
   tggtgaccac gttaccaacc tgagaaagat gggtgctcct gagtctggat tggctgagta      780
   cctgttcgac aagcacactt tgggagactc tgataacgaa tctggaggtg gagacgatga      840
   tgacaagtgt gatttgccac agacacactc cctgggctcg cgcagaaccc tgatgctgtt      900
   ggctcagatg agaaagatct ggatccttaa ttaactgggc ctcatgggcc ttccgctcac      960
   tgcccgcttt ccagtcggga aacctgtcgt gccagctgca ttaacatggt catagctgtt     1020
   tccttgcgta ttgggcgctc tccgcttcct cgctcactga ctcgctgcgc tcggtcgttc     1080
   gggtaaagcc tggggtgcct aatgagcaaa aggccagcaa aaggccagga accgtaaaaa     1140
   ggccgcgttg ctggcgtttt tccataggct ccgcccccct gacgagcatc acaaaaatcg     1200
   acgctcaagt cagaggtggc gaaacccgac aggactataa agataccagg cgtttccccc     1260
   tggaagctcc ctcgtgcgct ctcctgttcc gaccctgccg cttaccggat acctgtccgc     1320
   ctttctccct tcgggaagcg tggcgctttc tcatagctca cgctgtaggt atctcagttc     1380
   ggtgtaggtc gttcgctcca agctgggctg tgtgcacgaa ccccccgttc agcccgaccg     1440
   ctgcgcctta tccggtaact atcgtcttga gtccaacccg gtaagacacg acttatcgcc     1500
   actggcagca gccactggta acaggattag cagagcgagg tatgtaggcg gtgctacaga     1560
   gttcttgaag tggtggccta actacggcta cactagaaga acagtatttg gtatctgcgc     1620
   tctgctgaag ccagttacct tcggaaaaag agttggtagc tcttgatccg gcaaacaaac     1680
   caccgctggt agcggtggtt tttttgtttg caagcagcag attacgcgca gaaaaaaagg     1740
   atctcaagaa gatcctttga tcttttctac ggggtctgac gctcagtgga acgaaaactc     1800
   acgttaaggg attttggtca tgagattatc aaaaaggatc ttcacctaga tccttttaaa     1860
   ttaaaaatga agttttaaat caatctaaag tatatatgag taaacttggt ctgacagtta     1920
   ccaatgctta atcagtgagg cacctatctc agcgatctgt ctatttcgtt catccatagt     1980
   tgcctgactc cccgtcgtgt agataactac gatacgggag ggcttaccat ctggccccag     2040
   tgctgcaatg ataccgcgag aaccacgctc accggctcca gatttatcag caataaacca 2100
   gccagccgga agggccgagc gcagaagtgg tcctgcaact ttatccgcct ccatccagtc 2160
   tattaattgt tgccgggaag ctagagtaag tagttcgcca gttaatagtt tgcgcaacgt 2220
   tgttgccatt gctacaggca tcgtggtgtc acgctcgtcg tttggtatgg cttcattcag 2280
   ctccggttcc caacgatcaa ggcgagttac atgatccccc atgttgtgca aaaaagcggt 2340
   tagctccttc ggtcctccga tcgttgtcag aagtaagttg gccgcagtgt tatcactcat 2400
   ggttatggca gcactgcata attctcttac tgtcatgcca tccgtaagat gcttttctgt 2460
   gactggtgag tactcaacca agtcattctg agaatagtgt atgcggcgac cgagttgctc 2520
   ttgcccggcg tcaatacggg ataataccgc gccacatagc agaactttaa aagtgctcat 2580
   cattggaaaa cgttcttcgg ggcgaaaact ctcaaggatc ttaccgctgt tgagatccag 2640
   ttcgatgtaa cccactcgtg cacccaactg atcttcagca tcttttactt tcaccagcgt 2700
   ttctgggtga gcaaaaacag gaaggcaaaa tgccgcaaaa aagggaataa gggcgacacg 2760
   gaaatgttga atactcatac tcttcctttt tcaatattat tgaagcattt atcagggtta 2820
   ttgtctcatg agcggataca tatttgaatg tatttagaaa aataaacaaa taggggttcc 2880
   gcgcacattt ccccgaaaag tgccac 2906
<210> 11
   <211> 2701
   <212> DNA
   <213> künstliche Sequenz
<400> 11
   ctaaattgta agcgttaata ttttgttaaa attcgcgtta aatttttgtt aaatcagctc 60
   attttttaac caataggccg aaatcggcaa aatcccttat aaatcaaaag aatagaccga 120
   gatagggttg agtggccgct acagggcgct cccattcgcc attcaggctg cgcaactgtt 180
   gggaagggcg tttcggtgcg ggcctcttcg ctattacgcc agctggcgaa agggggatgt 240
   gctgcaaggc gattaagttg ggtaacgcca gggttttccc agtcacgacg ttgtaaaacg 300
   acggccagtg agcgcgacgt aatacgactc actatagggc gaattggcgg aaggccgtca 360
   aggcctaggc gcgccatgag ctcggtgacc acgttaccaa cctgagaaag atgggtgctc 420
   ctgagtctgg attggctgag tacctgttcg acaagcacac tttgggagac tctgataacg      480
   aatctggagg tggagacgat gatgacaagt cggtgtctga gattcagctg atgcacaacc      540
   tgggtaagca cctgaactct atggagagag tggagtggct gagaaagaag ttgcaggatg      600
   tccacaactt ctagtaggga tccaagcttg gaaaagagag ttcctcctgg tgaagattct      660
   aaggacgtcg ctgctcctca cagacagcct ttgacctctt ctgagcgcat cgatggtacc      720
   tcttaattaa ctggcctcat gggccttccg ctcactgccc gctttccagt cgggaaacct      780
   gtcgtgccag ctgcattaac atggtcatag ctgtttcctt gcgtattggg cgctctccgc      840
   ttcctcgctc actgactcgc tgcgctcggt cgttcgggta aagcctgggg tgcctaatga      900
   gcaaaaggcc agcaaaaggc caggaaccgt aaaaaggccg cgttgctggc gtttttccat      960
   aggctccgcc cccctgacga gcatcacaaa aatcgacgct caagtcagag gtggcgaaac     1020
   ccgacaggac tataaagata ccaggcgttt ccccctggaa gctccctcgt gcgctctcct     1080
   gttccgaccc tgccgcttac cggatacctg tccgcctttc tcccttcggg aagcgtggcg     1140
   ctttctcata gctcacgctg taggtatctc agttcggtgt aggtcgttcg ctccaagctg     1200
   ggctgtgtgc acgaaccccc cgttcagccc gaccgctgcg ccttatccgg taactatcgt     1260
   cttgagtcca acccggtaag acacgactta tcgccactgg cagcagccac tggtaacagg     1320
   attagcagag cgaggtatgt aggcggtgct acagagttct tgaagtggtg gcctaactac     1380
   ggctacacta gaagaacagt atttggtatc tgcgctctgc tgaagccagt taccttcgga     1440
   aaaagagttg gtagctcttg atccggcaaa caaaccaccg ctggtagcgg tggttttttt     1500
   gtttgcaagc agcagattac gcgcagaaaa aaaggatctc aagaagatcc tttgatcttt     1560
   tctacggggt ctgacgctca gtggaacgaa aactcacgtt aagggatttt ggtcatgaga     1620
   ttatcaaaaa ggatcttcac ctagatcctt ttaaattaaa aatgaagttt taaatcaatc     1680
   taaagtatat atgagtaaac ttggtctgac agttaccaat gcttaatcag tgaggcacct     1740
   atctcagcga tctgtctatt tcgttcatcc atagttgcct gactccccgt cgtgtagata     1800
   actacgatac gggagggctt accatctggc cccagtgctg caatgatacc gcgagaacca     1860
   cgctcaccgg ctccagattt atcagcaata aaccagccag ccggaagggc cgagcgcaga     1920
   agtggtcctg caactttatc cgcctccatc cagtctatta attgttgccg ggaagctaga     1980
   gtaagtagtt cgccagttaa tagtttgcgc aacgttgttg ccattgctac aggcatcgtg     2040
   gtgtcacgct cgtcgtttgg tatggcttca ttcagctccg gttcccaacg atcaaggcga     2100
   gttacatgat cccccatgtt gtgcaaaaaa gcggttagct ccttcggtcc tccgatcgtt     2160
   gtcagaagta agttggccgc agtgttatca ctcatggtta tggcagcact gcataattct     2220
   cttactgtca tgccatccgt aagatgcttt tctgtgactg gtgagtactc aaccaagtca     2280
   ttctgagaat agtgtatgcg gcgaccgagt tgctcttgcc cggcgtcaat acgggataat     2340
   accgcgccac atagcagaac tttaaaagtg ctcatcattg gaaaacgttc ttcggggcga     2400
   aaactctcaa ggatcttacc gctgttgaga tccagttcga tgtaacccac tcgtgcaccc     2460
   aactgatctt cagcatcttt tactttcacc agcgtttctg ggtgagcaaa aacaggaagg     2520
   caaaatgccg caaaaaaggg aataagggcg acacggaaat gttgaatact catactcttc     2580
   ctttttcaat attattgaag catttatcag ggttattgtc tcatgagcgg atacatattt     2640
   gaatgtattt agaaaaataa acaaataggg gttccgcgca catttccccg aaaagtgcca     2700
   c                                                                     2701
<210> 12
   <211> 189
   <212> PRT
   <213> Künstliche Sequenz
<220>
   <221> FTH1
   <222> (1)..(183)
   <223> humanes Ferritin (schwere Kette)
<220>
   <221> H6
   <222> (184)..(189)
   <223> 6-Histidin-His-Tag
<400> 12
   Met Thr Thr Ala Ser Thr Ser Gln Val Arg Gln Asn Tyr His Gln Asp
   1               5                   10                  15
   Ser Glu Ala Ala Ile Asn Arg Gln Ile Asn Leu Glu Leu Tyr Ala Ser
               20                  25                  30
   Tyr Val Tyr Leu Ser Met Ser Tyr Tyr Phe Asp Arg Asp Asp Val Ala
           35                  40                  45
   Leu Lys Asn Phe Ala Lys Tyr Phe Leu His Gln Ser His Glu Glu Arg
       50                  55                  60
   Glu His Ala Glu Lys Leu Met Lys Leu Gln Asn Gln Arg Gly Gly Arg
   65                  70                  75                  80
   Ile Phe Leu Gln Asp Ile Lys Lys Pro Asp Cys Asp Asp Trp Glu Ser
                   85                  90                  95
   Gly Leu Asn Ala Met Glu Cys Ala Leu His Leu Glu Lys Asn Val Asn
               100                 105                 110
   Gln Ser Leu Leu Glu Leu His Lys Leu Ala Thr Asp Lys Asn Asp Pro
           115                 120                 125
   His Leu Cys Asp Phe Ile Glu Thr His Tyr Leu Asn Glu Gln Val Lys
       130                 135                 140
   Ala Ile Lys Glu Leu Gly Asp His Val Thr Asn Leu Arg Lys Met Gly
   145                 150                 155                 160
   Ala Pro Glu Ser Gly Leu Ala Glu Tyr Leu Phe Asp Lys His Thr Leu
                   165                 170                 175
   Gly Asp Ser Asp Asn Glu Ser His His His His His His
               180                 185
<210> 13
   <211> 597
   <212> PRT
   <213> Künstliche Sequenz
<220>
   <221> FTH1
   <222> (1)..(183)
   <223> humanes Ferritin (schwere Kette)
<220>
   <221> G3
   <222> (184)..(186)
   <223> G3-Linker
<220>
   <221> Ek
   <222> (187)..(191)
   <223> Enteropeptidase-Spaltstelle
<220>
   <221> mhACT
   <222> (192)..(591)
   <223> maturer humaner alphal-Antichymotrypsin-Precursor
<220>
   <221> H6
   <222> (592)..(597)
   <223> 6Histidin-His-Tag
<400> 13
   Met Thr Thr Ala Ser Thr Ser Gln Val Arg Gln Asn Tyr His Gln Asp
   1               5                   10                  15
   Ser Glu Ala Ala Ile Asn Arg Gln Ile Asn Leu Glu Leu Tyr Ala Ser
               20                  25                  30
   Tyr Val Tyr Leu Ser Met Ser Tyr Tyr Phe Asp Arg Asp Asp Val Ala
           35                  40                  45
   Leu Lys Asn Phe Ala Lys Tyr Phe Leu His Gln Ser His Glu Glu Arg
       50                  55                  60
   Glu His Ala Glu Lys Leu Met Lys Leu Gln Asn Gln Arg Gly Gly Arg
   65                  70                  75                  80
   Ile Phe Leu Gln Asp Ile Lys Lys Pro Asp Cys Asp Asp Trp Glu Ser
                   85                  90                  95
   Gly Leu Asn Ala Met Glu Cys Ala Leu His Leu Glu Lys Asn Val Asn
               100                 105                 110
   Gln Ser Leu Leu Glu Leu His Lys Leu Ala Thr Asp Lys Asn Asp Pro
           115                 120                 125
   His Leu Cys Asp Phe Ile Glu Thr His Tyr Leu Asn Glu Gln Val Lys
   130 135 140
   Ala Ile Lys Glu Leu Gly Asp His Val Thr Asn Leu Arg Lys Met Gly
   145 150 155 160
   Ala Pro Glu Ser Gly Leu Ala Glu Tyr Leu Phe Asp Lys His Thr Leu
   165 170 175
   Gly Asp Ser Asp Asn Glu Ser Gly Gly Gly Asp Asp Asp Asp Lys His
   180 185 190
   Pro Asn Ser Pro Leu Asp Glu Glu Asn Leu Thr Gln Glu Asn Gln Asp
   195 200 205
   Arg Gly Thr His Val Asp Leu Gly Leu Ala Ser Ala Asn Val Asp Phe
   210 215 220
   Ala Phe Ser Leu Tyr Lys Gln Leu Val Leu Lys Ala Pro Asp Lys Asn
   225 230 235 240
   Val Ile Phe Ser Pro Leu Ser Ile Ser Thr Ala Leu Ala Phe Leu Ser
   245 250 255
   Leu Gly Ala His Asn Thr Thr Leu Thr Glu Ile Leu Lys Gly Leu Lys
   260 265 270
   Phe Asn Leu Thr Glu Thr Ser Glu Ala Glu Ile His Gln Ser Phe Gln
   275 280 285
   His Leu Leu Arg Thr Leu Asn Gln Ser Ser Asp Glu Leu Gln Leu Ser
   290 295 300
   Met Gly Asn Ala Met Phe Val Lys Glu Gln Leu Ser Leu Leu Asp Arg
   305 310 315 320
   Phe Thr Glu Asp Ala Lys Arg Leu Tyr Gly Ser Glu Ala Phe Ala Thr
   325 330 335
   Asp Phe Gln Asp Ser Ala Ala Ala Lys Lys Leu Ile Asn Asp Tyr Val
   340 345 350
   Lys Asn Gly Thr Arg Gly Lys Ile Thr Asp Leu Ile Lys Asp Leu Asp
   355 360 365
   Ser Gln Thr Met Met Val Leu Val Asn Tyr Ile Phe Phe Lys Ala Lys
   370 375 380
   Trp Glu Met Pro Phe Asp Pro Gln Asp Thr His Gln Ser Arg Phe Tyr
   385 390 395 400
   Leu Ser Lys Lys Lys Trp Val Met Val Pro Met Met Ser Leu His His
   405 410 415
   Leu Thr Ile Pro Tyr Phe Arg Asp Glu Glu Leu Ser Cys Thr Val Val
   420 425 430
   Glu Leu Lys Tyr Thr Gly Asn Ala Ser Ala Leu Phe Ile Leu Pro Asp
   435 440 445
   Gln Asp Lys Met Glu Glu Val Glu Ala Met Leu Leu Pro Glu Thr Leu
   450 455 460
   Lys Arg Trp Arg Asp Ser Leu Glu Phe Arg Glu Ile Gly Glu Leu Tyr
   465 470 475 480
   Leu Pro Lys Phe Ser Ile Ser Arg Asp Tyr Asn Leu Asn Asp Ile Leu
   485 490 495
   Leu Gln Leu Gly Ile Glu Glu Ala Phe Thr Ser Lys Ala Asp Leu Ser
   500 505 510
   Gly Ile Thr Gly Ala Arg Asn Leu Ala Val Ser Gln Val Val His Lys
   515 520 525
   Ala Val Leu Asp Val Phe Glu Glu Gly Thr Glu Ala Ser Ala Ala Thr
   530 535 540
   Ala Val Lys Ile Thr Leu Leu Ser Ala Leu Val Glu Thr Arg Thr Ile
   545                 550                 555                 560
   Val Arg Phe Asn Arg Pro Phe Leu Met Ile Ile Val Pro Thr Asp Thr
                   565                 570                 575
   Gln Asn Ile Phe Phe Met Ser Lys Val Thr Asn Pro Lys Gln Ala His
               580                 585                 590
   His His His His His
           595
<210> 14
   <211> 382
   <212> PRT
   <213> Künstliche Sequenz
<220>
   <221> FTH1
   <222> (1)..(183)
   <223> humanes Ferritin (schwere Kette)
<220>
   <221> G3
   <222> (184)..(186)
   <223> G3-Linker
<220>
   <221> Ek
   <222> (187)..(191)
   <223> Enteropeptidase-Spaltstelle
<220>
   <221> mhIL6
   <222> (192)..(376)
   <223> matures humanes Interleukin 6
<220>
   <221> H6
   <222> (377)..(382)
   <223> 6-Histidin-His-Tag
<400> 14
   Met Thr Thr Ala Ser Thr Ser Gln Val Arg Gln Asn Tyr His Gln Asp
   1               5                   10                  15
   Ser Glu Ala Ala Ile Asn Arg Gln Ile Asn Leu Glu Leu Tyr Ala Ser
               20                  25                  30
   Tyr Val Tyr Leu Ser Met Ser Tyr Tyr Phe Asp Arg Asp Asp Val Ala
           35                  40                  45
   Leu Lys Asn Phe Ala Lys Tyr Phe Leu His Gln Ser His Glu Glu Arg
       50                  55                  60
   Glu His Ala Glu Lys Leu Met Lys Leu Gln Asn Gln Arg Gly Gly Arg
   65                  70                  75                  80
   Ile Phe Leu Gln Asp Ile Lys Lys Pro Asp Cys Asp Asp Trp Glu Ser
                   85                  90                  95
   Gly Leu Asn Ala Met Glu Cys Ala Leu His Leu Glu Lys Asn Val Asn
               100                 105                 110
   Gln Ser Leu Leu Glu Leu His Lys Leu Ala Thr Asp Lys Asn Asp Pro
           115                 120                 125
   His Leu Cys Asp Phe Ile Glu Thr His Tyr Leu Asn Glu Gln Val Lys
       130                 135                 140
   Ala Ile Lys Glu Leu Gly Asp His Val Thr Asn Leu Arg Lys Met Gly
   145                 150                 155                 160
   Ala Pro Glu Ser Gly Leu Ala Glu Tyr Leu Phe Asp Lys His Thr Leu
                   165                 170                 175
   Gly Asp Ser Asp Asn Glu Ser Gly Gly Gly Asp Asp Asp Asp Lys Ala
               180                 185                 190
   Pro Val Pro Pro Gly Glu Asp Ser Lys Asp Val Ala Ala Pro His Arg
           195                 200                 205
   Gln Pro Leu Thr Ser Ser Glu Arg Ile Asp Lys Gln Ile Arg Tyr Ile
       210                 215                 220
   Leu Asp Gly Ile Ser Ala Leu Arg Lys Glu Thr Cys Asn Lys Ser Asn
   225                 230                 235                 240
   Met Cys Glu Ser Ser Lys Glu Ala Leu Ala Glu Asn Asn Leu Asn Leu
                   245                 250                 255
   Pro Lys Met Ala Glu Lys Asp Gly Cys Phe Gln Ser Gly Phe Asn Glu
               260                 265                 270
   Glu Thr Cys Leu Val Lys Ile Ile Thr Gly Leu Leu Glu Phe Glu Val
           275                 280                 285
   Tyr Leu Glu Tyr Leu Gln Asn Arg Phe Glu Ser Ser Glu Glu Gln Ala
       290                 295                 300
   Arg Ala Val Gln Met Ser Thr Lys Val Leu Ile Gln Phe Leu Gln Lys
   305                 310                 315                 320
   Lys Ala Lys Asn Leu Asp Ala Ile Thr Thr Pro Asp Pro Thr Thr Asn
                   325                 330                 335
   Ala Ser Leu Leu Thr Lys Leu Gln Ala Gln Asn Gln Trp Leu Gln Asp
               340                 345                 350
   Met Thr Thr His Leu Ile Leu Arg Ser Phe Lys Glu Phe Leu Gln Ser
           355                 360                 365
   Ser Leu Arg Ala Leu Arg Gln Met His His His His His His
       370                 375                 380
<210> 15
   <211> 356
   <212> PRT
   <213> Künstliche Sequenz
<220>
   <221> FTH1
   <222> (1)..(183)
   <223> humanes Ferritin (schwere Kette)
<220>
   <221> G3
   <222> (184)..(186)
   <223> G3-Linker
<220>
   <221> Ek
   <222> (187)..(191)
   <223> Enteropeptidase-Spaltstelle
<220>
   <221> mhIFNalpha-2a
   <222> (192)..(356)
   <223> matures humanes Interferon alpha 2a
<400> 15
   Met Thr Thr Ala Ser Thr Ser Gln Val Arg Gln Asn Tyr His Gln Asp
   1               5                   10                  15
   Ser Glu Ala Ala Ile Asn Arg Gln Ile Asn Leu Glu Leu Tyr Ala Ser
               20                  25                  30
   Tyr Val Tyr Leu Ser Met Ser Tyr Tyr Phe Asp Arg Asp Asp Val Ala
           35                  40                  45
   Leu Lys Asn Phe Ala Lys Tyr Phe Leu His Gln Ser His Glu Glu Arg
       50                  55                  60
   Glu His Ala Glu Lys Leu Met Lys Leu Gln Asn Gln Arg Gly Gly Arg
   65                  70                  75                  80
   Ile Phe Leu Gln Asp Ile Lys Lys Pro Asp Cys Asp Asp Trp Glu Ser
                   85                  90                  95
   Gly Leu Asn Ala Met Glu Cys Ala Leu His Leu Glu Lys Asn Val Asn
               100                 105                 110
   Gln Ser Leu Leu Glu Leu His Lys Leu Ala Thr Asp Lys Asn Asp Pro
           115                 120                 125
   His Leu Cys Asp Phe Ile Glu Thr His Tyr Leu Asn Glu Gln Val Lys
   130 135 140
   Ala Ile Lys Glu Leu Gly Asp His Val Thr Asn Leu Arg Lys Met Gly
   145 150 155 160
   Ala Pro Glu Ser Gly Leu Ala Glu Tyr Leu Phe Asp Lys His Thr Leu
   165 170 175
   Gly Asp Ser Asp Asn Glu Ser Gly Gly Gly Asp Asp Asp Asp Lys Cys
   180 185 190
   Asp Leu Pro Gln Thr His Ser Leu Gly Ser Arg Arg Thr Leu Met Leu
   195 200 205
   Leu Ala Gln Met Arg Lys Ile Ser Leu Phe Ser Cys Leu Lys Asp Arg
   210 215 220
   His Asp Phe Gly Phe Pro Gln Glu Glu Phe Gly Asn Gln Phe Gln Lys
   225 230 235 240
   Ala Glu Thr Ile Pro Val Leu His Glu Met Ile Gln Gln Ile Phe Asn
   245 250 255
   Leu Phe Ser Thr Lys Asp Ser Ser Ala Ala Trp Asp Glu Thr Leu Leu
   260 265 270
   Asp Lys Phe Tyr Thr Glu Leu Tyr Gln Gln Leu Asn Asp Leu Glu Ala
   275 280 285
   Cys Val Ile Gln Gly Val Gly Val Thr Glu Thr Pro Leu Met Lys Glu
   290 295 300
   Asp Ser Ile Leu Ala Val Arg Lys Tyr Phe Gln Arg Ile Thr Leu Tyr
   305 310 315 320
   Leu Lys Glu Lys Lys Tyr Ser Pro Cys Ala Trp Glu Val Val Arg Ala
   325 330 335
   Glu Ile Met Arg Ser Phe Ser Leu Ser Thr Asn Leu Gln Glu Ser Leu
   340 345 350
   Arg Ser Lys Glu
   355
<210> 16
   <211> 225
   <212> PRT
   <213> Künstliche Sequenz
<220>
   <221> FTH1
   <222> (1)..(183)
   <223> humanes Ferritin (schwere Kette)
<220>
   <221> G3
   <222> (189)..(186)
   <223> G3-Linker
<220>
   <221> Ek
   <222> (187)..(191)
   <223> Enteropeptidase-Spaltstelle
<220>
   <221> PTH
   <222> (192)..(225)
   <223> Teil des humanen Parathyroid-Hormons
<400> 16
   Met Thr Thr Ala Ser Thr Ser Gln Val Arg Gln Asn Tyr His Gln Asp
   1 5 10 15
   Ser Glu Ala Ala Ile Asn Arg Gln Ile Asn Leu Glu Leu Tyr Ala Ser
   20 25 30
   Tyr Val Tyr Leu Ser Met Ser Tyr Tyr Phe Asp Arg Asp Asp Val Ala
   35 40 45
   Leu Lys Asn Phe Ala Lys Tyr Phe Leu His Gln Ser His Glu Glu Arg
   50 55 60
   Glu His Ala Glu Lys Leu Met Lys Leu Gln Asn Gln Arg Gly Gly Arg
   65                  70                  75                  80
   Ile Phe Leu Gln Asp Ile Lys Lys Pro Asp Cys Asp Asp Trp Glu Ser
                   85                  90                  95
   Gly Leu Asn Ala Met Glu Cys Ala Leu His Leu Glu Lys Asn Val Asn
               100                 105                 110
   Gln Ser Leu Leu Glu Leu His Lys Leu Ala Thr Asp Lys Asn Asp Pro
           115                 120                 125
   His Leu Cys Asp Phe Ile Glu Thr His Tyr Leu Asn Glu Gln Val Lys
       130                 135                 140
   Ala Ile Lys Glu Leu Gly Asp His Val Thr Asn Leu Arg Lys Met Gly
   145                 150                 155                 160
   Ala Pro Glu Ser Gly Leu Ala Glu Tyr Leu Phe Asp Lys His Thr Leu
                   165                 170                 175
   Gly Asp Ser Asp Asn Glu Ser Gly Gly Gly Asp Asp Asp Asp Lys Ser
               180                 185                 190
   Val Ser Glu Ile Gln Leu Met His Asn Leu Gly Lys His Leu Asn Ser
           195                 200                 205
   Met Glu Arg Val Glu Trp Leu Arg Lys Lys Leu Gln Asp Val His Asn
       210                 215                 220
   Phe
   225
<210> 17
   <211> 6980
   <212> DNA
   <213> künstliche Sequenz
<220>
   <221> MOX-T
   <222> (13)..(340)
   <223> MOX terminator H. polymorpha
<220>
   <221> FMD-P
   <222> (360)..(1425)
   <223> FMD promoter H. polymorpha
<220>
   <221> HARS1
   <222> (1560)..(2055)
   <223> HARS1 H. polymorpha
<220>
   <221> ScURA3
   <222> (2855)..(4012)
   <223> URA3 gene from S. cerevisiae
<220>
   <221> ori
   <222> (5097)..(5764)
   <223> ori pBR322
<220>
   <221> ampR
   <222> (5909)..(6769)
<220>
   <221> ampR
   <222> (5909)..(6769)
   <223> á-lactamase
<400> 17
   ggtacccctg ctcaatctcc ggaatggtga tctgatcgtt cctgaaaacc tcgacattgg      60
   ctccctcctg acacaggtac tcgtacaggt tccaggtaaa cgagtcgtag ttgtcgatca     120
   tgacaacgtt cttagaagcg gccggcattt tgaaggtgac taatagccta agaaaatatt     180
   taatttaatt ttcattaaat tttcctatac tcgctatttc agcttttcat ctcatcactt     240
   cataaacgat ataaaccaga aaaagaacta ttttcaaaca cgcttctcaa aagcggtatg     300
   tccttccacg tctccttaga atctggcaag tccgcgaggg ggatccagat ctgaattccc     360
   gatgaaggca gagagcgcaa ggaggcggta tttatagtgc cattcccctc tctgagagac     420
   ccggatggta gtcgagtgta tcggagacag cttgatgtag actccgtgcc tgccggctcc     480
   tcttattggc ggacaccagt gagacacccc ggaacttgct gtttttctgc aaaatccggg     540
   gtgaccagtg ggagcctatt tgcacacacg agcgggacac cccactctgg tgaagagtgc      600
   caaagtcatt ctttttcccg ttgcggggca gccgattgca tgttttagga aaatattacc      660
   tttgctacac cctgtcagat ttaccctcca cacatatata ttccgtcacc tccagggact      720
   attattcgtc gttgcgccgc cagcggaaga tatccagaag ctgttttccg agagactcgg      780
   ttggcgcctg gtatatttga tggatgtcgc gctgcctcac gtcccggtac ccaggaacgc      840
   ggtgggatct cgggcccatc gaagactgtg ctccagactg ctcgcccagc aggtgtttct      900
   tgatcgccgc ctctaaattg tccgcgcatc gccggtaaca tttttccagc tcggagtttg      960
   cgtttagata cagtttctgc gatgccaaag gagcctgcag attataacct cggatgctgt     1020
   cattcagcgc ttttaatttg acctccagat agttgctgta tttctgttcc cattggctgc     1080
   tgcgcagctt cgtataactc gagttattgt tgcgctctgc ctcggcgtac tggctcatga     1140
   tctggatctt gtccgtgtcg cttttcttcg agtgtttctc gcaaacgatg tgcacggcct     1200
   gcagtgtcca atcggagtcg agctggcgcc gaaactggcg gatctgagcc tccacactgc     1260
   cctgtttctc tatccacggc ggaaccgcct cctgccgttt cagaatgttg ttcaagtggt     1320
   actctgtgcg gtcaatgaag gcgttattgc cggtgaaatc tttgggaagc ggttttcctc     1380
   ggggaagatt acgaaattcc ccgcgtcgtt gcgcttcctg gatctcgagg agatcgttct     1440
   ccgcgtcgag gagatcgttc tccgcgtcga caccattcct tgcggcggcg gtgctcaacg     1500
   gcctcaacct actactgggc tgcttcctaa tgcaggagtc gcataaggga gagcgtcgac     1560
   aaacccgcgt ttgagaactt gctcaagctt ctggtaaacg ttgtagtact ctgaaacaag     1620
   ccctagcact ctgatctgtt tctcttgggt agcggtgagt ggtttattgg agttcactgg     1680
   tttcagcaaa tctgtcatct agacaatatt gttactaaat ttttttgaac tacaattgtt      1740
   cgtaattcat ctattattat acatcctcgt cagcaatttc tggcagacgg agtttactaa     1800
   acgtctttga gtatgaggcc gagaaatcca gctctgtggc catactcagt cttgacagcc     1860
   tgctgatgtt ggctgcgttc aacgcaataa gcgtgtcctc cgactccgag ttgtgctcgt     1920
   tatcgtcgtt ctcatcctcg gaaaaatcac acgaaagaac atactcacca gtaggctttc     1980
   tggtccctgg ggcacggctg tttctgacgt attccggcgt tgataatagc tcgaaagtga     2040
   acgccgagtc gcgggagtcg accgatgccc ttgagagcct tcaacccagt cagctccttc     2100
   cggtgggcgc ggggcatgac tatcgtcgcc gcacttatga ctgtcttctt tatcatgcaa     2160
   ctcgtaggac aggtgccggc agcgctctgg gtcattttcg gcgaggaccg ctttcgctgg 2220
   agcgcgacga tgatcggcct gtcgcttgcg gtattcggaa tcttgcacgc cctcgctcaa 2280
   gccttcgtca ctggtcccgc caccaaacgt ttcggcgaga agcaggccat tatcgccggc 2340
   atggcggccg acgcgctggg ctacgtcttg ctggcgttcg cgacgcgagg ctggatggcc 2400
   ttccccatta tgattcttct cgcttccggc ggcatcggga tgcccgcgtt gcaggccatg 2460
   ctgtccaggc aggtagatga cgaccatcag ggacagcttc aaggatcgct cgcggctctt 2520
   accagcctaa cttcgatcat tggaccgctg atcgtcacgg cgatttatgc cgcctcggcg 2580
   agcacatgga acgggttggc atggattgta ggcgccgccc tataccttgt ctgcctcccc 2640
   gcgttgcgtc gcggtgcatg gagccgggcc acctcgacct gaatggaagc cggcggcacc 2700
   tcgctaacgg attcaccact ccaagaattg gagccaatca attcttgcgg agaactgtga 2760
   atgcgcaaac caacccttgg cagaacatat ccatcgcgtc cgccatctcc agcagccgca 2820
   cgcggcgcat cggggggggg gggggggggg gggggtttca attcatcatt ttttttttat 2880
   tctttttttt gatttcggtt tccttgaaat ttttttgatt cggtaatctc cgaacagaag 2940
   gaagaacgaa ggaaggagca cagacttaga ttggtatata tacgcatatg tagtgttgaa 3000
   gaaacatgaa attgcccagt attcttaacc caactgcaca gaacaaaaac ctgcaggaaa 3060
   cgaagataaa tcatgtcgaa agctacatat aaggaacgtg ctgctactca tcctagtcct 3120
   gttgctgcca agctatttaa tatcatgcac gaaaagcaaa caaacttgtg tgcttcattg 3180
   gatgttcgta ccaccaagga attactggag ttagttgaag cattaggtcc caaaatttgt 3240
   ttactaaaaa cacatgtgga tatcttgact gatttttcca tggagggcac agttaagccg 3300
   ctaaaggcat tatccgccaa gtacaatttt ttactcttcg aagacagaaa atttgctgac 3360
   attggtaata cagtcaaatt gcagtactct gcgggtgtat acagaatagc agaatgggca 3420
   gacattacga atgcacacgg tgtggtgggc ccaggtattg ttagcggttt gaagcaggcg 3480
   gcagaagaag taacaaagga acctagaggc cttttgatgt tagcagaatt gtcatgcaag 3540
   ggctccctat ctactggaga atatactaag ggtactgttg acattgcgaa gagcgacaaa 3600
   gattttgtta tcggctttat tgctcaaaga gacatgggtg gaagagatga aggttacgat 3660
   tggttgatta tgacacccgg tgtgggttta gatgacaagg gagacgcatt gggtcaacag 3720
   tatagaaccg tggatgatgt ggtctctaca ggatctgaca ttattattgt tggaagagga 3780
   ctatttgcaa agggaaggga tgctaaggta gagggtgaac gttacagaaa agcaggctgg 3840
   gaagcatatt tgagaagatg cggccagcaa aactaaaaaa ctgtattata agtaaatgca 3900
   tgtatactaa actcacaaat tagagcttca atttaattat atcagttatt acccgggaat 3960
   ctcggtcgta atgattttta taatgacgaa aaaaaaaaat tggaaagaaa accccccccc 4020
   cccccccccc cccccccccc cccccgcagc gttgggtcct ggccacgggt gcgcatgatc 4080
   gtgctcctgt cgttgaggac ccggctaggc tggcggggtt gccttactgg ttagcagaat 4140
   gaatcaccga tacgcgagcg aacgtgaagc gactgctgct gcaaaacgtc tgcgacctga 4200
   gcaacaacat gaatggtctt cggtttccgt gtttcgtaaa gtctggaaac gcggaagtca 4260
   gcgccctgca ccattatgtt ccggatctgc atcgcaggat gctgctggct accctgtgga 4320
   acacctacat ctgtattaac gaagcgctgg cattgaccct gagtgatttt tctctggtcc 4380
   cgccgcatcc ataccgccag ttgtttaccc tcacaacgtt ccagtaaccg ggcatgttca 4440
   tcatcagtaa cccgtatcgt gagcatcctc tctcgtttca tcggtatcat tacccccatg 4500
   aacagaaatc ccccttacac ggaggcatca gtgaccaaac aggaaaaaac cgcccttaac 4560
   atggcccgct ttatcagaag ccagacatta acgcttctgg agaaactcaa cgagctggac 4620
   gcggatgaac aggcagacat ctgtgaatcg cttcacgacc acgctgatga gctttaccgc 4680
   agctgcctcg cgcgtttcgg tgatgacggt gaaaacctct gacacatgca gctcccggag 4740
   acggtcacag cttgtctgta agcggatgcc gggagcagac aagcccgtca gggcgcgtca 4800
   gcgggtgttg gcgggtgtcg gggcgcagcc atgacccagt cacgtagcga tagcggagtg 4860
   tatactggct taactatgcg gcatcagagc agattgtact gagagtgcac catatgcggt 4920
   gtgaaatacc gcacagatgc gtaaggagaa aataccgcat caggcgctct tccgcttcct 4980
   cgctcactga ctcgctgcgc tcggtcgttc ggctgcggcg agcggtatca gctcactcaa 5040
   aggcggtaat acggttatcc acagaatcag gggataacgc aggaaagaac atgtgagcaa 5100
   aaggccagca aaaggccagg aaccgtaaaa aggccgcgtt gctggcgttt ttccataggc 5160
   tccgcccccc tgacgagcat cacaaaaatc gacgctcaag tcagaggtgg cgaaacccga 5220
   caggactata aagataccag gcgtttcccc ctggaagctc cctcgtgcgc tctcctgttc 5280
   cgaccctgcc gcttaccgga tacctgtccg cctttctccc ttcgggaagc gtggcgcttt 5340
   ctcatagctc acgctgtagg tatctcagtt cggtgtaggt cgttcgctcc aagctgggct 5400
   gtgtgcacga accccccgtt cagcccgacc gctgcgcctt atccggtaac tatcgtcttg 5460
   agtccaaccc ggtaagacac gacttatcgc cactggcagc agccactggt aacaggatta 5520
   gcagagcgag gtatgtaggc ggtgctacag agttcttgaa gtggtggcct aactacggct 5580
   acactagaag gacagtattt ggtatctgcg ctctgctgaa gccagttacc ttcggaaaaa 5640
   gagttggtag ctcttgatcc ggcaaacaaa ccaccgctgg tagcggtggt ttttttgttt 5700
   gcaagcagca gattacgcgc agaaaaaaag gatctcaaga agatcctttg atcttttcta 5760
   cggggtctga cgctcagtgg aacgaaaact cacgttaagg gattttggtc atgagattat 5820
   caaaaaggat cttcacctag atccttttaa attaaaaatg aagttttaaa tcaatctaaa 5880
   gtatatatga gtaaacttgg tctgacagtt accaatgctt aatcagtgag gcacctatct 5940
   cagcgatctg tctatttcgt tcatccatag ttgcctgact ccccgtcgtg tagataacta 6000
   cgatacggga gggcttacca tctggcccca gtgctgcaat gataccgcga gacccacgct 6060
   caccggctcc agatttatca gcaataaacc agccagccgg aagggccgag cgcagaagtg 6120
   gtcctgcaac tttatccgcc tccatccagt ctattaattg ttgccgggaa gctagagtaa 6180
   gtagttcgcc agttaatagt ttgcgcaacg ttgttgccat tgctgcaggc atcgtggtgt 6240
   cacgctcgtc gtttggtatg gcttcattca gctccggttc ccaacgatca aggcgagtta 6300
   catgatcccc catgttgtgc aaaaaagcgg ttagctcctt cggtcctccg atcgttgtca 6360
   gaagtaagtt ggccgcagtg ttatcactca tggttatggc agcactgcat aattctctta 6420
   ctgtcatgcc atccgtaaga tgcttttctg tgactggtga gtactcaacc aagtcattct 6480
   gagaatagtg tatgcggcga ccgagttgct cttgcccggc gtcaacacgg gataataccg 6540
   cgccacatag cagaacttta aaagtgctca tcattggaaa acgttcttcg gggcgaaaac 6600
   tctcaaggat cttaccgctg ttgagatcca gttcgatgta acccactcgt gcacccaact 6660
   gatcttcagc atcttttact ttcaccagcg tttctgggtg agcaaaaaca ggaaggcaaa 6720
   atgccgcaaa aaagggaata agggcgacac ggaaatgttg aatactcata ctcttccttt 6780
   ttcaatatta ttgaagcatt tatcagggtt attgtctcat gagcggatac atatttgaat 6840
   gtatttagaa aaataaacaa ataggggttc cgcgcacatt tccccgaaaa gtgccacctg     6900
   acgtctaaga aaccattatt atcatgacat taacctataa aaataggcgt atcacgaggc     6960
   cctttcgtct tcaagaattg                                                 6980

## Beschreibung

Die Erfindung bezieht sich auf ein Verfahren zum Herstellen eines Ziel-Proteins, bei dem eine Zellkultur mit Wirtszellen einer Hefeart bereitgestellt werden, wobei die Wirtszellen eine DNA-Sequenz enthalten, die für das Ziel-Protein codiert, wobei die Wirtszellen mit Hilfe der für das Ziel-Protein codierenden DNA-Sequenz veranlasst werden, dass Ziel-Protein zu exprimieren, und wobei das Ziel-Protein aus der Zellkultur abgetrennt wird. Ferner bezieht sich die Erfindung auf die genannten Wirtszellen.

Verfahren der eingangs genannten Art, bei denen Hefearten der Gattungen Saccharomyces, Schizosaccharomyces, Kluyveromyces, Hansenula, Arxula, Schwanniomyces, Candida, Pichia oder Yarrowia verwendet werden, sind zum Herstellen einer Vielzahl von rekombinanten Ziel-Proteinen bekannt. Beispielsweise werden die Hefen Saccharomyces cerevisiae, Kluyveromyces lactis, Pichia pastoris oder Hansenula polymorpha verwendet. Hefen werden für die heterologe Proteinexpression insbesondere deshalb verwendet, weil sie die Vorteile kurzer Generationszeiten und geringer Nährmedienansprüche mit denen einer posttranslationalen Modifikation eines eukaryotischen Faltungs- und Sekretionsapparates vereinen. Bei einer Vielzahl von Ziel-Proteinen werden hohe Expressionsraten erreicht, wobei für die Expression des gewünschten Ziel-Proteins ein starker Promoter eingesetzt wird, der zugleich ein Promoter für die Expression eines Enzymproteins ist, dass an einer Hauptstoffwechselfunktion der Hefe beteiligt ist. Beispielsweise wird bei der Hefe Pichia pastoris der Promoter von Alkoholoxidase (AOX1), die für die Oxidation von Methanol verantwortlich ist, bei der rekombinanten Proteinexpression verwendet. Bei Hefen wird sowohl eine intrazelluläre als auch eine sekretorische Expression heterologer Proteine genutzt.

Während bei einer Reihe von Ziel-Proteinen ausreichend hohe Expressionsraten erzielt werden, wird bei anderen Ziel-Proteinen nur eine unzureichend geringe Expressionsrate erreicht. Darüber hinaus wurde festgestellt, dass einige sekretorisch exprimierte Proteine im sekretorischen Pfad eine unerwünschte posttranslationale Modifikation erfahren. Ferner wurde festgestellt, dass einige Ziel-Proteine eine unerwünschte Degradation erfahren.

Aufgabe der Erfindung ist es daher, eine höhere Ausbeute und einen korrekten Aufbau bei solchen Ziel-Proteinen zu erreichen, die bislang bei den oben genannten Hefe-Arten schwierig oder in unzureichender Menge oder Qualität zu exprimieren sind.

Diese Aufgabe wird erfindungsgemäß durch ein Verfahren mit den Merkmalen des Anspruchs 1 gelöst. Ferner wird diese Aufgabe durch Wirtszellen mit den Merkmalen des Anspruchs 13 gelöst.

Bei dem erfindungsgemäßen Verfahren zum Herstellen eines Ziel-Proteins wird zunächst eine Zellkultur mit Wirtszellen einer Hefeart der Gattungen Saccharomyces, Schizosaccharomyces, Kluyveromyces, Hansenula, Arxula, Schwanniomyces, Candida, Pichia oder Yarrowia bereitgestellt. Diese Wirtszellen enthalten wenigstens eine erste DNA-Sequenz, die für ein heterologes Ferritin-Protein codiert, und wenigstens eine zweite DNA-Sequenz, die für das Ziel-Protein codiert. Die wenigstens eine zweite DNA-Sequenz ist derart mit der wenigstens einen ersten DNA-Sequenz gekoppelt, dass bei der Expression der ersten und der zweiten DNA-Sequenz bzw. DNA-Sequenzen das Ziel-Protein an das zuvor synthetisierte Ferritin-Protein gekoppelt wird, so dass ein Verbund-Protein gebildet wird. Wenn hier davon gesprochen wird, dass die erste und die zweite DNA-Sequenz miteinander "gekoppelt" sind, so meint dies eine funktionelle Kopplung, welche zu dem Ergebnis der genannten Bildung eines Verbund-Proteins führt; die Kopplung ist aber nicht auf eine unmittelbare Aufeinanderfolge der beiden Sequenzen innerhalb einer einzigen DNA beschränkt. Der Schritt des Bereitstellens der Wirtszellen umfasst beispielsweise das Erzeugen eines entsprechenden Stammes, dem beispielsweise eine Erzeugung eines geeigneten Plasmid-Vektors vorausgeht, und die Vermehrung der Wirtszellen. Die Wirtszellen werden dann mit Hilfe der für das heterologe Ferritin-Protein codierenden ersten DNA-Sequenz und der für das Ziel-Protein codierenden zweiten DNA-Sequenz (beispielsweise durch Aktivierung eines Promoters durch Zugabe eines Induktors) veranlasst, das das Ferritin-Protein und das Ziel-Protein enthaltende Verbund-Protein zu exprimieren, wobei das Verbund-Protein in den Wirtszellen gehalten wird. Dann wird das Verbund-Protein aus der Zellkultur abgetrennt und schließlich das Ziel-Protein von dem Verbund-Protein abgetrennt.

Dementsprechend ist die erfindungsgemäße Wirtszelle eine Wirtszelle einer Hefeart der Gattungen Saccharomyces, Schizosaccharomyces, Kluyveromyces, Hansenula, Arxula, Schwanniomyces, Candida, Pichia oder Yarrowia, wobei die Wirtszelle wenigstens eine erste DNA-Sequenz enthält, die für ein heterologes Ferritin-Protein codiert, und wenigstens eine zweite DNA-Sequenz enthält, die für das Ziel-Protein codiert, wobei die zweite DNA-Sequenz derart mit der ersten DNA-Sequenz gekoppelt ist, dass bei der Expression der ersten und der zweiten DNA-Sequenz das Ziel-Protein an das zuvor synthetisierte Ferritin-Protein gekoppelt wird, so dass ein Verbund-Protein gebildet wird, und das Verbund-Protein in den Wirtszellen gehalten wird.

Ferritin ist ein in den meisten Zellarten höherer Wirbeltiere, einschließlich des Menschen, sowie der meisten anderen Organismen zu findendes Protein, welches den Haupt-Eisenspeicher bildet. Ferritine von Wirbeltieren umfassen zwei Arten von Untereinheiten, welche leichte Kette (L) und schwere Kette (H) genannt werden. Beim Menschen sind die leichte und die schwere Kette zu 55% homolog; sie unterscheiden sich in ihrer Größe und Oberflächenladung und ihren Eisenbeladungseigenschaften. Natürliches Ferritin bildet kugelartige Makromolekülpartikel, welche aus 24 strukturell gleichen Untereinheiten bestehen und welche in ihrem Inneren bis zu 4500 Eisenatome als ein Eisenoxyhydroxid-Polymer speichern können.

Rekombinante Ferritin-Proteine wurden in verschiedenen Systemen erzeugt, so beispielsweise in Escherichia coli (siehe: P. Santambrogio, S. Levi, A. Cozzi, E. Rovidaj, A. Albertini und P. Aros, "Production and Characterization of Recombinant Heteropolymers of Human Ferritin H and L Chains", in J. Biol. Chem., 268, 1993, S. 12744-12783), in Saccharomyces cerevisiae (siehe: Y. M. Shin, T. H. Kwon, K. S. Kim, K. S. Chae, D. H. Kim und M.S. Yang, "Enhanced Iron Uptake of Saccharomyces cerevisiae by Heterologous Expression of a Tadpole Ferritin Gene", in Appl. Environ. Microbiol. 67, 2001, S. 1280-1283), und in Pichia pastoris (siehe: J. L. Lee, H. S. Song, H. J. Kim, J. H. Park, D. K. Chung, C. S. Park, D. Jeung und H. Y. Kim, "Functional expression and production of human H-ferritin in Pichia pastoris", in Biotechnol. Lett. 25, 2003, S. 1019-1023). Es wurde gezeigt, dass durch heterologe Expression eines Kaulquappen-H-Ferritins die eisenspeichernden Eigenschaften von Saccharomyces cerevisiae um einen Faktor von 2,5 erhöht wurden. Bei einer humanes H-Ferritin exprimimierenden Pichia pastoris wurde sogar eine 9,6-fache Erhöhung der Eisenspeichereigenschaft berichtet (siehe Lee et al., ebenda).

Der Erfindung liegt der Gedanke zugrunde, ein Verbund-Protein aus dem Ferritin-Protein und dem Ziel-Protein zu exprimieren und sich dabei die bei dem Ferritin beobachtete hohe Expressionsrate und außerdem den Umstand zu Nutze zu machen, dass das Verbund-Protein, wie auch das Ferritin-Protein allein, in das Zytosol exprimiert wird und nicht in den sekretorischen Pfad gelangt. Auf diese Weise ist es möglich, Ziel-Proteine zu exprimieren, die anderenfalls nicht oder nicht in der gewünschten Rate exprimiert werden könnten. Hierbei hat sich gezeigt, dass man rekombinante Ferritin-Proteine, insbesondere humanes H-Ferritin (FTH1), auch bei einer methylotrophen Hefe, wie Hansenula polymorpha, mit einer außergewöhnlich hohen Expressionsrate herstellen konnte, welches sogar die des Schlüsselproteins des Methanolpfades übersteigt.

Die Ankopplung des Ziel-Proteins an das Ferritin-Protein erlaubt zudem eine relativ einfache Abtrennung des Verbund-Proteins aus der Kultur. Darüber hinaus wurde festgestellt, dass das Ferritin-Verbund-Protein in großen Mengen im Zytosol der Zellen stabil verbleibt, wobei die Zelle wächst und weiter produziert. Da das Verbund-Protein nicht in den sekretorischen Pfad gelangt, erhält man ein unglykosyliertes Ziel-Protein, wodurch eine unerwünschte hefe-typische Glykosylierung vermieden wird.

Bei einer bevorzugten Ausführungsform des erfindungsgemäßen Verfahrens ist die zweite DNA-Sequenz derart mit der ersten DNA-Sequenz gekoppelt, dass bei der Expression der ersten und der zweiten DNA-Sequenz das Ferritin-Protein N-terminal positioniert ist. Dies dient dazu, dass Verbund-Protein in das Zytosol der Hefezelle zu exprimieren.

Eine bevorzugte Weiterbildung des erfindungsgemäßen Verfahrens ist dadurch gekennzeichnet, dass die zweite DNA-Sequenz über eine dritte DNA-Sequenz mit der ersten DNA-Sequenz gekoppelt ist, wobei die dritte DNA-Sequenz für ein Kopplungspeptid codiert, wobei bei der Expression der ersten, der zweiten und der dritten DNA-Sequenz das Kopplungspeptid an das zuvor synthetisierte Ferritin-Protein und das Ziel-Protein an das zuvor synthetisierte Kopplungspeptid gekoppelt wird. Vorzugsweise umfasst das Kopplungspeptid ein Spacer-Peptid, wobei das Spacer-Peptid bei der Expression der ersten, der zweiten und der dritten DNA-Sequenz an das zuvor synthetisierte Ferritin-Protein gebunden wird. Insbesondere ist vorzugsweise vorgesehen, dass das Spacer-Peptid eine Glycin-Kette umfasst, die aus einer bis 10, vorzugsweise aus drei Glycin-Gruppen besteht. Durch das Spacer-Peptid wird eine gewisse räumliche Trennung zwischen dem Ferritin-Protein und dem Ziel-Protein erreicht, was zum einen die gegenseitige strukturelle Beeinflussung der beiden Bestandteile des Verbund-Proteins verringert und zum anderen dazu beiträgt, die Abtrennung des Ziel-Proteins von dem Verbund-Protein zu erleichtern.

Bei einer bevorzugten Weiterbildung dieses Verfahrens umfasst das Kopplungspeptid ein Trennstellen-Peptid, das eine von einem Enzym auflösbare Trennstelle bildet, wobei das Ziel-Protein an das Trennstellen-Peptid gebunden wird, wobei im Schritt d) die Trennstelle derart aufgelöst wird, dass das Ziel-Protein freigesetzt wird. Beispielsweise ist das Enzym eine Enteropeptidase und das Trennstellen-Peptid eine Enteropeptidase-Trennstelle. Das Trennstellen-Peptid umfasst zum Beispiel eine DDDDK-Aminosäuresequenz. Diese bevorzugte Ausführungsform erleichtert die Abtrennung des Ziel-Proteins. In Kombination mit der Trennstelle erhöht das Spacer-Peptid die Wahrscheinlichkeit, dass die Enteropeptidase-Trennstelle so eingebaut wird, dass sie nicht deaktiviert wird.

Bei einer bevorzugten Ausführungsform des erfindungsgemäßen Verfahrens codiert die erste DNA-Sequenz für eine schwere Ferritin-Kette. Vorzugsweise codiert die erste DNA-Sequenz für ein humanes FTH1-Protein.

Bei einer anderen bevorzugten Weiterbildung des erfindungsgemäßen Verfahrens werden im Schritt a) Wirtszellen einer methylotrophen Hefeart der Gattungen Hansenula oder Pichia bereitgestellt. Vorzugsweise werden Wirtszellen der Gattung Hansenula, insbesondere Wirtszellen der Hansenula polymorpha bereitgestellt. Es hat sich gezeigt, dass bei diesen Hefearten besonders hohe Ausbeuten der Ferritin-Verbundproteine erreicht werden können.

Eine bevorzugte Ausführungsform des Verfahrens ist dadurch gekennzeichnet, dass das Ziel-Protein ein heterologes funktionelles Protein ist, wobei das Ziel-Protein homolog nicht in einer Kopplung mit dem Ferritin-Protein auftritt. Dies bedeutet, dass das Ziel-Protein ein funktionelles Protein ist, welches üblicherweise nicht in Kombination mit dem Ferritin-Protein exprimiert wird.

Das Ziel-Protein ist vorzugsweise ein humanes Protein. Eine bevorzugte Ausführungsform ist dadurch gekennzeichnet, dass das Ziel-Protein eine Serinprotease, insbesondere matures humanes α1-Antichymotrypsin, umfasst. Eine andere bevorzugte Ausführungsform ist dadurch gekennzeichnet, dass das Ziel-Protein ein Peptidhormon, insbesondere matures humanes Interleukin oder humanes Parathyroid-Hormon oder ein Teilfragment davon, umfasst. Eine weitere bevorzugte Ausführungsform ist dadurch gekennzeichnet, dass das Ziel-Protein ein Interferon, insbesondere matures humanes Interferon-α, umfasst.

Vorteilhafte und/oder bevorzugte Weiterbildungen der Erfindung sind in den Unteransprüchen gekennzeichnet.

Im Folgenden wird die Erfindung anhand bevorzugter Ausführungsformen näher erläutert. In den Zeichnungen zeigen:
Fig. 1: eine Plasmidkarte eines bei der Herstellung der erfindungsgemäßen Wirtszellen verwendeten H. polymorpha FTH1-H6-Expressionsplasmids pFPMT-FTH1-H6;
Fig. 2A und 2B: SDS-PAGE und Western Blot des Kulturüberstands und der intrazellulären löslichen und unlöslichen Fraktionen eines FTH1-H6 produzierenden Stamms und eines Negativkontrollstamms, wobei Fig. 2A die mit Ponceau Red angefärbte Nitrocellulose-Membran zeigt, wobei alle transferierten Proteinbanden angezeigt werden, und wobei Fig. 2B dieselbe Membran nach der immunologischen Detektion von Proteinen mit His Tag zeigt;
Fig. 3: SDS-PAGE und Western Blot von Gesamtzellextrakten rekombinanter Hansenula-polymorpha-Stämme, die Ferritin-mhACT - oder Ferritin-mhIL6-Verbund-Proteine synthetisieren;
Fig. 4: SDS-PAGE und Western Blot der intrazellulären löslichen und unlöslichen Fraktionen rekombinanter Hansenula-polymorpha-Stämme, die Ferritin-mhIFNα-2a-Verbund-Proteine synthetisieren;
Fig. 5: SDS-PAGE der intrazellulären löslichen und unlöslichen Fraktionen von Stammgemischen oder Stämmen, die FTH1-G3-Ek-PTH-Verbund-Proteine oder FTH1-H6-Protein synthetisieren;
Fig. 6: eine Abschätzung der Ausbeuten von intrazellulären löslichen FTH1-H6 und FTH1-G3-Ek-PTH mittels SDS-PAGE anhand eines Vergleichs mit einem Standardprotein; und
Fig. 7A und 7B: eine Analyse von Partikelcharakter und Eisenbindung von intrazellulärem löslichem FTH1-H6 aus H. polymorpha, wobei Fig. 7A eine Anfärbung mit Coomassie Brilliant Blue G250 zur Anzeige von Proteinbanden und Fig. 7B eine Anfärbung durch Preußisch-Blau-Reaktion zur Anzeige von Fe(III) zeigt.

### Experimentelle Vorgehensweise und Ergebnisse

### 1. Erzeugung eines FTH1-Expressionsplasmids

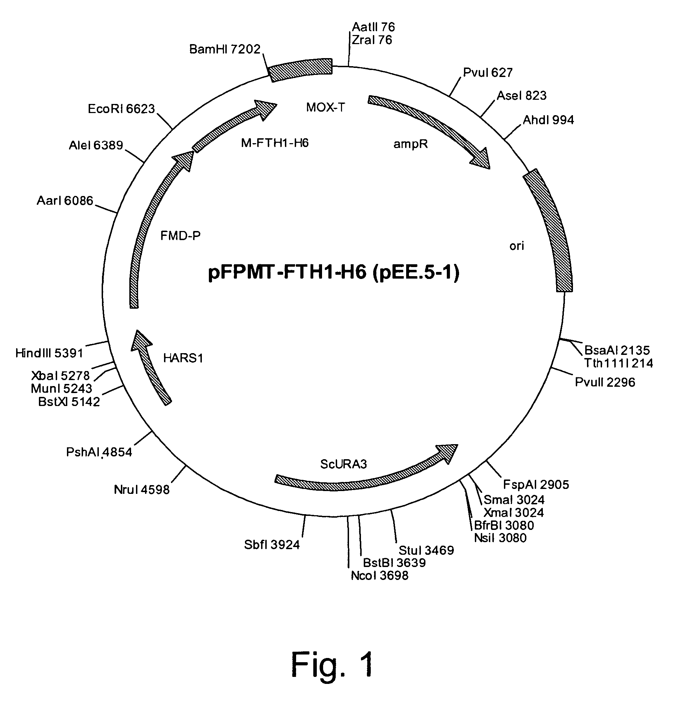

Eine Aminosäuresequenz der schweren Kette des humanen Ferritin wurde aus der NCBI-Proteindatenbank gewonnen (NP_002023). Es wurde ein synthetischer offener Leserahmen (ORF - Open Reading Frame) entwickelt und für die Expression in Hansenula polymorpha optimiert (GeneArt). An das 3'-Ende der Sequenz wurden die Codons eines Sechs-Histidin-His-Tags (H6) hinzugefügt, um eine Analyse und Reinigung durch kommerziell verfügbare Kits und Antikörper zu ermöglichen. Dieser FTH1-H6-ORF wird flankiert von Erkennungssequenzen EcoRI (5'-Ende) und BamHI (3'-Ende), die mit dem kurzen Polylinker des H. polymorpha-Expressionsvektors pFPMT121 kompatibel sind. Um das Expressionsplasmid pFPMT-FTH1-H6 zu erzeugen, wurden das 6,968 kb EcoRI/BamHI-Fragment von pFPMT-chLysSS-6H-RVG und das 0,579 kb EcoRI/BamHI-Fragment des GeneArt-Plasmids 0830183_FTH1-H6_pMA miteinander verknüpft, und E.coli DH10β wurden mit dieser Probe transformiert. Zehn Ampicillin-resistente E.coli-Klone wurden hinsichtlich des Vorhandenseins von restriktionspositiven Plasmid-Klonen analysiert. Die Plasmid-DNA von neun dieser Klone zeigten das erwartete Restriktionsmuster. Das Insert eines dieser Klone, pEE.5-1, wurde mit den Sequenzierprimern FMD-Pf und MOX-Tr (Qiagen, 27.11.2008) sequenziert. Es wurde gefunden, dass die Sequenz des Inserts vollständig korrekt ist. Die Abbildung von pFPMT-FTH1-H6 (pEE.5-1) ist in Figur 1 gezeigt.

Fig. 1 zeigt eine Plasmidkarte des Hansenula polymorpha FTH1-H6-Expressionsplasmids pFPMT-FTH1-H6 (pEE.5-1), wobei für die Elemente folgende Abkürzungen verwendet werden:
- FMD-P:: Promoter des H. polymorpha Formiatdehydrogenasegens (FMD);
- MOX-T:: Terminator des H. polymorpha Methanoloxidasegens (MOX);
- ampR:: Ampicillin-Resistenzgen;
- ori:: Replikationsursprung aus pBR322;
- ScURA3:: URA3-Gen aus Saccharomyces cerevisiae für die Selektion in H. polymorpha ura3-Stämmen;
- HARS1:: Autonom replizierende Sequenz aus H. polymorpha;
- FTH1-H6:: Fusions-ORF kodierend für humanes Ferritin (H-Kette) mit C-terminalem His-Tag (vgl. Sequenzprotokoll, SEQ ID NO. 1).

Das auf die oben genannte Weise gewonnene FTH1-Expressionsplasmid bildete die Basis für die nachfolgend beschriebene Erzeugung eines rekombinanten Hansenula-polymorpha-Stamms, welcher FTH1-H6 exprimiert.

Darüber hinaus bildete dieses Plasmid einen Ausgangspunkt für die unten im Abschnitt 3. beschriebene Erzeugung von Expressionsplasmiden, welche Verbund-Proteine kodieren, bei denen das Ferritin-Protein FTH1 mit bestimmten Ziel-Proteinen über ein Kopplungspeptid verbunden ist.

### 2. Erzeugung eines rekombinanten FTH1-H6 exprimierenden Hansenula polymorpha-Stamms

Der Hansenula-polymorpha-Wirtsstamm RB11 (relevanter Genotyp ura3) wurde mit pFPMT-FTH1-H6 (pEE.5-1) transformiert, und 36 Uracil-prototrophische einzelne Kolonien wurden für Stammerzeugungen isoliert. Zu diesem Zweck lies man die Klone für 48 h bei 37 °C als 3ml-YNB/Glukose-Kulturen bei 180rpm wachsen (erster Durchlaufschritt). Nachfolgend wurden 20 µl Aliquots dieser Kulturen auf frische YNB/Glukose (3 ml) übertragen, welche erneut für 48 h bei 37 °C wuchsen (zweiter Durchlaufschritt). Nach dem vierten Durchlaufschritt wurden 20 µl Aliquots der verschiedenen Kulturen verwendet, um 3 ml Vollmediums (YPD) zu beimpfen. Die Kulturen wurden für 24 h bei 37 °C inkubiert (180rpm; erster Stabilisierungsschritt). Ein zweiter Stabilisierungsschritt wurde durchgeführt; danach wurden Aliquots der verschiedenen Kulturen verwendet, um 3 ml YNB/Glukose zu beimpfen. Diese Kulturen wurden für 48 h bei 37 °C inkubiert. Nach diesem abschließenden Auswahlschritt wurden 3 µl jeder Kultur auf eine YNB/Glukose-Platte gebracht (dotted). Die sich ergebenden Dots wurden Pools genannt; sie stellen Mischungen von Stämmen dar, welche typischerweise ähnliche Eigenschaften haben, aber eine gewisse Mikroheterogenität in ihren individuellen Expressionsraten aufweisen. Wenn das Screening zu einer Identifikation produktiver Pools führt, müssen diese Pools in einzelne Stämme separiert werden, und ein Set von Stämmen pro Pool muss erneut analysiert werden. Der theoretische Hintergrund für die angewendete Art der Stammerzeugung ist folgender. Während der Durchlaufschritte wird das Plasmid in das Genom der verschiedenen Zellen integriert. Die Stabilisierungsschritte dienen als Werkzeug, um einen Plasmidverlust in Zellen mit episomalem Plasmid zu erzwingen. So können nach dem Aufbringen auf den Platten, die selektive Medien enthalten, nur solche Zellen, welche in das Genom integrierte Plasmidkopien enthalten, wachsen, um Kolonien zu bilden.

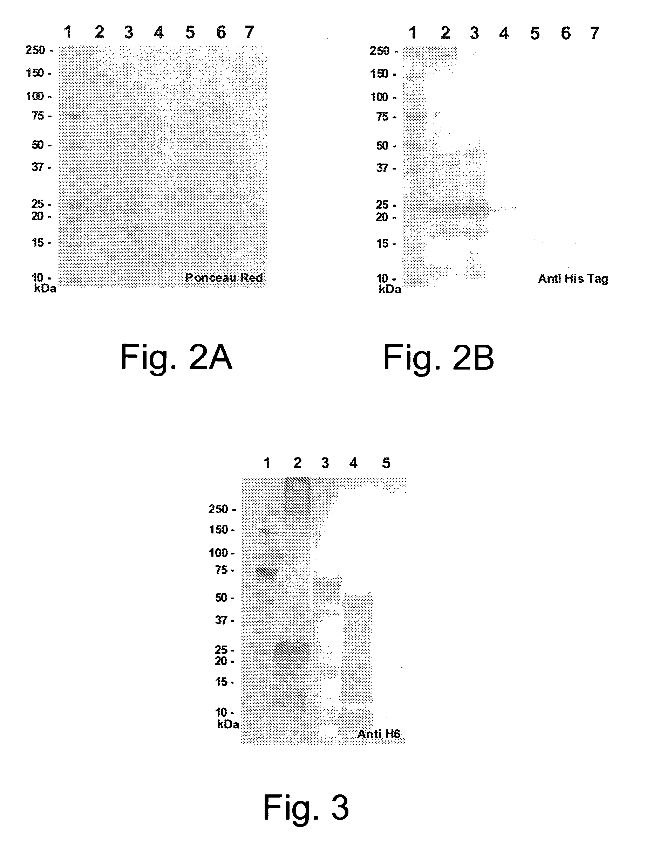

15 Pools der Serien pEE.5-1/RB11 wurden einer Derepression/MeOH-Induktion im 3 ml-Teströhrchenmaßstab unterzogen. Die intrazellulären löslichen und unlöslichen Fraktionen wurden präpariert und durch SDS-PAGE und Western-Blot analysiert. Der Best-Producer-Pool wurde identifiziert und auf einer YNB/Glukose-Platte separiert. Einzelne Kolonien dieser Platte wurden isoliert und als Stämme definiert. Ein Stamm wurde weitergehenden Analysen unterzogen. Figur 2 zeigt einen Western-Blot des intrazellulären Materials dieses Stamms pEE.5-1/RB11 #2-1 nach Derepression/MeOH-Induktion.

Fig. 2 zeigt SDS-PAGE und Western Blot des Kulturüberstands und der intrazellulären löslichen und unlöslichen Fraktionen eines FTH1-H6-produzierenden Stamms und eines Negativkontrollstamms. Die angegebenen Stämme wurden einer Methanolinduktion im 3ml-Maßstab unterzogen. Zu diesem Zweck wurden 3ml YPD-Medium jeweils mit einer einzelnen Kolonie beimpft. Diese Vorkulturen wurden für 16 Stunden bei 37°C geschüttelt (180rpm). Anschließend wurden die Zellen abzentrifugiert und in 3ml sterilem Wasser resuspendiert. 20µl dieser Suspensionen wurden in 3ml YNB/2% Glycerin überführt. Diese Kulturen wurden für 48 Stunden geschüttelt (37°C, 180rpm). Während dieser Phase wird der FMD-Promotor dereprimiert. Danach wurden die Zellen abzentrifugiert und in je 3ml YNB/1% Methanol resuspendiert. Diese Kulturen wurden wie oben beschrieben für weitere 24 Stunden inkubiert (Methanolinduktion). Anschließend wurden die Kulturüberstände und die intrazellulären löslichen und unlöslichen Fraktionen präpariert und mittels SDS-PAGE und anschließendem Western Blot analysiert. Zur Detektion wurde ein His Tag-spezifischer Antikörper (Kaninchen) in Kombination mit einem AP-konjugierten Ziege-anti-Kaninchen-Antikörper und NBT/BCIP als Chromogen eingesetzt. Fig. 2A zeigt die mit Ponceau Red angefärbte Nitrocellulose-Membran, wobei alle transferierten Proteinbanden angezeigt werden. Fig. 2B zeigt dieselbe Membran nach der immunologischen Detektion von Proteinen mit His Tag. Der in diesem Experiment eingesetzte Negativkontrollstamm enthält den Vektor pFPMT121 ohne Insert.

Das Beladungsschema bei den in Figuren 2A und 2B gezeigten Spuren ist in der nachfolgenden Tabelle 1 dargestellt ("FTH1-H6" bezeichnet die für humanes Ferritin (H-Kette) codierende Fusions-ORF mit C-terminalem His-Tag; vgl. Sequenzprotokoll, SEQ ID NO. 1)).

**Tabelle 1**

| | | | | |
|---|---|---|---|---|
| 1 | Precision Plus Protein Standards | | | 10µl |
| 2 | pEE.5-1/RB11 #2-1 | FTH1-H6 | intrazelluläre lösliche Fraktion | 14µl |
| 3 | pEE.5-1/RB11 #2-1 | FTH1-H6 | intrazelluläre unlösliche Fraktion | 14µl |
| 4 | pEE.5-1/RB11 #2-1 | FTH1-H6 | Kulturüberstand | 14µl |
| 5 | pFPMT121/RB11 | Negativkontrollstamm | intrazelluläre lösliche Fraktion | 14µl |
| 6 | pFPMT121/RB11 | Negativkontrollstamm | intrazelluläre unlösliche Fraktion | 14µl |
| 7 | pFPMT121/RB11 | Negativkontrollstamm | Kulturüberstand | 14µl |

Das FTH1-H6-Fusionsprotein hat ein kalkuliertes Molekulargewicht von 22,05 kDa. Figur 2A zeigt die NC-Membran nach einem Semi-Dry-Transfer in dem Färbemittel Ponceau Red. Dieses Verfahren visualisiert sämtliche Proteinbanden. In den Spuren 2 und 3 sind die intrazelluläre lösliche bzw. unlösliche Fraktion des Ferritinstamms gezeigt. In beiden Spuren wurde ein Band sehr hoher Intensität, das einem scheinbaren Molekulargewicht von etwa 23 kDa entspricht, beobachtet, welches auf den Anti-His-Tag-Antikörper anspricht (Figur 2B, Spuren 2 und 3) und somit dem rekombinaten Ferritin entspricht. Das scheinbare und das berechnete Molekulargewicht stimmen überein, und die Tatsache, dass das Material durch den verwendeten Antikörper erkannt wird, zeigt, dass zumindest der C-terminale Teil des Proteins intakt sein muss. Der Überstand der Kultur pEE.5-1/RB11 # 2-1 zeigt kein Ferritin, was konsistent ist mit dem intrazellulären Verweilen des Fusions- oder Verbund-Proteins (vgl. Spuren 4 in Figuren 2A und 2B). Der Negativkontrollstamm pFPMT121/RB11 zeigt kein Ferritin (Spuren 5 bis 7) in den Figuren 2A und 2B).

Zusammenfassend ist festzustellen, dass
1. die Expression des zytosolischen FTH1-H6 enorm hohe Mengenanteile des heterologen Proteins zur Verfügung stellt. Das Ferritin-Band ist selbst nach MeOH-Induktion bei Weitem das dominante Band in der SDS-PAGE, welches sogar die Bänder übertrifft, die den Proteinen des Methanol-Pfades entsprechen, wie beispielsweise Moxp und Fmdp.
2. etwa 30 % des Proteins in der intrazellulären löslichen Fraktion und die übrigen 70 % in der intrazellulären unlöslichen Fraktion gefunden wurden.
3. Erzeugung von Expressionsplasmiden, die mit verschiedenen Zielproteinen verbundenes FTH1 codieren

Wie einleitend ausgeführt, liegt der Erfindung der Gedanke zugrunde, dass die hohen Expressionsraten des rekombinanten FTH1-H6-ORF genutzt werden können, um sogenannte Problemproteine als FTH1-Fusionsproteine zu exprimieren. Problemproteine sind solche, bei denen die Expressionsrate zu gering ist oder welche unerwünschte posttranslationale Modifikationen erfahren oder bei denen eine unerwünschte Degradation festzustellen ist.

Bei der Schaffung der Fusionsproteine ist folgendes zu beachten:
Erstens sollte der FTH1-H6-Teil des Fusionsproteins N-terminal positioniert sein, um ein Verbleiben im Zytosol zu erzielen.

Zweitens sollten, um einen flexiblen Übergang zwischen FTH1 und dem Zielprotein zu erreichen, beispielsweise drei Glycinreste (G3) zwischen den Fusionspartnern hinzugefügt werden.

Drittens sollte, um die Option des Freigebens des Zielproteins zu erleichtern, eine Enterokinase-Schnittstelle (Ek; DDDDK) oder eine andere Spaltstelle zwischen dem G3-Spacer und der Zielproteinsequenz positioniert sein.

Die Struktur des Fusionsproteins wäre dann: FTH1-G3-Ek-Ziel-protein (+/- Tag). Um das Konzept zu testen, wurden vier verschiedene Proteine als Fusionspartner gewählt:
1. ein maturer humaner α1-Antichymotrypsin-Precursor (hACT), wobei die Sequenz aus der Zugriffsnummer P01011 gewonnen wurde;
2. matures humanes Interleukin 6 (hIL6), wobei die Sequenz aus der Zugriffsnummer NP_000591 gewonnen wurde;
3. matures humanes Interferon α-2a (IFNα-2a); und
4. Teilfragment des humane Parathyroid-Hormaons (PTH) (Zugriffsnummer NP_000306.1).

Die Plasmidkonstruktionen sind in der nachfolgenden Tabelle 2 zusammengefasst.

**Tabelle 2:**

| Fusionsprotein | Fragment 1 | Fragment 2 | Fragment 3 | Charakterisierter Plasmid-Klon |
|---|---|---|---|---|
| FTH1-G3-Ek-hACT-H6 | pEE.1-1 BsrGI/EcoRI 8,083 kb | pEE.5-1 EcoRI/BstEII 0,453 kb | GeneArt plasmid 0908426_256_pMA BstEII/BsrGI 0,234 kb | pFPMT-FTH1-G3-Ek-mhACT-H6 (pEE.11-1) |
| FTH1-G3-Ek-hIL6-H6 | pEE.4-1 ClaI/EcoRI 7,471 kb | pEE.5-1 EcoRI/BstEII 0,453 kb | GeneArt plasmid 0908655_348_pMA-RQ BstEII/ClaI 0,202 kb | pFPMT-FTH1-G3-Ek-mhIL6-H6 (pEE.12-2) |
| FTH1-G3-Ek-IFNα-2a | pFW.2-5 BgIII/EcoRI 7,406 kb | pEE.5-1 EcoRI/BstEII 0,453 kb | GeneArt plasmid 0908655_348_pMA-RQ BstEII/BgIII 0,193 kb | pFPMT-FTH1-G3-Ek-IFNalpha-2a (pEE.13-7) |
| FTH1-G3-Ek-PTH | pEE.5-1 EcoRI/BamHI 6.968kb | pEE.5-1 EcoRI/BstEII 0,453 kb | GeneArt plasmid 0939988_EE14-15 BstEII/BamHI 0.234kb | pFPMT-FTH1-G3-EK-PTH (per.14-2) |

Die Spalte "Fusionsprotein" kennzeichnet die Zusammensetzung der verschiedenen Fusionsproteine. Es wurden folgende Abkürzungen verwendet:
FTH1: humanes Ferritin, schwere Kette;
G3: 3-Glycin-Spacer;
Ek: Enterokinase- bzw. Enteropeptidase-Schnittstelle (DDDDK);
H6: 6-Histidin-His-Tag;
hACT: humanes α1-Antichymotrypsin;
hIL6: humanes Interleukin 6
IFN α-2a: Interferon- α-2a;
PTH: Parathyroid-Hormaon;
m: matur (reif);
h: human;
EcoRI, BstEII, BamHI, BgIII, ClaI, BsrGI: für die Fragmentgenerierungen eingesetzte Restriktionsenzyme.

Die Spalten "Fragment 1" bis "Fragment 3" listen die Plasmid-Fragmente auf, die bei den jeweiligen Verknüpfungen beteiligt sind. In sämtlichen Fällen wurde eine 3-Fragment-Ligation ausgeführt. Die DNA-Fragmente wurden über 1 %-Agarosegele isoliert. Proben jeder Ligationsreaktion wurden in E.coli DH10β eingeführt. Ein bis zwei restriktionspositive Plasmid-Klone pro Serie wurden zum Sequenzieren des Inserts ausgewählt. Die letzte Spalte zeigt den jeweiligen Zielplasmidklon mit korrekter Insertsequenz, der für nachfolgende Hefestammgenerierungen eingesetzt wurde.

### 4. Erzeugung von H. polymorpha-Stämmen, die verschiedene FTH1-Verbund-Proteine exprimieren

### 4.1 mhACT und mhIL6

Die Expressionsplasmide pFPMT-FTH1-G3-Ek-mhACT-H6 (pEE.11-1) und pFPMT-FTH1-G3-Ek-mhIL6-H6 (pEE.12-2) (vgl. Tabelle 2) wurden in den H. polymorpha Stamm RB11 eingebracht, und je 24 sich ergebende Uracil-prototrophe Kolonien wurden für Stammerzeugungen verwendet, wie sie in dem obigen Abschnitt 2. beschrieben worden ist (mit der Ausnahme, dass die Anzahl der Durchlaufschritte auf drei reduziert wurde). Die Identifikation des Best-Producer-Stamms wurde wie oben für FTH1-H6 beschrieben, ausgeführt. Wiederum wurde ein His-Tag-spezifischer primärer Antikörper zur Erfassung der Fusionsproteinbänder verwendet. Ein starker Erzeugerstamm jeder Serie und der FTH1-H6-Expressionsstamm pEE.5-1/RB11 #2-1 (siehe oben, Abschnitt 2.) wurden parallel einer Derepression/MeOH-Induktion in dem 3 ml-Teströhrchenmaßstab ausgesetzt, und die Gesamtzellextrakte wurden durch SDS-PAGE/Western-Blot analysiert. Das Ergebnis ist in Figur 3 dargestellt.

Fig. 3 zeigt eine SDS-PAGE und einen Western Blot von Gesamtzellextrakten von rekombinanten H. polymorpha-Stämmen, die FTH1-G3-EK-mhACT-H6 oder -mhIL6-H6 exprimieren. Die angegebenen Stämme wurden einer Methanolinduktion unterzogen. Anschließend wurden die Gesamtzellextrakte präpariert und mittels SDS-PAGE und anschließendem Western Blot analysiert. Die experimentellen Prozeduren wurden oben in Verbindung mit Fig. 2 beschrieben.

Das Beladungsschema bei den in Figur 3 gezeigten Spuren ist in der nachfolgenden Tabelle 3 angegeben (Abkürzungen: mhACT: matures humanes α1-Antichymotrypsin; mhIL6: matures humanes Interleukin 6; G3: Spacer-Peptid bestehend aus drei Glycinresten; Ek: Enterokinase-Erkennungsstelle; H6: Sechs-Histidin-His Tag):

**Tabelle 3:**

| | | | | |
|---|---|---|---|---|
| 1 | Precision Plus Protein Standards | | | 10µl |
| 2 | pEE.5-1/RB11 #2-1 | FTH1-H6 | Gesamtzellextrakt | 30µl |
| 3 | pEE.11-1/RB11 #1-3 | FTH1-G3-Ek-mhACT-H6 | Gesamtzellextrakt | 30µl |
| 4 | pEE.12-2/RB11 #1-1 | FTH1- G3-Ek-mhIL6-H6 | Gesamtzellextrakt | 30µl |
| 5 | pFPMT121/RB11 | Negativkontrollstamm | Gesamtzellextrakt | 30µl |

Bei sowohl FTH1-G3-Ek-mhACT-H6 als auch FTH1-G3-Ek-mhIL6-H6 konnten spezifische Produktbänder der erwarteten Molekulargewichte erzielt werden (vgl. Figur 3, Spuren 3 und 4). Jedoch erreichten bei diesem Experiment die Ausbeuten nicht diejenigen, die bei FTH1-H6 allein beobachtet wurden (vgl. Spur 2). Es ist ein gut bekanntes Phänomen bei der rekombinanten Genexpression im Hansenula polymorpha, dass infolge der ungerichteten Mehr-Kopien-Integration des Expressionsvektors die beobachteten Produktivitäten signifikant zwischen einzelnen Experimenten variieren können.

Nachfolgende Analysen erbrachten, dass in beiden Fällen etwa 90 % der Fusionsproteine in der intrazellulären unlöslichen Fraktion gefunden worden. Eine Kulturoptimierung kann hilfreich sein, um die Produktivität dieser Stämme zu verbessern. Die Optimierung von Zellauflösungsbedingungen könnte zu einer Erhöhung des löslichen Produktes führen.

Für sowohl IL6 als auch ACT kann die Expression als Ferritin-Fusionsprotein Vorteile bieten:
1. Die bekannte Expression von sekretorischen mhIL6 ergab, dass eine Subpopulation der Moleküle N-glykosyliert ist. Die Daten bei dem FTH1-G3-Ek-mhIL6-H6-Fusionsprotein markieren einen erfolgreichen Beweis, dass das Prinzip der Verwendung des Ferritins als Fusionspartners zum Erzeugen unglykosylierten mhIL6 funktioniert.
2. Im Falle des mhACT führte die zuvor durchgeführte sekretorische Expression zu schlecht verarbeitetem Material. Die intrazellulare Expression des FTH1-G3-Ek-mhACT-H6-Fusionsproteins schafft die Möglichkeit, reifes ACT korrekter Größe durch Trennen mit Enterokinase freizusetzen.

### 4.2 mhIFNα-2a

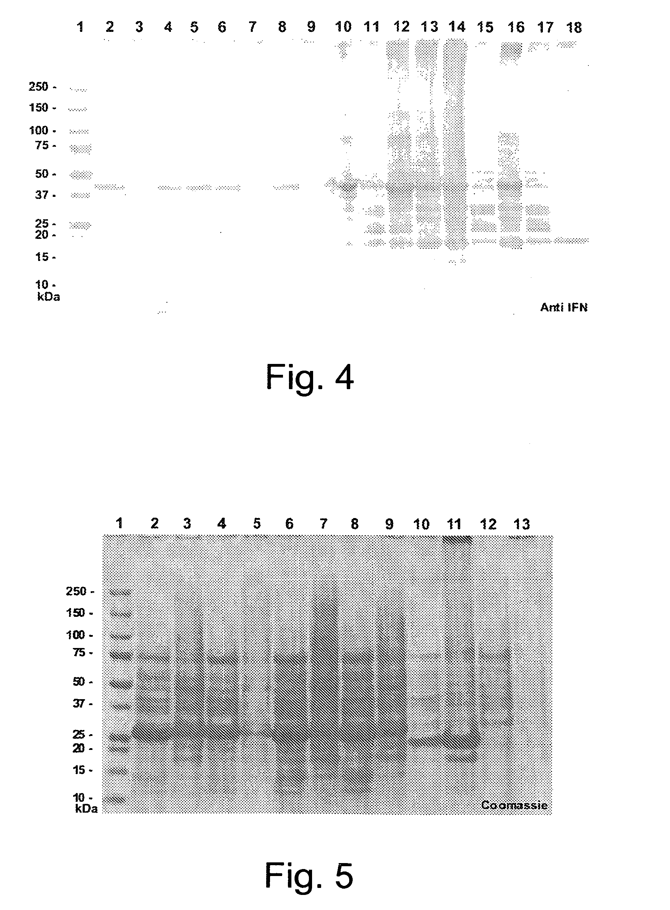

Die Stammerzeugung für die pFPMT-FTH1-G3-Ek-mhIFNα-2a-Serien (pEE.13-7/RB11) wurde wie oben beschrieben ausgeführt (drei Durchlaufschritte). Jedoch wurden in diesem Falle nur Pools, aber keine einzelnen Stämme analysiert. Figur 4 zeigt die Analyse der intrazellulären löslichen und unlöslichen Fraktionen von sieben resultierenden Pools bei Derepression/MeOH-Induktion in dem 3 ml-Teströhrchenmaßstab, wie es oben beschrieben ist. Für die Detektion wurde ein IFNα-spezifischer Antikörper verwendet.

Fig. 4 zeigt SDS-PAGE und Western Blot der intrazellulären löslichen und unlöslichen Fraktionen von rekombinanten H. polymorpha-Stammgemischen, die FTH1-IFNα-2a-Fusionsgene exprimieren. Die genannten Stammgemische wurden einer Methanolinduktion unterzogen. Anschließend wurden die löslichen und unlöslichen intrazellulären Fraktionen mittels SDS-PAGE und Western Blot untersucht, wie es oben zu Fig. 2 beschrieben ist.

Das Beladungsschema ist in der nachfolgenden Tabelle 4 angegeben (Abkürzungen: mhIFNα-2a: matures humanes Interferon α-2a; G3: Spacer-Peptid bestehend aus drei Glycinresten; Ek: Enterokinase-Erkennungsstelle; H6: Sechs-Histidin-His Tag).

**Tabelle 4:**

| | | | | |
|---|---|---|---|---|
| 1 | Precision Plus Protein Standards | | | 10µl |
| 2 | pEE.13-7/RB11 #1 | FTH1-G3-Ek-mhIFNα-2a | intrazelluläre lösliche Fraktion | 30µl |
| 3 | pEE.13-7/RB11 #2 | FTH1-G3-Ek- mhIFNα-2a | intrazelluläre lösliche Fraktion | 30µl |
| 4 | pEE.13-7/RB11 #3 | FTH1-G3-Ek- mhIFNα-2a | intrazelluläre lösliche Fraktion | 30µl |
| 5 | pEE.13-7/RB11 #4 | FTH1-G3-Ek-mhIFNα-2a | intrazelluläre lösliche Fraktion | 30µl |
| 6 | pEE.13-7/RB11 #5 | FTH1-G3-Ek-mhIFNα-2a | intrazelluläre lösliche Fraktion | 30µl |
| 7 | pEE.13-7/RB11 #6 | FTH1-G3-Ek-mhIFNα-2a | intrazelluläre lösliche Fraktion | 30µl |
| 8 | PEE.13-7/RB11 #7 | FTH1-G3-Ek-mhIFNα-2a | intrazelluläre lösliche Fraktion | 10µl |
| 9 | pFPMT121/RB11 | Negativkontrollstamm | intrazelluläre lösliche Fraktion | 30µl |
| 10 | pEE.13-7/RB11 #1 | FTH1-G3-Ek-mhIFNα-2a | intrazelluläre unlösliche Fraktion | 10µl |
| 11 | pEE.13-7/RB11 #2 | FTH1-G3-Ek-mhIFNα-2a | intrazelluläre unlösliche Fraktion | 10µl |
| 12 | pEE.13-7/RB11 #3 | FTH1-G3-Ek-mhIFNα-2a | intrazelluläre unlösliche Fraktion | 10µl |
| 13 | pEE.13-7/RB11 #4 | FTH1-G3-Ek-mhIFNα-2a | intrazelluläre unlösliche Fraktion | 10µl |
| 14 | pEE.13-7/RB11 #5 | FTH1-G3-Ek-mhIFNα-2a | intrazelluläre unlösliche Fraktion | 10µl |
| 15 | pEE.13-7/RB11 #6 | FTH1-G3-Ek-mhIFNα-2a | intrazelluläre unlösliche Fraktion | 10µl |
| 16 | pEE.13-7/RB11 #7 | FTH1-G3-Ek-mhIFNα-2a | intrazelluläre unlösliche Fraktion | 10µl |
| 17 | pFPMT121/RB11 | Negativkontrollstamm | intrazelluläre unlösliche Fraktion | 10µl |
| 18 | Interferon α-2a Standard | | | 20µl |

Ein indirekter Vergleich erbrachte, dass die für die stärksten Pools, die FTH1-G3-Ek-mhIFNα-2a exprimieren, beobachteten Ausbeuten (siehe beispielsweise Figur 4, Spur 12) in der Größenordnung waren, die für den Stamm pEE.5-1/RB11 #2-1 (der FTH1-H6 exprimiert, siehe Figur 2) beobachtet wurde. Jedoch wurde, wie in den Fällen der FTH1-G3-Ek-mhACT-H6- und FTH1-G3-Ek-mhIL6-H6-exprimierenden Stämme, die Mehrheit des Produkts in der intrazellulären unlöslichen Fraktion bobachtet (Figur 4, vgl. Spuren 2 bis 8 und 10 bis 16). Wiederum kann eine Optimierung der Zellauflösungsbedingungen hilfreich sein, um den Anteil des löslichen Produkts zu erhöhen.

Es ist bekannt, IFNα-2a effizient in sekretorischer Form in H. polymorpha zu erzeugen, was einen beträchtlichen Prozentsatz inkorrekt verarbeiteter Vorformen (Precursor) zeigt, was es sehr schwierig macht, korrektes IFNα-2a zu reinigen. Eine intrazellulare Produktion und eine In-Vitro-Reifung überwindet dieses Problem, da die korrekte Form aus dem FTH1-G3-Ek-IFNα-2a-Fusionsprotein durch Enterokinase-Digestion freigesetzt werden kann.

### 4.3 PTH

In den voranstehenden Abschnitten wurde gezeigt, dass Fusionsproteine, die aus FTH1-G3-Ek-mhACT-H6, -mhIL6-H6 und -mhIFNα-2a bestehen, in H. polymorpha in beträchtlichen Mengen erzeugt werden könnten. Während diese Zielproteine auch als sekretorische MFα-Prepro-Fusionsproteine exprimiert werden könnten, sind das humane Parathyroid-Hormon sowie Teilfragmente davon(PTH) als Problemproteine bekannt. Ein Teilfragment des Parathyroid-Hormons wird zwar exprimiert und sekretiert, aber rapide in dem Kulturrückstand abgebaut.

Es wurde ein Expressionsplasmid pFPMT-FTH1-G3-Ek-PTH (pEE.14-2; vgl. Tabelle 1) in H. polymorpha RB11 eingeführt, und eine Reihe von 36 Pools wurde erzeugt. Figur 5 zeigte eine SDS-PAGE-Analyse von vier dieser Pools in direktem Vergleich mit dem FTH1-H6-Stamme pEE.5-1/RB11 #2-1 und einem Negativkontrollstamm bei Derepression/MeOH-Induktion in YNB-Medium.

Fig. 5 zeigt SDS-PAGE der intrazellulären löslichen und unlöslichen Fraktionen von Stammgemischen oder Stämmen, die FTH1-G3-Ek-PTH oder FTH1-H6 exprimieren. Methanolinduktion und Präparation der intrazellulären löslichen und unlöslichen Fraktionen der angegebenen Stämme und Stammgemische erfolgte so wie es zu Fig. 2 beschrieben wurde. Nach SDS-PAGE wurde das Proteingel mit Coomassie Brilliant Blue G250 angefärbt.

Das Beladungsschema ist in der nachfolgenden Tabelle 5 angegeben (Abkürzungen: PTH: Teilfragment des humanen Parathyroid-Hormaons; alle weiteren: siehe oben zu Fig. 3).

**Tabelle 5:**

| | | | | |
|---|---|---|---|---|
| 1 | Precision Plus Protein Standards | | | 10µl |
| 2 | pEE.14-2/RB11 #9 | FTH1-G3-Ek-PTH | intrazelluläre lösliche Fraktion | 17,5µl |
| 3 | pEE.14-2/RB11 #9 | FTH1-G3-Ek-PTH | intrazelluläre unlösliche Fraktion | 17,5µl |
| 4 | pEE.14-2/RB11 #15 | FTH1-G3-Ek-PTH | intrazelluläre lösliche Fraktion | 17,5µl |
| 5 | pEE.14-2/RB11 #15 | FTH1-G3-Ek-PTH | intrazelluläre unlösliche Fraktion | 17,5µl |
| 6 | pEE.14-2/RB11 #20 | FTH1-G3-Ek-PTH | intrazelluläre lösliche Fraktion | 17,5µl |
| 7 | pEE.14-2/RB11 #20 | FTH1-G3-Ek-PTH | intrazelluläre unlösliche Fraktion | 17,5µl |
| 8 | pEE.14-2/RB11 #23 | FTH1-G3-Ek-PTH | intrazelluläre lösliche Fraktion | 17,5µl |
| 9 | pEE.14-2/RB11 #23 | FTH1-G3-Ek-PTH | intrazelluläre unlösliche Fraktion | 17,5µl |
| 10 | pEE.5-1/RB11 #2-1 | FTH1-G3-Ek-PTH | intrazelluläre lösliche Fraktion | 17,5µl |
| 11 | pEE.5-1/RB11 #2-1 | FTH1-G3-Ek-PTH | intrazelluläre unlösliche Fraktion | 17,5µl |
| 12 | pFPMT121/RB11 | Negativkontrollstamm | intrazelluläre lösliche Fraktion | 17,5µl |
| 13 | pFPMT121/RB11 | Negativkontrollstamm | intrazelluläre unlösliche Fraktion | 17,5µl |

Figur 5 sind zwei hervorstechende Ergebnisse zu entnehmen. Erstens kann das FTH1-G3-Ek-PTH-Fusionsprotein bei sehr hohen Pegeln exprimiert werden (siehe Spuren 2 bis 9), welche zumindest in der Größenordnung liegen, die bei FTH1-H6 beobachtet wurde (Spuren 10 und 11). Somit stellt diese erfindungsgemäße Herstellung das erste Beispiel einer effizienten PTH-Expression in H. polymorpha dar.

Zweitens: während die Mehrheit (etwa 70 %) des FTH1-H6-Gen-produkts in der intrazellulären unlöslichen Fraktion gefunden wird (vgl. Spuren 10 und 11; siehe auch Spuren 2 und 3 der Figur 2), verhält sich das FTH1-G3-Ek-PTH-Genprodukt anders: Die Mehrheit (etwa 90 %) wird in der intrazellulären löslichen Fraktion gefunden (vgl. Spuren 2/3, 4/5, 6/7 und 8/9).

Diese bei dem FTH1-G3-Ek-PTH-Fusionsprotein gewonnenen Daten markieren den erfolgreichen Praxisbeweis für das Funktionieren des Prinzips, Ferritine als Funktionspartner für schwer zu exprimierende Proteine zu verwenden.

### 5. Bestimmung der Ausbeute von intracellulär löslichem FTH1-H6 und FTH1-G3-Ek-PTH-Protein in H. polymorpha

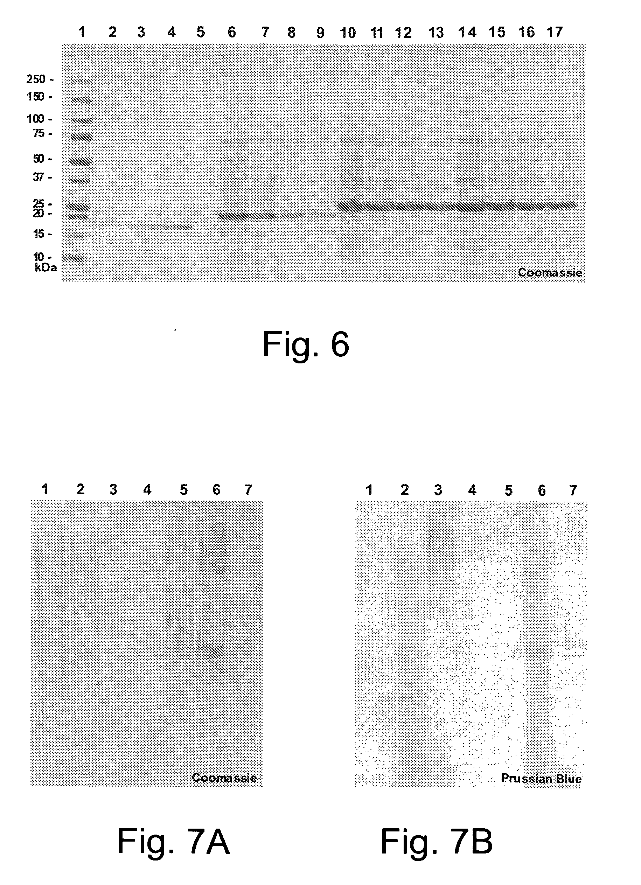

Es wurden die Ausbeuten des Ferritin und seiner Fusionsproteine bei Derepression/MeOH-Induktion in H. polymorpha untersucht. Zu diesem Zweck wurden Pools pEE.14-2/RB11 #20 und pEE.14-2/RB11 #23 (beide exprimieren FTH1-G3-Ek-PTH; vgl. Figur 5) auf YNB/Glukoseplatten separiert, und einzelne Kolonien wurden isoliert und als Stämme definiert. Jeweils ein Stamm jedes Pools wurde ausgewählt und einer neuen Derepression/MeOH-Induktion in der beschriebenen Weise unterzogen (3 ml Maßstab; 37 °C; 180rpm). Der Stamm pEEE.5-1/RB11 #2-1 (der FTH1-H6 ohne Fusionspartner exprimiert) wurde parallel auf die gleiche Weise kultiviert. Die Stämme erreichten bei diesem Experiment die folgenden OD600-Werte: pEE.5-1/RB11 #2-1: 3,58; pEE.14-2/RB11 #20-1: 7,54 und pEE.14-2/RB11 #23-3: 7,80. Die intrazellulären löslichen Fraktionen wurden aufbereitet, und es wurde für jeden Stamm eine Reihe von Verdünnungsschritten ausgeführt. Zusammen mit einer Verdünnungsserie eines Standardproteins (in diesem Falle Interferon α-2a) wurden die Proben separiert auf einem 4-12 % Bis-Tris-Proteingel (denaturierend/reduzierend). Dieses Gel ist in Figur 6 gezeigt.

Fig. 6 zeigt eine Abschätzung der Ausbeuten von intrazellulärem löslichen FTH1-H6 und FTH1-G3-Ek-PTH mittels SDS-PAGE anhand eines Vergleichs mit einem Standardprotein. Nach Methanolinduktion der angegebenen Stämme wie oben zu Fig. 2 beschrieben wurden die intrazellulären löslichen Fraktionen präpariert. Von diesem Material wurden Verdünnungsserien angelegt und mittels SDS-PAGE analysiert. Als Standardprotein wurde gereinigtes Interferon α-2a mit bekannter Konzentration eingesetzt. Das Proteingel wurde nach Beendigung der Auftrennung mit Coomassie Brilliant Blue G250 anfgefärbt.

Das Beladungsschema ist in der nachfolgenden Tabelle 6 angegeben (Abkürzungen: KV: Kulturvolumen; alle weiteren siehe oben zu Fig. 2).

**Tabelle 6:**

| | | | | |
|---|---|---|---|---|
| 1 | Precision Plus Protein Standards | | | 10µl |
| 2 | Interferon α-2a | | | 250ng |
| 3 | Interferon α-2a | | | 500ng |
| 4 | Interferon α-2a | | | 750ng |
| 5 | keine Beladung | | | |
| 6 | PEE.5-1/RB11 Stamm #2-1 | FTH1-G3-Ek-PTH | intrazelluläre lösliche Fraktion | 22,0µl KV |
| 7 | pEE.5-1/RB11 Stamm #2-1 | FTH1-G3-Ek-PTH | intrazelluläre lösliche Fraktion | 11,0µl KV |
| 8 | pEE.5-1/RB11 Stamm #2-1 | FTH1-G3-Ek-PTH | intrazelluläre lösliche Fraktion | 7,3µl KV |
| 9 | pEE.5-1/RB11 Stamm #2-1 | FTH1-G3-Ek-PTH | intrazelluläre lösliche Fraktion | 5,5µl KV |
| 10 | pEE.14-2/RB11 Stamm #20-1 | FTH1-G3-Ek-PTH | intrazelluläre lösliche Fraktion | 11,6µl KV |
| 11 | pEE.14-2/RB11 Stamm #20-1 | FTH1-G3-Ek-PTH | intrazelluläre lösliche Fraktion | 7,7µl KV |
| 12 | pEE.14-2/RB11 Stamm #20-1 | FTH1-G3-Ek-PTH | intrazelluläre lösliche Fraktion | 5,8µl KV |
| 13 | pEE.14-2/RB11 Stamm #20-1 | FTH1-G3-Ek-PTH | intrazelluläre lösliche Fraktion | 4,6µl KV |
| 14 | pEE.14-2/RB11 Stamm #23-3 | FTH1-G3-Ek-PTH | intrazelluläre lösliche Fraktion | 11,2µl KV |
| 15 | pEE.14-2/RB11 Stamm #23-3 | FTH1-G3-Ek-PTH | intrazelluläre lösliche Fraktion | 7,5µl KV |
| 16 | pEE.14-2/RB11 Stamm #23-3 | FTH1-G3-Ek-PTH | intrazelluläre lösliche Fraktion | 5,6µl KV |
| 17 | pEE.14-2/RB11 Stamm #23-3 | FTH1-G3-Ek-PTH | intrazelluläre lösliche Fraktion | 4,5µl KV |

Für eine Abschätzung der Ausbeuten des aus H. polymorpha gewonnenen Materials haben wir die Proben mit den jeweiligen Kulturvolumina korreliert, die dem in den verschiedenen Spuren zu sehenden Material entsprechen. Unsere Abschätzung war folgende:
1. 750ng Interferon α-2a (Spur 4) könnte FTH1-H6 von 7,3µl Kulturvolumen (Spur 8) entsprechen. Gemäß dieser Abschätzung hat der Stamm pEE.5-1/RB11 #2-1 100mg FTH1-H6 L⁻¹ erzeugt.
2. Gemäß dieser Berechnung könnte Spur 6 3 x 750ng = 2,25µg Protein entsprechen.
3. Verglichen mit Spur 6 (2,25µg FTH1-H6) könnten die Spuren 13 (pEE.14-2/RB11 Stamm 20-1; Material entspricht 4,6µl Kulturvolumen) und 17 (pEE.14-2/RB11 Stamm 23-3; Material entspricht 4,5µl Kulturvolumen) 2,25µg bis 4,5µg FTH1-G3-Ek-PTH enthalten.
4. Gemäß dieser Abschätzung hat der Stamm pEE.14-2/RB11 #20-1 und -23-3 0,5g bis 1g FTH1-G3-Ek-PTH Protein pro Liter in dem 3ml-Teströhrchenmaßstab erzeugt (wobei finale OD600-Werte von 7,54 bzw. 7,80 erreicht wurden).
5. Es sei angenommen, dass H. polymorpha eine finale OD600 von 250 bei einer Fermentation mit hoher Zellendichte erreichen könnte. Dann würden wir eine Erhöhung der erlangten Ausbeute im Vergleich zu dem Teströhrchenmaßstab erwarten. Wir können uns vorstellen, dass die Werte für intrazelluläres FTH1-G3-Ek-PTH in die Größenordnung der gegenwärtigen Höchstwerte kommen können (sekretorische Phytase, 13,4g L⁻¹; siehe Meyer A. F., et al., "An expression system matures: a highly efficient and costeffective process for phytase production by recombinant strains of Hansenula polymorpha", Biotechnol. Bioeng. 63, 1999, Seiten 373-381). Die Abschätzung der Ausbeuten sind in Tabelle 7 zusammengefasst:

**Tabelle 7:**

| **Stamm** | **Protein** | **OD600** | **Ausbeute in der intrazellulären löslichen Fraktion (g L⁻¹)** |
|---|---|---|---|
| pEE.51/RB # 5-1 | FTH1-H& | 3,85 | 0,1 |
| pEE.14-2/RB11 # 20-1 | FTH1-G3-Ek-PTH | 7,54 | 0,5 - 1,0 |
| PEE.14-2/RB11 # 23-3 | FTH1-G3-Ek-PTH | 7,80 | 0,5 - 1,0 |

Tabelle 7 zeigt die Ausbeuten von FTH1-H6 oder FTH1-G3-Ek-PTH in der intrazellulären löslichen Fraktion, wie es ausgehend von dem in Figur 6 gezeigten Proteingel abgeschätzt wurde.

### 6. Analyse des Partikelcharakters und der Eisenbindekapazität von in H. polymorpha erzeugtem intracellulärem löslichem Ferritin

Um zu analysieren, ob das aus H. polymorpha abgeleitete FTH1-H6-Material Partikel bildet, wurden der Stamm pEE.5-1/RB11 # 2-1 und ein Negativkontrollstamm kultiviert (Derepression/- MeOH-Induktion in YNB; 37 °C; 180rpm; 3 ml-Maß), und die intrazellulären löslichen Fraktionen wurden aufbereitet. Vor dem Einstellen auf 1 x native und nicht-reduzierende Probenpufferbedingungen wurden die Proben auf dreierlei verschiedene Weise behandelt. 1. Ohne weitere Behandlung; 2. durch Einstellung auf 1 mM Ammonium-Fe(II)-Sulfat, gefolgt von 15' Inkubation bei Raumtemperatur; und 3. wie bei 2., aber mit einer zusätzlichen Inkubation für 15' bei 75 °C (Denaturierung). Aliquots dieser Proben wurden auf 5 %-Tris-HCl-Acrylamidgelen unter nativen und nichtreduzierenden Bedingungen separiert. Ein Gel wurde mit Coomassie Blue gefärbt, um Proteinbanden sichtbar zu machen. Dies ist in Figur 7A gezeigt. Ein anderes Gel wurde einer Preussisch-BlauFärbung ausgesetzt, wie sie von Kim et al. "Expression of Heteropolymeric Ferritin Improves Iron Storage in Saccharomyces cerevisiae", Appl. Environ. Microbiol.69, 2003, S. 1999 bis 2005, beschrieben ist, welche Fe(III) anzeigt. Dies ist in Figur 7B gezeigt. Die Spuren 1 und 5 zeigen die Proben ohne weitere Behandlung. Die Spuren 2 und 6 zeigen die Proben, die mit Ammonium-Fe(II)-Sulfat auf eine Endkonzentration von 1mM eingestellt und anschließend für 15 Minuten bei Raumtemperatur inkubiert wurden. Die Spuren 3 und 7 zeigen die Proben, die für 15 Minuten bei Raumtemperatur und anschließend 15 Minuten bei 75°C inkubiert wurden. Die dominanten Banden in Figur 7A und B, Spuren 6, zeigten exakt die gleiche Mobilität. Das Beladungsschema ist in der nachfolgenden Tabelle 8 angegeben.

**Tabelle 8:**

| | | | | |
|---|---|---|---|---|
| 1 | pFPMT121/RB11 | Negativkontrollstamm | keine weitere Behandlung | 15µl |
| 2 | pFPMT121/RB11 | Negativkontrollstamm | 1mM Ammonium-Fe(II)-Sulfat / 15' Raumtemperatur | 15µl |
| 3 | pFPMT121/RB11 | Negativkontrollstamm | 1mM Ammonium-Fe(II)-Sulfat / 15' Raumtemperatur /15' 75°C | 15µl |
| 4 | keine Beladung | | | |
| 3 | pEE.5-1/RB11 Stamm #2-1 | FTH1-H6 | keine weitere Behandlung | 15µl |
| 4 | pEE.5-1/RB11 Stamm #2-1 | FTH1-H6 | 1mM Ammonium-Fe(II)-Sulfat / 15' Raumtemperatur | 15µl |
| 5 | pEE.5-1/RB11 Stamm #2-1 | FTH1-H6 | 1mM Ammonium-Fe(II)-Sulfat / 15' Raumtemperatur /15' 75°C | 15µl |

Wie es in Figur 7A gezeigt ist, erzeugt das Material des Ferritin-Stamms, das gemäß den Verfahren 1 oder 2 behandelt worden ist, Banden (Spuren 5 und 6), während das entsprechende Material des Negativkontrollstamms keine Banden an diesen Positionen erzeugt (Spuren 1 und 2). Das Erscheinen der Banden unter diesen Gelbedingungen ist als Hinweis darauf zu verstehen, dass durch das FTH1-H6-Protein Partikel gebildet wurden.

Figur 7B ist zu entnehmen, dass das Material des Ferritin-Stamms unter Bedingung 2 (1mM Amonium-Fe(II)-Sulfat/15' Inkubation bei Raumtemperatur) ein Eisensignal zur Verfügung stellt (Figur 7B, Spur 6), welches exakt mit den mutmaßlichen Ferritin-Partikel-Banden, die in Figur 7A gezeigt sind (Spur 6) komigriert. Die ohne weitere Behandlung mit Ammonium-Fe(II)-Sulfat (Figur 7A, Spur 5) auftretenden Partikel-Banden zeigen kein Eisensignal (Figur 7B, Spur 5). Der Negativkontrollstamm zeigt keine entsprechenden Banden oder Signale (Figuren 7A und B, Spuren 1 bzw. 2).

Wir interpretieren diese Ergebnisse wie folgt: 1. Die Expression von FTH1-H6 in H. polymorpha führt zu Ferritin-Partikeln. 2. Diese Partikel fügen sich mit oder ohne hohe Eisenkonzentrationen zusammen. 3. Die Partikel können Eisen binden und speichern, sofern es verfügbar ist.

Anliegend sind im Anhang A die Sequenzen für folgende oben genannte Plasmide und Proteine beigefügt:

| | | |
|---|---|---|
| 1. | pFPMT-CL pre-mhACT-H6 (pEE.1-1) : | SEQ ID NO: 1 |
| 2. | pFPMT-CL pre-mhCGb-H6 (pEE.2-2) : | SEQ ID NO: 2 |
| 3. | pFPMT-CL pre-mhIL6-H6 (pEE.4-1) : | SEQ ID NO: 3 |
| 4. | pFPMT-FTH1-H6 (pEE.5-1) : | SEQ ID NO: 4 |
| 5. | pFPMT-FTH1-G3-Ek-mhACT-H6 (pEE.11-1) : | SEQ ID NO: 5 |
| 6. | pFPMT-FTH1-G3-Ek-mhIL6-H6 (per.12-2) : | SEQ ID NO: 6 |
| 7. | pFPMT-FTH1-G3-Ek-IFNalpha-2a (pEE.13-7): | SEQ ID NO: 7 |
| 8. | pFPMT-FTH1-G3-EK-PTH (per.14-2) : | SEQ ID NO: 8 |
| 9. | GeneArt plasmid 0908426_256_pMA : | SEQ ID NO: 9 |
| 10. | GeneArt plasmid 0908655_348_pMA-RQ : | SEQ ID NO: 10 |
| 11. | GeneArt plasmid 0939988_EE14-15 : | SEQ ID NO: 11 |
| 12. | FTH1-H6 : | SEQ ID NO: 12 |
| 13. | FTH1-G3-Ek-mhACT-H6 : | SEQ ID NO: 13 |
| 14. | FHT1-G3-Ek-mhIL6-H6 : | SEQ ID NO: 14 |
| 15. | FTH1-G3-Ek-IFNα-2a : | SEQ ID NO: 15 |
| 16. | FTH1-G3-Ek-PTH : | SEQ ID NO: 16 |
| 17. | pFPMT121 : | SEQ ID NO: 17 |

Anhang A enthält die Sequenz-Auflistungen, dargestellt als Textdatei.

## Patentansprüche

1. Verfahren zum Herstellen eines Ziel-Proteins, wobei:
a) eine Zellkultur mit Wirtszellen einer Hefeart der Gattungen Saccharomyces, Schizosaccharomyces, Kluyveromyces, Hansenula, Arxula, Schwanniomyces, Candida, Pichia oder Yarrowia bereitgestellt wird, wobei die Wirtszellen
wenigstens eine erste DNA-Sequenz enthalten, die für ein heterologes Ferritin-Protein codiert, und
wenigstens eine zweite DNA-Sequenz enthalten, die für das Ziel-Protein codiert,
wobei die zweite DNA-Sequenz derart mit der ersten DNA-Sequenz gekoppelt ist, dass bei der Expression der ersten und der zweiten DNA-Sequenz das Ziel-Protein an das zuvor synthetisierte Ferritin-Protein gekoppelt wird, so dass ein Verbund-Protein gebildet wird, und
b) die Wirtszellen mit Hilfe der für das heterologe Ferritin-Protein codierenden ersten DNA-Sequenz und der für das Ziel-Protein codierenden zweiten DNA-Sequenz veranlasst werden, das das Ferritin-Protein und das Ziel-Protein enthaltende Verbund-Protein zu exprimieren, wobei das Verbund-Protein in den Wirtszellen gehalten wird,
c) das Verbund-Protein aus der Zellkultur abgetrennt wird, und
d) das Ziel-Protein von dem Verbund-Protein abgetrennt wird.

2. Verfahren nach Anspruch 1, **dadurch gekennzeichnet, dass** die zweite DNA-Sequenz derart mit der ersten DNA-Sequenz gekoppelt ist, dass bei der Expression der ersten und der zweiten DNA-Sequenz das Ferritin-Protein N-terminal positioniert ist.

3. Verfahren nach Anspruch 1 oder 2, **dadurch gekennzeichnet, dass** die zweite DNA-Sequenz über eine dritte DNA-Sequenz mit der ersten DNA-Sequenz gekoppelt ist, wobei die dritte DNA-Sequenz für ein Kopplungspeptid codiert, wobei bei der Expression der ersten, der zweiten und der dritten DNA-Sequenz das Kopplungspeptid an das zuvor synthetisierte Ferritin-Protein und das Ziel-Protein an das zuvor synthetisierte Kopplungspeptid gekoppelt wird.

4. Verfahren nach Anspruch 3, **dadurch gekennzeichnet, dass** das Kopplungspeptid ein Spacer-Peptid umfasst, wobei das Spacer-Peptid bei der Expression der ersten, der zweiten und der dritten DNA-Sequenz an das zuvor synthetisierte Ferritin-Protein gebunden wird.

5. Verfahren nach Anspruch 3 oder 4, **dadurch gekennzeichnet, dass** das Kopplungspeptid ein Trennstellen-Peptid umfasst, das eine von einem Enzym auflösbare Trennstelle bildet, wobei das Ziel-Protein an das Trennstellen-Peptid gebunden wird, wobei im Schritt d) die Trennstelle derart aufgelöst wird, dass das Ziel-Protein freigesetzt wird.

6. Verfahren nach Anspruch 5, **dadurch gekennzeichnet, dass** das Enzym eine Enteropeptidase ist und das Trennstellen-Peptid eine Enteropeptidase-Trennstelle umfasst.

7. Verfahren nach einem der Ansprüche 1 - 6, **dadurch gekennzeichnet, dass** die erste DNA-Sequenz für eine schwere Ferritin-Kette codiert.

8. Verfahren nach einem der Ansprüche 1 - 7, **dadurch gekennzeichnet, dass** im Schritt a) Wirtszellen einer methylotrophen Hefeart der Gattungen Hansenula oder Pichia bereitgestellt werden.

9. Verfahren nach einem der Ansprüche 1 - 8, **dadurch gekennzeichnet, dass** das Ziel-Protein ein heterologes funktionelles Protein ist, wobei das Ziel-Protein homolog nicht in einer Kopplung mit dem Ferritin-Protein auftritt.

10. Verfahren nach Anspruch 9, **dadurch gekennzeichnet, dass** das Ziel-Protein eine Serinprotease, insbesondere matures humanes α1-Antichymotrypsin, umfasst.

11. Verfahren nach Anspruch 9, **dadurch gekennzeichnet, dass** das Ziel-Protein ein Peptidhormon, insbesondere matures humanes Interleukin oder humanes Parathyroid-Hormon oder ein Teilfragment davon, umfasst.

12. Verfahren nach Anspruch 9, **dadurch gekennzeichnet, dass** das Ziel-Protein ein Interferon, insbesondere matures humanes Interferon-α, umfasst.

13. Wirtszelle einer Hefeart der Gattungen Saccharomyces, Schizosaccharomyces, Kluyveromyces, Hansenula, Arxula, Schwanniomyces, Candida, Pichia oder Yarrowia, wobei die Wirtszelle
wenigstens eine erste DNA-Sequenz enthält, die für ein heterologes Ferritin-Protein codiert, und
wenigstens eine zweite DNA-Sequenz enthält, die für ein heterologes Ziel-Protein codiert,
wobei die zweite DNA-Sequenz derart mit der ersten DNA-Sequenz gekoppelt ist, dass bei einer Expression der ersten und der zweiten DNA-Sequenz das Ziel-Protein an das zuvor synthetisierte Ferritin-Protein derart gekoppelt wird, dass ein Verbund-Protein gebildet und das Verbund-Protein in der Wirtszelle gehalten wird.

14. Wirtszelle nach Anspruch 13, **dadurch gekennzeichnet, dass** die zweite DNA-Sequenz derart mit der ersten DNA-Sequenz gekoppelt ist, dass bei der Expression der ersten und der zweiten DNA-Sequenz das Ferritin-Protein N-terminal positioniert ist.

15. Wirtszelle nach Anspruch 13 oder 14, **dadurch gekennzeichnet, dass** die zweite DNA-Sequenz über eine dritte DNA-Sequenz mit der ersten DNA-Sequenz gekoppelt ist, wobei die dritte DNA-Sequenz für ein Kopplungspeptid codiert, wobei bei der Expression der ersten, der zweiten und der dritten DNA-Sequenz das Kopplungspeptid an das zuvor synthetisierte Ferritin-Protein und das Ziel-Protein an das zuvor synthetisierte Kopplungspeptid gekoppelt wird.

16. Wirtszelle nach Anspruch 15, **dadurch gekennzeichnet, dass** das Kopplungspeptid ein Spacer-Peptid umfasst, wobei das Spacer-Peptid bei der Expression der ersten, der zweiten und der dritten DNA-Sequenz an das zuvor synthetisierte Ferritin-Protein gebunden wird.

17. Wirtszelle nach Anspruch 15 oder 16, **dadurch gekennzeichnet, dass** das Kopplungspeptid ein Trennstellen-Peptid umfasst, das eine von einem Enzym auflösbare Trennstelle bildet, wobei das Ziel-Protein an das Trennstellen-Peptid gebunden wird,

18. Wirtszelle nach einem der Ansprüche 13 - 17, **dadurch gekennzeichnet, dass** die erste DNA-Sequenz für eine schwere Ferritin-Kette codiert.

## Claims

1. A method of producing a target protein, wherein:
a) a cell culture with host cells of yeast type of the genus Saccharomyces, Schizosaccharomyces, Kluyveromyces, Hansenula, Arxula, Schwanniomyces, Candida, Pichia or Yarrowia is prepared, wherein the host cells contain at least one first DNA sequence, which codes for a heterologous ferritin protein, and contain at least one second DNA sequence, which codes for the target protein, wherein the second DNA sequence is coupled to the first DNA sequence such that on expression of the first and the second DNA sequence the target protein is coupled to the previously synthesised ferritin protein so that a composite protein is formed, and
b) the host cells are induced, with the aid of the first DNA sequence coding for the heterologous ferritin protein and the second DNA sequence coding for the target protein, to express the composite protein containing the ferritin protein and the target protein, wherein the composite protein is held in the host cells,
c) the composite protein is separated from the cell culture, and
d) the target protein is separated from the composite protein.

2. A method as claimed in claim 1, **characterised in that** the second DNA sequence is coupled to the first DNA sequence such that on expression of the first and the second DNA sequence the ferritin protein is in the N-terminal position.

3. A method as claimed in claim 1 or 2, **characterised in that** the second DNA sequence is coupled to the first DNA sequence via a third DNA sequence, wherein the third DNA sequence codes for a coupling peptide, wherein, on expression of the first, second and third DNA sequence, the coupling peptide is coupled to the previously synthesised ferritin protein and the target protein is coupled to the previously synthesised coupling peptide.

4. A method as claimed in claim 3, **characterised in that** the coupling peptide incudes a spacer peptide, wherein the spacer peptide, on expression of the first, second and third DNA sequence, is bonded to the previously synthesised ferritin protein.

5. A method as claimed in claim 3 or 4, **characterised in that** the coupling peptide includes a cleaving peptide, which forms a cleavage site which may be broken by an enzyme, wherein the target protein is bonded to the cleaving peptide, wherein in step d) the cleavage site is broken such that the target protein is liberated.

6. A method as claimed in claim 5, **characterised in that** the enzyme is an enteropeptidase and the cleaving peptide includes an enteropeptidase cleavage site.

7. A method as claimed in one of claims 1-6, **characterised in that** the first DNA sequence codes for a ferritin heavy chain.

8. A method as claimed in one of claims 1-7, **characterised in that** in step a) host cells of a methylotropic yeast type of the genus Hansenula or Pichia are prepared.

9. A method as claimed in one of claims 1-8, **characterised in that** the target protein is a heterologous, functional protein, wherein the target protein does not occur homologiously in a coupling with the ferritin protein.

10. A method as claimed in claim 9, **characterised in that** the target protein includes a serine protease, particularly mature human α1-antichymotrypsin.

11. A method as claimed in claim 9, **characterised in that** the target protein includes a peptide hormone, particularly mature, human interleukin or human parathyroid hormone or a fragment thereof.

12. A method as claimed in claim 9, **characterised in that** the target protein includes an interferon, particularly mature, human interferon-α.

13. A host cell of a yeast type of the genus Saccharomyces, Schizosaccharomyces, Kluyveromyces, Hansenula, Arxula, Schwanniomyces, Candida, Pichia or Yarrowia, wherein the host cell contains at least one first DNA sequence, which codes for a heterologous ferritin protein, and contains at least one second DNA sequence, which codes for heterologous target protein, wherein the second DNA sequence is coupled to the first DNA sequence such that, on expression of the first and second DNA sequence, the target protein is coupled to the previously synthesised ferritin protein such that a composite protein is formed and the composite protein is held in the host cell.

14. A host cell as claimed in claim 13, **characterised in that** the second DNA sequence is coupled to the first DNA sequence such that, on expression of the first and second DNA sequence, the ferritin protein is in the N-terminal position.

15. A host cell as claimed in claim 13 or 14, **characterised in that** the second DNA sequence is coupled to the first DNA sequence via a third DNA sequence, wherein the third DNA sequence codes for a coupling peptide, wherein, on expression of the first, second and third DNA, the coupling peptide is coupled to the previously synthesised ferritin protein and the target protein is coupled to the previously synthesised coupling peptide.

16. A host cell as claimed in claim 15, **characterised in that** the coupling peptide includes a spacer peptide, wherein the spacer peptide, on expression of the first, second and third DNA sequence, is bonded to the previously synthesised ferritin protein.

17. A host cell as claimed in claim 15 or 16, **characterised in that** the coupling peptide includes a cleaving peptide, which forms a cleavage site breakable by an enzyme, wherein the target protein is bonded to the cleaving peptide.

18. A host cell as claimed in one of claims 13 - 17, **characterised in that** the first DNA sequence codes for a ferritin heavy chain.

## Revendications

1. Procédé de fabrication d'une protéine cible, dans lequel :
a) on prépare une culture cellulaire de cellules hôtes d'un type de levure des espèces Saccharomyces, Schizosaccharomyces, Kluyveromyces, Hansenula, Arxula, Schwanniomyces, Candida, Pichia ou Yarrowia, dans laquelle les cellules hôtes
contiennent au moins une première séquence d'ADN qui code pour une protéine de ferritine hétérologue et
contiennent au moins une deuxième séquence d'ADN qui code pour la protéine cible,
dans lequel la deuxième séquence d'ADN est couplée à la première séquence d'ADN de sorte que, lors de l'expression de la première et de la deuxième séquence d'ADN, la protéine cible soit couplée à la protéine de ferritine préalablement synthétisée de sorte que soit formée une protéine composite, et
b) les cellules hôtes sont, à l'aide de la première séquence d'ADN codant pour la protéine de ferritine hétérologue et de la deuxième séquence d'ADN codant pour la protéine cible, amenées à exprimer la protéine composite contenant la protéine de ferritine et la protéine cible, la protéine composite étant maintenue dans les cellules hôtes,
c) la protéine composite est séparée de la culture cellulaire et
d) la protéine cible est séparée de la protéine composite.

2. Procédé selon la revendication 1, **caractérisé en ce que** la deuxième séquence d'ADN est couplée à la première séquence d'ADN de sorte que, lors de l'expression de la première et de la deuxième séquence d'ADN, la protéine de ferritine soit placée en position N-terminale.

3. Procédé selon la revendication 1 ou la revendication 2, **caractérisé en ce que** la deuxième séquence d'ADN est couplée à la première séquence d'ADN via une troisième séquence d'ADN, moyennant quoi la troisième séquence d'ADN code pour un peptide de couplage, où, lors de l'expression de la première, de la deuxième et de la troisième séquence d'ADN, le peptide de couplage est couplé à la protéine de ferritine préalablement synthétisée et la protéine cible au peptide de couplage préalablement synthétisé.

4. Procédé selon la revendication 3, **caractérisé en ce que** le peptide de couplage comprend un peptide d'espacement, dans lequel le peptide d'espacement est lié à la protéine de ferritine préalablement synthétisée lors de l'expression de la première, de la deuxième et de la troisième séquence d'ADN.

5. Procédé selon la revendication 3 ou la revendication 4, **caractérisé en ce que** le peptide de couplage comprend un peptide d'interface, qui forme une interface soluble par un enzyme, dans lequel la protéine cible est liée au peptide d'interface et dans lequel, à l'étape d), l'interface est dissoute de sorte que la protéine cible soit libérée.

6. Procédé selon la revendication 5, **caractérisé en ce que** l'enzyme est une entéropeptidase et le peptide d'interface comprend une interface d'entéropeptidase.

7. Procédé selon l'une quelconque des revendications 1 à 6, **caractérisé en ce que** la première séquence d'ADN code pour une chaîne de ferritine lourde.

8. Procédé selon l'une quelconque des revendications 1 à 7, **caractérisé en ce que**, à l'étape a), on prépare des cellules hôtes d'un type de levure méthylotrophe des espèces Hansenula ou Pichia.

9. Procédé selon l'une quelconque des revendications 1 à 8, **caractérisé en ce que** la protéine cible est une protéine fonctionnelle hétérologue, dans lequel la protéine cible n'intervient pas de manière homologue dans un couplage avec la protéine de ferritine.

10. Procédé selon la revendication 9, **caractérisé en ce que** la protéine cible est une protéase de sérine, en particulier une α1-antichymotrypsine humaine mature.

11. Procédé selon la revendication 9, **caractérisé en ce que** la protéine cible est une hormone peptidique, en particulier une interleukine humaine mature ou une hormone de parathyroïde humaine ou encore un fragment partiel de celle-ci.

12. Procédé selon la revendication 9, **caractérisé en ce que** la protéine cible comprend un interféron, en particulier un interféron-α humain mature.

13. Cellule hôte d'un type de levure des espèces Saccharomyces, Schizosaccharomyces, Kluyveromyces, Hansenula, Arxula, Schwanniomyces, Candida, Pichia ou Yarrowia, dans laquelle la cellule hôte :
contient au moins une première séquence d'ADN qui code pour une protéine de ferritine hétérologue, et contient au moins une deuxième séquence d'ADN qui code pour une protéine cible hétérologue,
dans laquelle la deuxième séquence d'ADN est couplée à la première séquence d'ADN de sorte que, lors d'une expression de la première et de la deuxième séquence d'ADN, la protéine cible soit couplée à la protéine de ferritine préalablement synthétisée de sorte que soit formée une protéine composite et que la protéine composite soit conservée dans la cellule hôte.

14. Cellule hôte selon la revendication 13, **caractérisée en ce que** la deuxième séquence d'ADN est couplée à la première séquence d' ADN de sorte que, lors de l'expression de la première et de la deuxième séquence d'ADN, la protéine de ferritine assume une position N-terminale.

15. Cellule hôte selon la revendication 13 ou la revendication 14, **caractérisée en ce que** la deuxième séquence d'ADN est couplée à la première séquence d'ADN via une troisième séquence d'ADN, moyennant quoi la troisième séquence d'ADN code pour un peptide de couplage, dans laquelle, lors de l'expression de la première, de la deuxième et de la troisième séquence d'ADN, le peptide de couplage est couplé à la protéine de ferritine préalablement synthétisée et la protéine cible est couplée au peptide de couplage préalablement synthétisé.

16. Cellule hôte selon la revendication 15, **caractérisée en ce que** le peptide de couplage comprend un peptide d'espacement, dans laquelle le peptide d'espacement est lié à la protéine de ferritine préalablement synthétisée lors de l'expression de la première, de la deuxième et de la troisième séquence d'ADN.

17. Cellule hôte selon la revendication 15 ou la revendication 16, **caractérisée en ce que** le peptide de couplage comprend un peptide d'interface, qui forme une interface soluble par un enzyme, dans laquelle la protéine cible est liée au peptide d'interface.

18. Cellule hôte selon l'une quelconque des revendications 13 à 17, **caractérisée en ce que** la première séquence d'ADN code pour une chaîne de ferritine lourde.
